# EUROPEAN PATENT APPLICATION

(11) **EP 4 379 394 A1**
(43) Date of publication of application: **05.06.2024**
(21) Application number: 22849459.7
(22) Date of filing: 26.07.2022
(51) Int. Cl.: G01N 35/08, B01D 19/00, B01J 19/00, B05D 3/06, B81B 1/00, B81C 1/00, B81C 3/00, G01N 37/00, G03F 7/40

(54) **MICROFLUIDIC CHIP AND METHOD FOR MANUFACTURING MICROFLUIDIC MICROCHIP**

(30) Priority: 27.07.2021 JP 2021122577; 27.07.2021 JP 2021122578; 27.07.2021 JP 2021122579; 27.07.2021 JP 2021122580; 15.12.2021 JP 2021203547
(71) Applicant: TOPPAN INC., Tokyo 110-0016 (JP)
(72) Inventor: HAKII, Hidemitsu, Tokyo 110-0016 (JP); FUKUGAMI, Norihito, Tokyo 110-0016 (JP)
(74) Representative: Plasseraud IP
(86) International application number: PCT/JP2022/028683
(87) International publication number: WO 2023/008398

(57) **Abstract**

There is provided a microfluidic chip and a method of producing the microfluidic chip, which can suppress poor bonding between a resin layer forming a channel and a lid component, and can suppress deformation of the channel. A microfluidic chip (1) includes a substrate (10), a partition layer (11) configured by a resin material and provided on the substrate (10) to form a channel, and a cover layer (12) provided on the surface of the partition layer (11) facing away from the substrate (10), wherein the partition layer (11) has an elastic modulus in the range of 1 MPa or more and 10 GPa or less.

## Description

### [Technical Field]

The present disclosure relates to microfluidic chips and methods of producing the same.

### [Background Art]

In recent years, technologies have been proposed which apply lithoprocessing or thick-film processing techniques to form microscopic reaction fields enabling testing of the order of several microliters to several nanoliters. Such techniques using microscopic reaction fields are referred to as µ-TAS (micro-total analysis system).

µ-TAS is applied to areas such as genetic testing, chromosome testing, cell testing, and drug development, as well as biotechnology, testing for trace substances in environments, investigation of environments for growing agricultural products and other products, and genetic testing of agricultural products. Introduction of µ-TAS can bring about significant effects such as automation, higher speed, higher accuracy, lower cost, promptness, and reduced environmental impact.

In µ-TAS, micrometer-sized channels (microchannels) formed on substrates are often used. Such substrates are called chips, microchips, microfluidic chips, etc.

Such microfluidic chips have been produced using techniques such as injection molding, mold formation, cutting, and etching. Glass substrates are mainly used as substrates for microfluidic chips because they are easy to produce and enable optical detection. On the other hand, development of microfluidic chips using resin materials, which are lightweight, less susceptible to breakage than glass substrates, and inexpensive, is also underway. Microfluidic chips using resin materials are mainly produced using a method of forming resin channel patterns by photolithography and bonding lid components thereto. According to this method, fine channel patterns that have been difficult to form using conventional techniques can be formed.

Substrates of microfluidic chips can be bonded using a thermocompression bonding method which uses a heat press machine, ultrasonic welding machine, etc. (e.g., PTL 1), or a method using an adhesive (e.g., PTL 2). Another method has also been proposed in which substrates are bonded by converting a process gas into plasma at or near atmospheric pressure and modifying the substrate surfaces without using an adhesive (e.g., PTL 3).

### [Citation List]

### [Patent Literature]

PTL 1: JP 2002-139419 A
PTL 2: JP 2003-60127 A
PTL 3: JP 2011-104886 A

### [Summary of the Invention]

### [Solution to Problem]

When bonding a resin layer forming a channel to a lid component during production of a microfluidic chip, hardness of the resin layer is an important factor.

For example, if the resin layer is excessively hard and if there are slight irregularities at the bonding interface between the resin layer and the lid component, the resin layer cannot elastically deform to conform to the contact surface of the lid component, and this may deteriorate adhesion and allow gaps to occur. However, if the resin layer forming the channel is excessively soft, the channel may be significantly deformed when bonding the resin layer to the lid component, and this may change the height of the channel, for example, by which slight gaps may be formed between the bonding surfaces of the resin layer and the lid component.

Thus, occurrence of poor bonding between the resin layer and the lid component due to formation of gaps or due to other causes may raise an issue of fluid leakage from the gaps. Such poor bonding can be the cause of significantly reduced productivity. Accordingly, microfluidic chips are required to improve bonding properties between the resin layer forming a channel and the lid component, i.e., to suppress occurrence of poor bonding.

Furthermore, if the resin layer forming the channel is excessively soft, the resin layer may be significantly deformed and the fine channel may also be deformed, collapsed, or blocked. In this case, such microfluidic chips, if they are used for testing with µ-TAS, do not necessarily allow reaction, etc. to proceed properly, and thus are unable to provide an expected performance. Accordingly, microfluidic chips are required to suppress deformation (including blockage) of the channels and enable provision of an expected performance.

In light of the issues set forth above, the present disclosure aims to provide a microfluidic chip and a method of forming the microfluidic chip which can suppress occurrence of poor bonding between a resin layer forming a channel and a lid component, and can suppress deformation of the channel.

### [Solution to Problem]

In order to solve the above issues, a microfluidic chip according to an aspect of the present disclosure includes a base section; a partition section that is configured by a resin material and provided on the base section to form a channel; and a cover section that is provided on a surface of the partition section facing away from the base section, wherein the partition section has an elastic modulus in the range of 1 MPa or more and 10 GPa or less.

A microfluidic chip according to another aspect of the present disclosure includes a base section; a partition section that forms a channel on the base section; and an upper lid section that is formed on a surface of the partition section facing away from the base section to serve as a lid for the channel, wherein the upper lid section and the partition section are joined together by welding.

A microfluidic chip according to still another aspect of the present disclosure includes a base section; a partition section that forms a channel on the base section; a cover section that is located on one side of the partition section facing away from the base section to serve as a lid for the channel; and an intermediate component located between the cover section and the channel.

A method of producing a microfluidic chip according to an aspect of the present disclosure includes steps of applying a resin onto a base section; exposing the applied resin; developing and cleaning the exposed resin to form a partition section that defines a channel on the base section; post-baking the partition section; and bonding a cover section to a surface of the partition section facing away from the base section, wherein the partition section has an elastic modulus in a range of 1 MPa or more and 10 GPa or less.

A method of producing a microfluidic chip according to another aspect of the present disclosure includes steps of applying a first photosensitive resin onto a base section; applying a second photosensitive resin onto the applied first photosensitive resin; exposing the first photosensitive resin and the second photosensitive resin; developing and cleaning the exposed first photosensitive resin and the exposed second photosensitive resin to form a partition section that defines a channel on the base section; and heat-treating and fluidizing the second photosensitive resin on the partition section to form an upper lid section for the channel.

### [Advantageous Effects of the Invention]

According to the aspects of the present disclosure, there can be provided a microfluidic chip that can suppress occurrence of poor bonding between the resin layer forming a channel and the lid component, and can suppress deformation of the channel.

### [Brief Description of the Drawings]

Fig. 1 is a set of schematic diagrams illustrating a configuration example of a microfluidic chip according to Embodiment 1-1 of the present disclosure, in which (a) is a plan view and (b) is a cross-sectional view.
Fig. 2 is a flowchart illustrating an example of a method of producing the microfluidic chip according to Embodiment 1-1 of the present disclosure.
Fig. 3 is a schematic cross-sectional view illustrating a configuration example of a microfluidic chip according to Embodiment 1-2 of the present disclosure.
Fig. 4 is a schematic cross-sectional view illustrating a configuration example of a microfluidic chip according to Embodiment 1-3 of the present disclosure.
Fig. 5 is a set of schematic diagrams illustrating a configuration example of a microfluidic chip according to Embodiment 2-1 of the present disclosure, in which (a) is a plan view and (b) is a cross-sectional view.
Fig. 6 is a schematic cross-sectional view illustrating an example of a cross section of a channel of the microfluidic chip according to Embodiment 2-1 of the present disclosure.
Fig. 7 is a schematic cross-sectional view illustrating an enlarged cross section of the microfluidic chip according to Embodiment 1-2 of the present disclosure.
Fig. 8 is a flowchart illustrating an example of a method of producing the microfluidic chip according to Embodiment 2-1 of the present disclosure.
Fig. 9 is a set of schematic cross-sectional views illustrating part of a process of producing a microfluidic chip using a production method according to Embodiment 2-1 of the present disclosure, in which (a) shows a resin material applied onto a substrate, (b) shows a groove formed between parts of a partition layer, and (c) shows an example of a microfluidic chip prepared using the production method.
Fig. 10 is a set of schematic cross-sectional views illustrating a process of producing a microfluidic chip using another production method according to Embodiment 2-1 of the present disclosure, in which (a) shows a resin material applied onto a substrate, (b) shows a groove formed between parts of a partition layer, and (c) shows an example of a microfluidic chip prepared using the production method.
Fig. 11 is a set of schematic cross-sectional views illustrating a process of producing a microfluidic chip using still another production method according to Embodiment 2-1 of the present disclosure, in which (a) shows a resin material applied onto a substrate, (b) shows a groove formed between parts of a partition layer, and (c) shows an example of a microfluidic chip prepared using the production method.
Fig. 12 is a schematic cross-sectional view illustrating an example of a cross section of a channel of a microfluidic chip according to Embodiment 2-2 of the present disclosure.

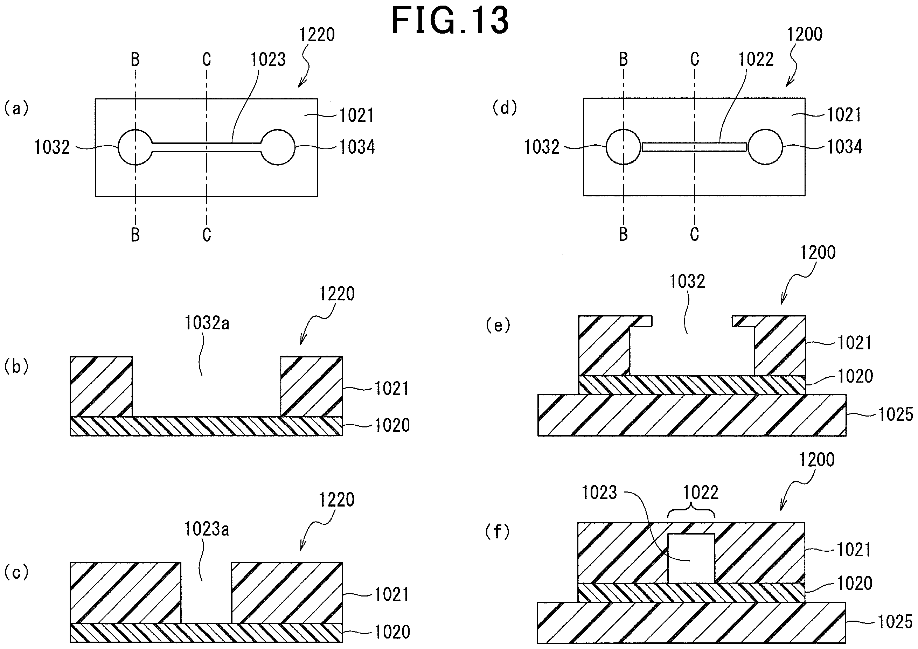

Fig. 13 is a set of schematic diagrams illustrating a process of producing a microfluidic chip using a method of producing a microfluidic chip according to Embodiment 2-2 of the present disclosure, in which (a) is a plan view of an example of a channel pattern, (b) is a cross-sectional view of an inlet region in the channel pattern, (c) is a cross-sectional view of a channel region in the channel pattern, (d) is a plan view of a microfluidic chip prepared using the production method, (e) is a cross-sectional view of an inlet of the microfluidic chip prepared using the production method, and (f) is a cross-sectional view of a channel section of the microfluidic chip prepared using the production method.
Fig. 14 is a set of SEM images of a microfluidic chip according to the Second Example, in which (a) shows a cross section before post-baking, and (b) shows a cross section after post-baking.
Fig. 15 is a set of schematic diagrams illustrating a configuration example of a microfluidic chip according to Embodiment 3-1 of the present disclosure, in which (a) is a plan view, and (b) is a cross-sectional view.
Fig. 16 is a schematic cross-sectional view illustrating an example of a cross section of a channel of the microfluidic chip according to Embodiment 3-1 of the present disclosure.
Fig. 17 is a schematic cross-sectional view illustrating an enlarged cross section of the microfluidic chip according to Embodiment 3-1 of the present disclosure.
Fig. 18 is a flowchart illustrating an example of a method of producing the microfluidic chip according to Embodiment 3-1 of the present disclosure.
Fig. 19 is a set of schematic cross-sectional views illustrating part of a process of producing a microfluidic chip using a production method according to Embodiment 3-1 of the present disclosure, in which (a) shows a resin material applied onto a substrate, (b) shows a groove formed between parts of a partition layer, and (c) shows an example of a microfluidic chip prepared using the production method.
Fig. 20 is a set of schematic cross-sectional views illustrating a process of producing a microfluidic chip using another production method according to Embodiment 3-1 of the present disclosure, in which (a) shows a resin material applied onto a substrate, (b) shows a groove formed between parts of a partition section, and (c) shows an example of a microfluidic chip prepared using the production method.
Fig. 21 is a set of schematic cross-sectional views illustrating a process of producing a microfluidic chip using still another production method according to Embodiment 3-1 of the present disclosure, in which (a) shows a resin material applied onto a substrate, (b) shows a groove formed between parts of a partition section, and (c) shows an example of a microfluidic chip prepared using the production method.
Fig. 22 is a schematic cross-sectional view illustrating an example of a cross section of a channel of a microfluidic chip according to Embodiment 3-2 of the present disclosure.
Fig. 23A is a set of schematic diagrams (No. 1) illustrating a process of producing a microfluidic chip using a production method according to Embodiment 3-2 of the present disclosure, in which (a) is a plan view of an example of a channel pattern, (b) is a cross-sectional view of an inlet region in the channel pattern, and (c) is a cross-sectional view of a channel region in the channel pattern.
Fig. 23B is a set of schematic diagrams (No. 2) illustrating a process of producing a microfluidic chip using a production method according to Embodiment 3-2 of the present disclosure, in which (d) is a plan view of the channel pattern after post-baking, (e) is a cross-sectional view of the channel pattern taken along the line B-B of (d), and (f) is a cross-sectional view of the channel pattern taken along the line C-C of (d).
Fig. 24 is a set of schematic cross-sectional views each illustrating a microfluidic chip according to Embodiment 3-3 of the present disclosure, in which (a) shows one example, and (b) shows another example.
Fig. 25 is a set of SEM images of a microfluidic chip according to the Third Example, in which (a) shows a cross section before post-baking, and (b) shows a cross section after post-baking.
Fig. 26 is a set of schematic diagrams illustrating a configuration example of a microfluidic chip according to Embodiment 4-1 of the present disclosure, in which (a) is a plan view, (b) is a plan view of an enlarged bubble removal section, and (c) is a cross-sectional view.
Fig. 27 is a schematic cross-sectional view illustrating an example of a cross section of a channel of the microfluidic chip according to Embodiment 4-1 of the present disclosure.
Fig. 28 is a schematic cross-sectional view illustrating an enlarged cross section of the microfluidic chip according to Embodiment 4-1 of the present disclosure.
Fig. 29 is a flowchart illustrating an example of a method of producing the microfluidic chip according to Embodiment 4-1 of the present disclosure.
Fig. 30 is a set of schematic cross-sectional views illustrating part of a process of producing a microfluidic chip using a production method according to Embodiment 4-1 of the present disclosure, in which (a) shows a resin material applied onto a substrate, (b) shows a groove formed between parts of a partition layer, and (c) shows an example of a microfluidic chip prepared using the production method.
Fig. 31 is a set of schematic cross-sectional views illustrating a process of producing a microfluidic chip using another production method according to Embodiment 4-1 of the present disclosure, in which (a) shows a resin material applied onto a substrate, (b) shows a groove formed between parts of a partition section, and (c) shows an example of a microfluidic chip prepared using the production method.
Fig. 32 is a set of schematic cross-sectional views illustrating a process of producing a microfluidic chip using still another production method according to Embodiment 4-1 of the present disclosure, in which (a) shows a resin material applied onto a substrate, (b) shows a groove formed between parts of a partition section, and (c) shows an example of a microfluidic chip prepared using the production method.
Fig. 33 is a set of schematic diagrams illustrating a channel pattern according to Embodiment 4-1 of the present disclosure, in which (a) is a plan view of the channel pattern before performing heat treatment (post-baking) , (b) is a cross-sectional view of a substrate, a partition layer, and a second photosensitive resin layer on the partition layer which define the channel pattern, taken along the line D-D of (a), and (c) is a cross-sectional view of the substrate, the partition layer, and a second photosensitive resin layer taken along the line E-E of (a).
Fig. 34 is a set of schematic diagrams illustrating a microfluidic chip according to Embodiment 4-1 of the present disclosure, in which (a) is a plan view of the microfluidic chip after heat treatment (post-baking), (b) is a cross-sectional view of the microfluidic chip taken along the line D-D of (a), and (c) is a cross-sectional view of the microfluidic chip taken along the line E-E of (a).
Fig. 35 is a schematic cross-sectional view illustrating an example of a cross section of a channel of a microfluidic chip according to Embodiment 4-2 of the present disclosure.

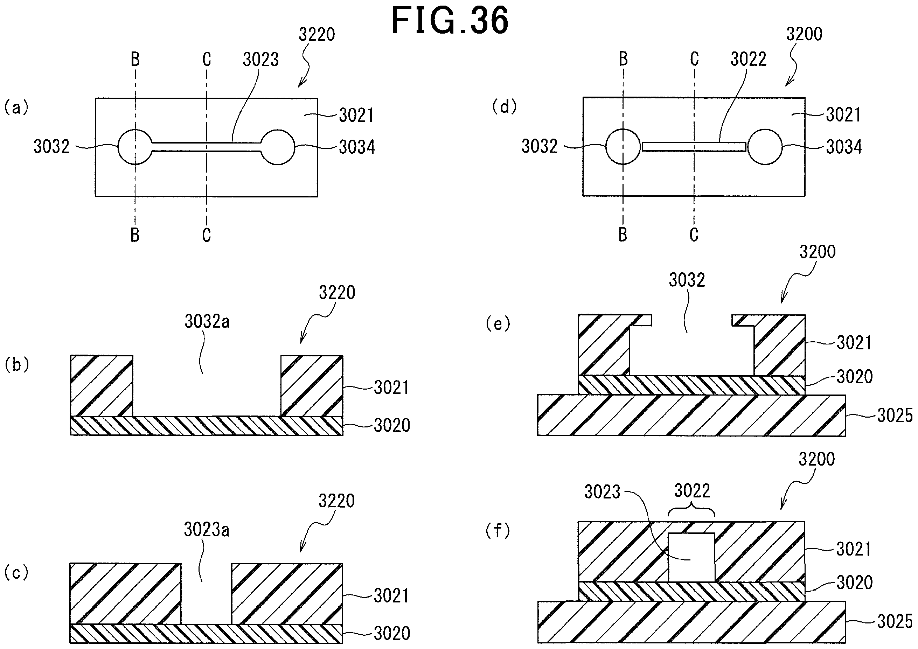

Fig. 36 is a set of schematic diagrams illustrating a process of producing a microfluidic chip using a method of producing the microfluidic chip according to Embodiment 4-2 of the present disclosure, in which (a) is a plan view of an example of a channel pattern, (b) is a cross-sectional view of an inlet region in the channel pattern, (c) is a cross-sectional view of a channel region in the channel pattern, (d) is a plan view of a microfluidic chip prepared using the production method, (e) is a cross-sectional view of an inlet of the microfluidic chip prepared using the production method, and (f) is a cross-sectional view of a channel section of the microfluidic chip prepared using the production method.
Fig. 37 is a set of schematic plan views, in which (a) shows a channel pattern before heat treatment (post-baking) according Embodiment 4-2 of the present disclosure, and (b) shows a microfluidic chip after heat treatment (post-baking) according to Embodiment 10 of the present disclosure.
Fig. 38 is a schematic cross-sectional view illustrating an example of a cross section of a channel of a microfluidic chip according to Embodiment 4-3 of the present disclosure.
Fig. 39 is a flowchart illustrating an example of a method of producing the microfluidic chip according to Embodiment 4-3 of the present disclosure.
Fig. 40 is a set of SEM images of a microfluidic chip according to Fourth Example, in which (a) shows a cross section before post-baking, and (b) shows a cross section after post-baking.

### [Description of the Embodiments]

The present disclosure will be described below through embodiments; however, the following embodiments should not limit the invention recited in the claims. Not all the combinations of features described in the embodiments are essential to the Solution to Problem of the invention. The drawings illustrate the invention only schematically, and the dimensions such as width and thickness of the components, and the ratios thereof are not to scale.

### 1. First embodiment

A microfluidic chip according to a first embodiment of the present disclosure will be described. In the following description, the substrate side of the microfluidic chip may be referred to as lower, and the opposite side of the microfluidic chip from the substrate side (lid component side) may be referred to as upper.

As a result of diligent studies, the present inventors have found that if the elastic modulus and Vickers hardness of the resin layer forming a channel in a microfluidic chip are controlled, the resin layer, when it is bonded to a lid component, can conform to slight irregularities at the bonding interface, such as height variation of the resin layer occurring when formed, and surface asperities or lack of flatness of the lid component, and that poor bonding due to generation of gaps or the like and deformation of fine channel can be suppressed. Thus, the present inventors have invented a microfluidic chip and a method of producing the microfluidic chip, which can suppress occurrence of poor bonding between a resin layer forming a channel and a lid component, and can suppress deformation of the channel.

Referring to the drawings, some modes (Embodiments 1-1, 1-2 and 1-3) of the first embodiment will be described.

### (1.1) Embodiment 1-1

### (1.1.1) Basic configuration of microfluidic chip

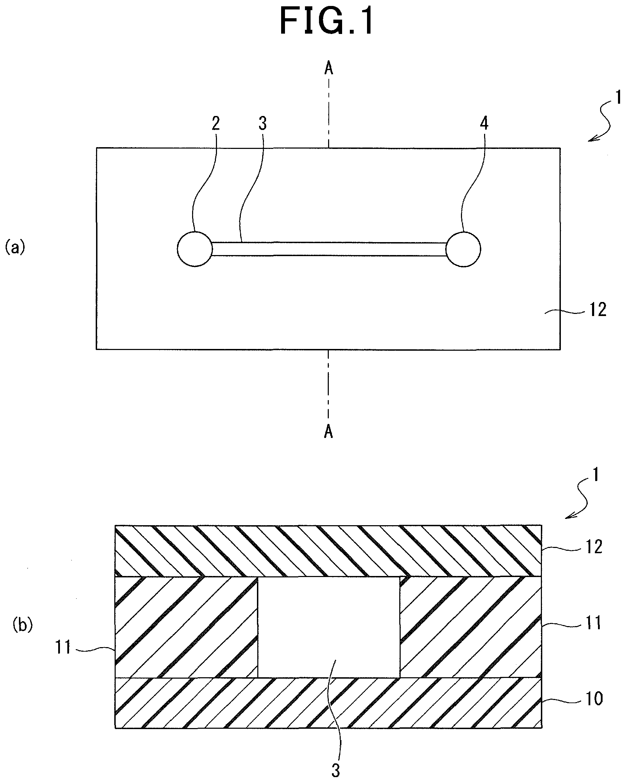

Fig. 1 is a set of schematic diagrams illustrating a configuration example of a microfluidic chip 1 according to Embodiment 1-1 of the present disclosure (hereinafter referred to as the present embodiment). Specifically, Fig. 1(a) is a schematic plan view illustrating the microfluidic chip 1 according to the present embodiment. Fig. 1(b) is a schematic cross-sectional view illustrating a cross section of the microfluidic chip 1 taken along the line A-A of Fig. 1(a).

As shown in Fig. 1(a), the microfluidic chip 1 includes an inlet 2 for introducing a fluid (e.g., liquid), a channel section 3 through which the fluid introduced from the inlet 2 flows, and an outlet 4 for discharging the fluid from the channel section 3. In the microfluidic chip 1, the channel section 3 is covered with a cover layer 12, and the inlet 2 and the outlet 4 are through-holes provided to the cover layer 12. Details of the cover layer 12 will be described later.

Fig. 1(a) shows the channel section 3 that can be seen through a transparent cover layer 12.

The microfluidic chip 1 may be provided with at least one or more inlets 2 and outlets 4, or may be provided with multiple of each. The microfluidic chip 1 may be provided with multiple channel sections 3, or may be designed such that the fluid introduced from the inlet 2 can be merged or separated.

Details of the structural components of the channel section 3 of the microfluidic chip 1 will be described later. As shown in Fig. 1(b), the microfluidic chip 1 includes a substrate (example of the base section) 10, a partition layer (example of the partition section) 11 provided on the substrate 10 to form a channel, and a cover layer (example of the cover section) 12 provided on the surface of the partition layer 11 facing away from the substrate 10. The channel section 3 through which the fluid introduced from the inlet 2 flows is a region surrounded by the substrate 10, the partition layer 11, and the cover layer 12. The channel section 3 is defined within the partition layer 11 provided on the substrate 19 and is covered with the cover layer 12, as a lid component, provided on the side opposite from the substrate 10. As mentioned above, a fluid is introduced into the channel section 3 from the inlet 2 (see Fig. 1(a)) provided to the cover layer 12, passes through the channel section 3, and is discharged from the outlet 4.

### (1.1.1.1) Substrate

The substrate 10 serves as a base of the microfluidic chip 1, and the channel section 3 is configured by the partition layer 11 provided on the substrate 10. In other words, the substrate 10 and the partition layer 11 configure a body of the microfluidic chip 1.

The substrate 10 can be formed using either a translucent material or a non-translucent material. For example, if the state inside the channel section 3 (state of the fluid) is detected and observed using light, a material having good transparency to light can be used. As the translucent material, resins, glass, etc. can be used. Examples of the resin used as a translucent material for forming the substrate 10 may be acrylic resins, methacrylic resins, polypropylenes, polycarbonate resins, cycloolefin resins, polystyrene resins, polyester resins, urethane resins, silicone resins, fluororesins, and the like, which are preferred from the perspective of suitability for forming the body of the microfluidic chip 1.

For example, if the state inside the channel section 3 (state of the fluid) is not required to be detected and observed using light, a non-translucent material may be used. Examples of non-translucent materials include silicon wafers and copper plates. The thickness of the substrate 10 is not particularly limited, but is preferred to be in the range of 10 µm (0.01 mm) or more and 10 mm or less because some degree of rigidity is required in the channel formation process.

### (1.1.1.2) Partition layer

The partition layer 11 is provided on the substrate to configure the channel section 3. The partition layer 11 can be formed using a resin material. For example, a photosensitive resin can be used as the resin material of the partition layer 11.

The photosensitive resin forming the partition layer 11 is preferred to have photosensitivity to light having a wavelength of 190 nm or more and 400 nm or less in the UV light region. As the photosensitive resin, photoresists such as liquid resists and dry film resists can be used. These photosensitive resins may be of positive type with which the photosensitive area becomes soluble, or may be of negative type with which the photosensitive area becomes insoluble. Examples of the photosensitive resin composition suitable for forming the partition layer 11 in the microfluidic chip 1 include negative radical type photosensitive resins containing alkali-soluble polymers, addition polymerizable monomers, and photopolymerization initiators. Examples of the photosensitive resin materials include acrylic resins, acrylic urethane resins (urethane acrylate resins), epoxy resins, polyamide resins, polyimide resins, polyurethane resins, polyester resins, polyether resins, polyolefin resins, polycarbonate resins, polystyrene resins, norbornene resins, phenol novolak resins, and other photosensitive resins. These resins may be used singly, or may be used by mixing or copolymerizing a plurality of them.

In the present embodiment, the resin material used for the partition layer 11 is not limited to photosensitive resins, but may be, for example, silicone rubber (PDMS: polydimethylsiloxane) or synthetic resins. Examples of the synthetic resins include polymethyl methacrylate resins (PMMA), polycarbonates (PC), polystyrene resins (PS), polypropylenes (PP), cycloolefin polymers (COP), and cycloolefin copolymers (COC). The resin material for the partition layer 11 is preferred to be appropriately selected according to usage.

The thickness of the partition layer 11, i.e., the height of the channel section 3, on the substrate 10 is not particularly limited, but is required to be larger than the substances (e.g., drugs, bacteria, cells, red blood cells, white blood cells, etc.) to be analyzed and tested which are contained in the fluid introduced into the channel section 3. Therefore, the thickness of the partition layer 11, i.e., the height of the channel section 3, is preferred to be in the range of 5 µm or more and 100 µm or less.

Similarly, since the width of the channel section 3 is required to be larger than the substances to be analyzed and tested, the width of the channel section 3 defined within the partition layer 11 is preferred to be in the range of 5 µm or more and 100 µm or less.

In the microfluidic chip 1 of the present embodiment, the partition layer 11 has an elastic modulus in the range of 1 MPa or more and 10 GPa or less. In the partition layer 11 and the cover layer 12 (lid component) formed of resin materials, slight irregularities may occur at the bonding interface when they are bonded together in the process of producing the microfluidic chip 1. If the elastic modulus of the partition layer exceeds 10 GPa, the partition layer 11 becomes excessively hard and will not be able to conform to slight irregularities at the bonding interface, and may cause gaps when bonded to the cover layer 12. In this case, poor bonding may occur when the partition layer 11 is bonded to the cover layer 12.

However, if the elastic modulus is less than 1 MPa, the partition layer 11 may become excessively soft and may be easily deformed when bonded or on other occasions in the process of producing the microfluidic chip 1. In this case, deformation or blocking may occur in the channel section 3.

In the microfluidic chip 1 according to the present embodiment, by setting the elastic modulus of the partition layer 11 to 10 GPa or less, it is possible to prevent the partition layer 11 from becoming excessively hard. Therefore, for example, the partition layer 11 can conform to slight irregularities at the bonding interface when bonded to the cover layer 12, and can prevent formation of gaps to suppress occurrence of poor bonding between the partition layer 11 and the cover layer 12.

Furthermore, in the microfluidic chip 1, by setting the elastic modulus of the partition layer 11 to 1 MPa or more, it is possible to prevent the partition layer 11 from becoming excessively soft. Therefore, for example, the partition layer 11 is suppressed from being significantly deformed such as by external forces applied when bonded to the cover layer 12, thereby suppressing deformation and blocking of the channel section 3.

Thus, in the microfluidic chip 1 of the present embodiment, the partition layer 11, as a resin layer forming the channel, having an elastic modulus in the range of 1 MPa or more and 10 GPa or less can suppress occurrence of poor bonding when it is bonded to the cover layer 12, as a lid component, and can also suppress deformation (including blocking) of the channel section 3 configured by the partition layer 11.

In the microfluidic chip 1 of the present embodiment, an elastic modulus in the range of 1 MPa or more and 2 GPa or less is more preferred because the partition layer 11 can also conform to the locally occurring unevenness on the bonding surface of the partition layer 11 or the cover layer 12, in addition to being able to suppress occurrence of poor bonding between the partition layer 11 and the cover layer 12 as a lid component and being able to suppress deformation (including blocking) of the channel section 3 configured by the partition layer 11.

In the microfluidic chip 1 of the present embodiment, an elastic modulus in the range exceeding 2 GPa and 10 GPa or less is even more preferred because the partition layer 11 can conform to larger unevenness such as undulation of the bonding surface of the partition layer 11 or the cover layer 12 or warpage of the substrate, in addition to being able to suppress occurrence of poor bonding between the partition layer 11 and the cover layer 12 as a lid component and being able to suppress deformation (including blocking) of the channel section 3 configured by the partition layer 11.

Control and measurement of the elastic modulus of the partition layer 11 in the microfluidic chip 1 will be described later.

Thus, it is important that the partition layer 11 has elasticity (elastic modulus) that is sufficient for conforming to irregularities or the like at the bonding interface when bonded to the cover layer 12 and suppressing occurrence of deformation or blocking in the channel section 3 due to external forces or the like being applied thereto during bonding. On the other hand, it is also important that deformation due to external forces is restored when, for example, the external forces have been removed after the partition layer 11 has been bonded to the cover layer 12, i.e., it is important that the partition layer 11 is less likely to be plastically deformed.

The present inventors have found that plastic deformation is less likely to occur during bonding of the microfluidic chip 1 if the Vickers hardness of the partition layer 11 formed of a resin material has a predetermined value or more. Specifically, in the microfluidic chip 1 of the present embodiment, the partition layer 11 is preferred to have a Vickers hardness of 2 HV or more. If the Vickers hardness is less than 2 HV, plastic deformation is likely to occur and deformation due to external forces is less likely to be restored. If the Vickers hardness of the partition layer 11 is 2 HV or more, plastic deformation is less likely to occur in the partition layer 11, and therefore, deformation or blocking of the channel section 3 can be more reliably suppressed. The Vickers hardness of the partition layer 11 is preferred to be 80 HV or less. If the Vickers hardness exceeds 80 HV, cracking or the like may occur due to the compressive force applied when the partition layer 11 and the cover layer 12 are bonded together. If the Vickers hardness is 80 HV or less, occurrence of cracking or the like can be suppressed which is due to the compressive force applied when the partition layer 11 and the cover layer 12 are bonded together.

Control and measurement of the Vickers hardness of the partition layer 11 in the microfluidic chip 1 will be described later.

### (1.1.1.3) Cover layer

As shown in Fig. 1(b), the cover layer 12 in the microfluidic chip 1 of the present embodiment is a lid component covering the channel section 3. As mentioned above, the cover layer 12 is provided on the surface of the partition layer 11 facing away from the substrate 10, and faces the substrate 10 via the partition layer 11. More specifically, as shown in Fig. 1(b), the side end portions of the cover layer 12, as viewed in cross section, are supported by the partition layer 11, the center region thereof faces the substrate 10, and the center region defines the upper part of the channel section 3.

The cover layer 12 can be formed using either a translucent material or a non-translucent material. For example, if the state inside the channel is detected and observed using light, a material having good transparency to light can be used. As the translucent material, resins, glass, etc. can be used. Examples of the resin for forming the cover layer 12 include acrylic resins, methacrylic resins, polypropylenes, polycarbonate resins, cycloolefin resins, polystyrene resins, polyester resins, urethane resins, silicone resins, and fluororesins, which are preferred from the perspective of suitability for forming the body of the microfluidic chip 1. The thickness of the cover layer 12 is not particularly limited but is preferred to be in the range of 10 µm or more and 10 mm or less from the perspective of providing through-holes corresponding to the inlet 2 and the outlet 4 of the cover layer 12. It is preferred that, before being bonded to the partition layer 11, the cover layer 12 is provided with holes in advance corresponding to the inlet 2 for introducing a fluid (liquid) and the outlet 4 for discharging the fluid.

The elastic modulus of the cover layer 12 may be greater than that of the partition layer 11. In this case, the cover layer 12 will be improved in physical strength more than that of the partition layer 11 and thus will be improved in durability against external forces applied when bonded to the partition layer 11.

The elastic modulus of the cover layer 12 may be smaller than that of the partition layer 11. In this case, the cover layer 12 becomes softer than the partition layer 11 and will be improved in conformability at the bonding interface between the partition layer 11 and the cover layer 12, and poor bonding will be suppressed even more. If the elastic modulus of the cover layer 12 becomes smaller than that of the partition layer 11, holes corresponding to the inlet 2 and the outlet 4 can be easily formed, and workload during production can be reduced.

In the microfluidic chip 1 of the present embodiment, the elastic modulus of the cover layer 12 may be in the range of 1 MPa or more and 10 GPa or less similarly to the elastic modulus of the partition layer 11.

### (1.1.2) Method of producing microfluidic chip

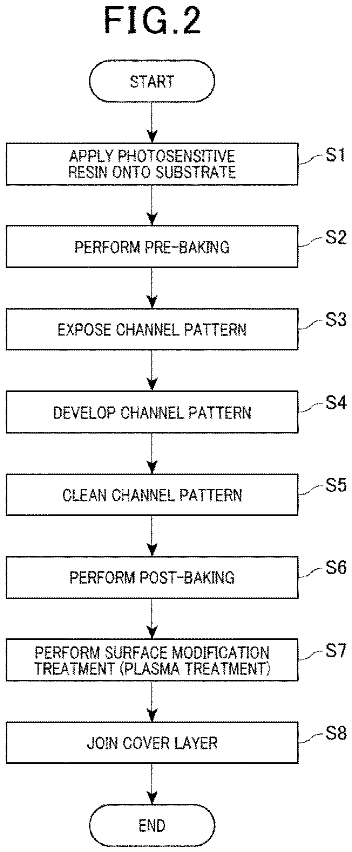

Next, a method of producing the microfluidic chip 1 of the present embodiment will be described. Fig. 2 is a flowchart illustrating an example of a method of producing the microfluidic chip 1 according to the present embodiment.

Description herein will be provided taking an example in which the partition layer 11 is formed of a photosensitive resin.

### (Step S1)

In the method of producing the microfluidic chip 1 of the present embodiment, a resin is applied first onto a substrate 10. Thus, a resin layer for forming the partition layer 11 is provided on the substrate 10. In the method of producing the microfluidic chip 1 of the present embodiment, for example, a resin layer (photosensitive resin layer) made of a photosensitive resin is formed on the substrate 10.

The photosensitive resin layer is formed on the substrate 10, for example, by applying a photosensitive resin onto the substrate 10. Examples of the coating method include spin coating, spray coating, and bar coating, among which, spin coating is preferred from the perspective of film thickness controllability. The photosensitive resin that can be applied to the substrate 10 can be in various forms such as liquid, solid, gel-like, and film-like forms. Of these forms, liquid resists are preferred to be used for forming the photosensitive resin layer.

The resin (e.g., photosensitive resin) may be applied to the substrate 10 such that the thickness of the resin layer (e.g., photosensitive resin layer), i.e., the thickness of the partition layer 11, will be in the range of 5 µm or more and 100 µm or less.

### (Step S2)

Following formation of the photosensitive resin on the substrate 10, heat treatment (pre-baking treatment) is performed for the purpose of removing solvent contained in the resin (e.g., photosensitive resin) applied to the substrate 10. In the method of producing the microfluidic chip 1 of the present embodiment, pre-baking treatment is not essential and may be adequately performed at an optimal temperature for an optimal duration of time according to the characteristics of the resin. For example, if the resin layer on the substrate 10 is formed of a photosensitive resin, pre-baking may be performed under optimal temperature and duration of time adequately determined according the characteristics of the photosensitive resin.

### (Step S3)

Next, the resin (e.g., photosensitive resin) applied to the substrate 10 is exposed. Specifically, a channel pattern is drawn by exposure on the photosensitive resin applied to the substrate 10. Exposure may be performed, for example, using an exposure device that uses UV light as a light source, or using a laser drawing device. Among them, proximity exposure using UV light as a light source or exposure using a contact exposure device is preferred. If a proximity exposure device is used, exposure is performed via a photomask with a channel pattern array for the microfluidic chip 1. The photomask may be a photomask with a light-shielding film that has a double-layer structure of chromium and chromium oxide.

As mentioned above, a photosensitive resin having photosensitivity to light with a wavelength of 190 nm or more and 400 nm or less in the UV light region, is used for the partition layer 11. Accordingly, in the present step (exposure), the photosensitive resin applied to the substrate 10 may be exposed to light with a wavelength of 190 nm or more and 400 nm or less.

If the photosensitive resin applied to the substrate 10 is a positive resist, the exposed regions are dissolved, forming the channel section 3, and the photosensitive resin in the unexposed regions remain as parts of the partition layer 11. If the photosensitive resin applied to the substrate 10 is a negative resist, the photosensitive resin in the exposed region remains as parts of the partition layer 11, with the unexposed regions dissolved, forming the channel section 3. In this way, in the method of producing the microfluidic chip 1 of the present embodiment, the partition layer 11 configuring the channel section 3 can be formed on the substrate 10 using photolithography.

If a chemically amplified resist or the like is used for forming a resin layer on the substrate 10, heat treatment (post-exposure baking: PEB) may be further performed after exposure to promote catalytic reaction of the acid generated due to exposure.

### (Step S4)

Next, the exposed photosensitive resin is developed to form a channel pattern.

The development is performed, for example, using a spray-, dip- or paddle-type or other types of developing device, by reaction of the photosensitive resin with a developer. Examples of the developer include sodium carbonate aqueous solution, tetramethylammonium hydroxide, potassium hydroxide, and organic solvents. The developer is not limited to these, but an optimal developer may be adequately used according to the characteristics of the photosensitive resin. The concentration or the time of development can be adequately adjusted to optimal conditions according to the characteristics of the photosensitive resin.

### (Step S5)

Next, the resin layer (photosensitive resin layer) on the substrate 10 is cleaned to completely remove the developer used for development. Cleaning may be performed, for example, using a spray-, shower-, immersion-type or other types of cleaning device. An optimal cleaning solution may be adequately used from among, for example, pure water, isopropyl alcohol, and the like to remove the developer used for development. Cleaning is followed by drying using a spin dryer, IPA vapor dryer, natural drying, etc.

### (Step S6)

Next, the channel pattern, i.e., the partition layer 11 forming the channel section 3, undergoes heat treatment (post-baking). With this post-baking treatment, residual moisture generated during development or cleaning is removed. Post-baking treatment is performed, for example, using a hot plate, oven, etc. If the drying performed at the cleaning step S5 is insufficient, the developer or moisture generated during cleaning may remain in the partition layer 11. Solvent that has not been removed in the pre-baking treatment may also remain in the partition layer 11. These materials can be removed by performing post-baking treatment.

### (Step S7)

The post-baking treatment is followed by a surface modification treatment on the partition layer 11 and the cover layer 12 (lid component) before being bonded to the partition layer 11. As an example of the surface modification treatment, a plasma treatment may be performed. A surface modification treatment, which is not essential in the method of producing the microfluidic chip 1 of the present embodiment, may be adequately performed as necessary.

### (Step S8)

Next, a cover layer 12 is bonded to the partition layer 11 that has undergone post-baking treatment. In the present step, as shown in Fig. 1(b), the cover layer 12 is bonded to the surface of the partition layer 11 facing away from the substrate 10. Thus, the channel section 3 is covered with the cover layer 12, thereby forming the microfluidic chip 1 shown in Figs. 1(a) and 1(b).

The substrates that have undergone surface modification treatment at step S7 are preferred be bonded together, for example, through thermocompression using a thermocompression machine or heat rolling machine.

It is preferred that the cover layer 12 is provided with holes in advance corresponding to the inlet 2 and the outlet 4 (see Fig. 1(a)) before being bonded to the partition layer 11. This can avoid raising dust or contamination issues, compared to the case of forming holes after being bonded to the partition layer 11.

The method of bonding the partition layer 11 and the cover layer 12 together is not limited to the thermocompression method mentioned above, but other methods may be used, such as a method using an adhesive or a method in which the partition layer 11 and the cover layer 12 are bonded together by providing a surface modification treatment to the bonding surfaces thereof without using an adhesive.

In the case of bonding using an adhesive, the adhesive can be determined based on the affinity thereof for the materials forming the partition layer 11 and the cover layer 12. The adhesive is not particularly limited as long as it can bond the partition layer 11 and the cover layer 12 together. For example, an acrylic resin adhesive, urethane resin adhesive, epoxy resin adhesive, or the like can be used as an adhesive of the present embodiment.

As a bonding method by performing surface modification treatment without using an adhesive, a plasma treatment, corona treatment, excimer laser treatment, or the like can be mentioned. In this case, an optimal treatment may be adequately selected for improving reactivity on the surface of the partition layer 11, according to affinity between the partition layer 11 and the cover layer 12, and adhesion compatibility therebetween. If bonding is performed using a surface modification treatment, processing up to and including bonding between the partition layer 11 and the cover layer 12 may be performed at step S7.

As described above, the method of producing the microfluidic chip 1 according to the present embodiment includes steps of applying a resin onto a substrate 10 (step S1), exposing the applied resin (step S3), developing and cleaning the exposed resin to form a partition layer 11 configuring a channel section 3 on the substrate 10 (steps S4 and S5), post-baking the partition layer 11 configuring the channel section 3 (step S6), and bonding a cover layer 12 to the surface of the partition layer 11 facing away from the substrate 10 (step S8).

A description has been given of an example in which a photosensitive resin is applied onto a substrate 10 to form a partition layer 11 thereon for configuring a channel section 3, using photolithography; however, the present disclosure is not limited to this. The resin used for forming a resin layer serving as the partition layer 11 on the substrate 10 may, for example, be silicone rubber (PDMS) or synthetic resin (PMMA, PC, PS, PP, COP, COC, etc.). For example, if the partition layer 11 is formed using silicone rubber, a channel pattern mold may be formed using photolithography, and the channel pattern of the mold may be transferred to the silicone rubber to form a channel pattern (the partition layer 11 configuring the channel section 3).

### (1.1.3) Method of controlling elastic modulus and Vickers hardness of partition layer

In the present embodiment, when the microfluidic chip 1 is produced using the above production method, the elastic modulus of the partition layer 11 will be in the range of 1 MPa or more and 10 GPa or less, and the Vickers hardness of the partition layer 11 will be 2 HV or more.

### (1.1.3.1) Method of controlling elastic modulus and Vickers hardness by performing post-baking treatment

In the present embodiment, the elastic modulus and Vickers hardness of the partition layer 11 can be controlled using the post-baking treatment (step S6) in the method of producing the microfluidic chip 1. Specifically, the elastic modulus and Vickers hardness of the partition layer 11 are controlled by the temperature and duration of the post-baking treatment. The original purpose of the post-baking treatment is to remove the developer or the moisture generated during cleaning (or to remove solvent of the resin applied to the substrate 10); however, in this case, curing of the partition layer 11 may progress as the moisture is removed. By controlling the progress of this curing, the elastic modulus and Vickers hardness of the partition layer 11 can be controlled.

If the temperature of the post-baking treatment is excessively high or if duration of the post-baking of the post-baking treatment is excessively long, curing of the partition layer 11 may progress more than necessary, and the elastic modulus and Vickers hardness may increase excessively. Therefore, the post-baking treatment is preferred to be performed at an optimal temperature for controlling the elastic modulus and Vickers hardness, that is, at a temperature lower than the glass transition temperature of the resin material forming the partition layer 11 by 30°C to 50°C. Duration of the post-baking treatment is preferred to be 30 minutes or less, and more preferred to be 10 minutes or less. Duration of the post-baking treatment is preferred to be 5 minutes or more.

Thus, by controlling temperature and duration of the post-baking treatment, the elastic modulus of the partition layer 11 can be controlled to the range of 1 MPa or more and 10 GP a or less. The Vickers hardness of the partition layer can be further controlled to 2 HV or more.

### (1.1.3.2) Method of controlling elastic modulus and Vickers hardness by exposure

In the present embodiment, the elastic modulus and Vickers hardness of the partition layer 11 can also be controlled by the UV exposure dose in the step of exposing the resin on the substrate 10 when producing the microfluidic chip 1. The exposure step (e.g., step S3) when producing the microfluidic chip 1 is originally performed for the purpose of forming the channel section 3 (channel pattern) defined within the partition layer 11. However, for example, in a radical polymerization type negative photosensitive resin, curing of the photosensitive region may progress during exposure. By controlling the progress of this curing, the elastic modulus and Vickers hardness of the partition layer 11 can be controlled.

It has been confirmed that, if the exposure dose is excessive, the elastic modulus and Vickers hardness of the partition layer 11 excessively increase. Also, an excessive exposure dose may cause such problems as deterioration in pattern resolution or generation of residues, or significant deviation of the pattern dimensions from the designed values. Similarly, if the exposure dose is insufficient, the pattern dimensions may deviate significantly from the designed values, or the channel pattern may not be formed. Therefore, an appropriate exposure dose for forming a channel pattern is determined first in the exposure step (first exposure step) of forming a channel pattern. Herein, the exposure dose is adjusted to control the elastic modulus and Vickers hardness of the resin applied to the substrate 10, within the range not causing any problem in channel pattern formation. Then, an exposure step (second exposure step) for controlling the elastic modulus and Vickers hardness of the partition layer 11 is performed for the purpose of suppressing poor bonding between the partition layer 11 forming the channel section 3 and the cover layer 12 and suppressing deformation of the channel section 3. The exposure dose in the second exposure step is required to be adequately determined according to the resin material of the partition layer 11, but is preferred to be higher than the exposure dose in the first exposure step of forming a channel pattern and is also preferred to be in the range of 10 mJ/cm² or more and 1,000 mJ/cm² or less.

The second exposure step may be performed following the first exposure step (as the treatment of step S3) for the channel pattern before development, or may be performed for the channel pattern after development but before performing the post-baking treatment (step S6).

For the first and second exposure steps, a proximity exposure device may be used, for example. In the second exposure step also, the channel pattern may be exposed to light with a wavelength (190 nm or more and 400 nm or less) as in step S3.

In this way, the elastic modulus of the partition layer 11 can be controlled to 1 MPa or more and 10 GPa or less by performing the second exposure step described above when producing the microfluidic chip 1 of the present disclosure. The Vickers hardness of the partition layer can be further controlled to 2 HV or more.

The elastic modulus of the partition layer 11 can also change depending on the resin material. For example, if the partition layer 11 is formed using silicone rubber (PDMS), the elastic modulus will be around 10 MPa and the Vickers hardness will be around 0.1 HV If the partition layer 11 is formed using a photosensitive resin, the elastic modulus will be 2 GPa or more and 10 GPa or less and the Vickers hardness will be around 40 HV

In this way, according to the present disclosure, there can be produced a microfluidic chip 1 in which the elastic modulus and Vickers hardness of the partition layer 11, in which a channel has been formed using photolithography, are controlled for the partition layer 11 to conform to slight irregularities at the bonding interface and enable bonding in a state in which deformation of fine channel is suppressed.

Specifically, by controlling the elastic modulus and Vickers hardness when producing the microfluidic chip 1 as described above, there can be provided a microfluidic chip 1 including a substrate 10, a partition layer 11 configured by a resin material and provided on the substrate 10 to form a channel section 3, and a cover layer 12 provided on the surface of the partition layer 11 facing away from the substrate 10, in which the partition layer 11 has an elastic modulus in the range of 1 MPa or more and 10 GPa or less. Thus, there can be provided a microfluidic chip that can suppress occurrence of poor bonding between the resin layer forming a channel and the lid component, and can suppress deformation of the channel.

### (1.1.4) Method of measuring elastic modulus and Vickers hardness

It is preferred that the elastic modulus and Vickers hardness of the partition layer 11 and the cover layer 12 are measured using a microindentation tester. The microindentation tester can perform quasi-static indentation tests on specimens to obtain physical properties of the specimens. With the microindentation tester, information, such as the hardness, elastic modulus, and power of plastic deformation and elastic deformation, can be obtained and analyzed by analyzing the indentation depth or its curve when a maximum load is applied to the specimens.

For example, with the microindentation tester, indentation testing may be performed on a specimen of a partition layer 11 with an indentation load of 2 mN using a square pyramid indenter with a face angle of 136° to measure the elastic modulus and Vickers hardness of the partition layer 11. An elastic modulus, which is the hardness in an elastic deformation region, is calculated from the slope of the measurement curve at unloading. Furthermore, an indentation may be estimated from the push amount of the indenter to calculate a Vickers hardness which is the hardness in a plastic deformation region. For the cover layer 12 also, the elastic modulus and Vickers hardness can be similarly measured.

### (1.1.5) Effects of first embodiment

The microfluidic chip 1 described above has the following effects.
(1) The microfluidic chip 1 according to the present embodiment includes a substrate 10, a partition layer 11 configured by a resin material and provided on the substrate 10 to form a channel section 3, and a cover layer 12 provided on the surface of the partition layer 11 facing away from the substrate 10, wherein the partition layer 11 has an elastic modulus in the range of 1 MPa or more and 10 GPa or less.

Thus, occurrence of poor bonding can be suppressed between the resin layer 11 forming the channel section 3 and the cover layer 12 as a lid component, and deformation of the channel section 3 can be suppressed.

(2) The microfluidic chip 1 according to the present embodiment includes a substrate 10, a partition layer 11 configured by a resin material and provided on the substrate 10 to form a channel section 3, and a cover layer 12 provided on the surface of the partition layer 11 facing away from the substrate 10, wherein the partition layer 11 may have an elastic modulus in the range of greater than 2 GPa and less than or equal to 10 GPa.

Thus, occurrence of poor bonding can be suppressed between the resin layer 11 forming the channel section 3 and the cover layer 12 as a lid component, and deformation of the channel section 3 can be suppressed.

(3) The microfluidic chip 1 according to the present embodiment includes a substrate 10, a partition layer 11 configured by a resin material and provided on the substrate 10 to form a channel section 3, and a cover layer 12 provided on the surface of the partition layer 11 facing away from the substrate 10, wherein the partition layer 11 may have an elastic modulus in the range of 1 MPa or more and 2 GPa or less and a Vickers hardness of 2 HV or more.

Thus, occurrence of poor bonding can be suppressed between the resin layer 11 forming the channel section 3 and the cover layer 12 as a lid component, and deformation of the channel section 3 can be suppressed.

(4) In the microfluidic chip 1 according to the present embodiment, the partition layer is preferred to have a Vickers hardness of 2 HV or more. Thus, plastic deformation is less likely to occur in the partition layer 11, and therefore, deformation or blocking of the channel section 3 can be more reliably suppressed.

(5) In the microfluidic chip 1 according to the present embodiment, the cover layer 12 may have an elastic modulus that is greater than that of the partition layer 11.

Thus, the cover layer 12 will be improved in physical strength more than that of the partition layer 11 and thus will be improved in durability against external forces applied when bonded to the partition layer 11.

(6) In the microfluidic chip 1 according to the present embodiment, the cover layer may have an elastic modulus that is smaller than that of the partition layer.

Thus, the cover layer 12 becomes softer than the partition layer 11 and will be improved in conformability at the bonding interface between the partition layer 11 and the cover layer 12 to further suppress poor bonding.

(7) In the microfluidic chip 1 according to the present embodiment, the resin material forming the partition layer 11 is preferred to be a photosensitive resin having photosensitivity to light with a wavelength of 190 nm or more and 400 nm or less in the UV light region.

Thus, a partition layer 11, which configures a channel section 3 on the substrate 10, can be formed by photolithography.

(8) The method of producing a microfluidic chip 1 according to the present embodiment includes steps of applying a resin onto a substrate 10, exposing the applied resin, developing and cleaning the exposed resin to form a partition layer 11 defining a channel section 3 on the substrate 10, post-baking the partition layer 11, and bonding a cover layer 12 to the surface of the partition layer 11 facing away from the substrate 10, wherein the partition layer 11 has an elastic modulus in the range of 1 MPa or more and 10 GPa or less.

Thus, there can be provided a microfluidic chip that can suppress occurrence of poor bonding between the partition layer 11 that is a resin layer forming the channel section 3 and the cover layer 12 that is a lid component, and can suppress deformation of the channel section 3.

(9) In the microfluidic chip 1 according to the present embodiment, the partition layer 11 has a Vickers hardness of 2 HV or more.

Thus, there can be provided a microfluidic chip in which plastic deformation is less likely to occur in the partition layer 11, and deformation or blocking of the channel section 3 can be more reliably suppressed.

(10) In the method of producing a microfluidic chip 1 according to the present embodiment, the elastic modulus and Vickers hardness of the partition layer 11 are controlled by performing post-baking treatment.

Thus, the elastic modulus and Vickers hardness can be controlled by photolithography.

(11) In the method of producing a microfluidic chip 1 according to the present embodiment, the elastic modulus and Vickers hardness of the partition layer 11 are controlled by UV exposure dose in the step of exposing the resin.

Thus, the elastic modulus and Vickers hardness can be controlled by photolithography.

(12) In the method of producing a microfluidic chip 1 according to the present embodiment, the photosensitive resin is exposed to light with a wavelength of 190 nm or more and 400 nm or less in the UV light region, in the step of exposing the resin.

Thus, a partition layer 11, which configures a channel section 3 on the substrate 10, can be formed by photolithography.

### (1.2) Embodiment 1-2

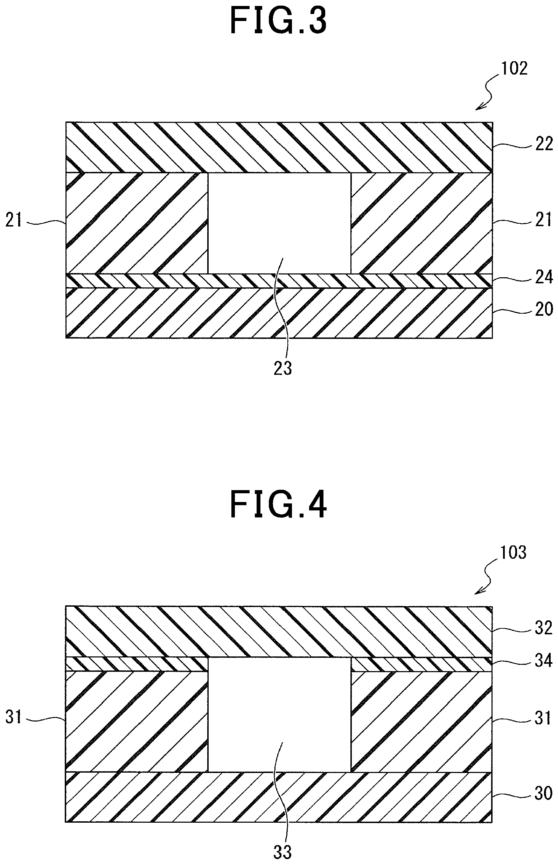

Referring to Fig. 3, a microfluidic chip according to Embodiment 1-2 of the present disclosure will be described. Fig. 3 is a cross-sectional view illustrating a configuration example of a microfluidic chip 102 according to Embodiment 1-2 of the present disclosure.

The microfluidic chip 102 includes a substrate 20, an adhesive layer 24 arranged on the substrate 20, a partition layer 21 forming a channel section 23 on the substrate 20, and a cover layer 22. In other words, the microfluidic chip 102 is different from the microfluidic chip 1 of Embodiment 1-1 in that the adhesive layer 24 is provided between the substrate and the partition layer.

The adhesive layer 24 will be described below. Since the configurations of the components (substrate 20, partition layer 21, cover layer 22, and channel section 23) other than the adhesive layer 24 are similar to those of the substrate 10, partition layer 11, cover layer 12, and channel section 3 of the microfluidic chip 1, explanation will be omitted.

For the purpose of increasing adhesion between the substrate 10 and a resin layer (e.g., photosensitive resin layer) in the microfluidic chip 102, the substrate 20 may undergo a hydrophobization surface treatment (HMDS treatment) or may be provided with a thin resin coating. If glass is used for the substrate 10, in particular, a thin-film adhesive layer 24 may be provided, as shown in Fig. 3, between the substrate 20 and the partition layer 21 (photosensitive resin layer). In this case, the fluid (e.g., liquid) passing through the channel section 23 contacts the adhesive layer 24, not the substrate. Therefore, the adhesive layer 24 may have resistance to the fluid introduced into the channel section 23. Provision of the adhesive layer 24 on the substrate 20 can contribute, for example, to improving resolution of the channel pattern formed in the photosensitive resin.

### (1.3) Embodiment 1-3

Referring to Fig. 4, a microfluidic chip according to Embodiment 1-3 of the present disclosure will be described. Fig. 4 is a cross-sectional view illustrating a configuration example of a microfluidic chip 103 according to Embodiment 1-3 of the present disclosure.

The microfluidic chip 103 includes a substrate 30, a partition layer 31 forming a channel section 33 on the substrate 30, a cover layer 32, and an adhesive layer 34 provided between the partition layer 31 and the cover layer 32. In other words, the microfluidic chip 103 is different from the microfluidic chip 1 of Embodiment 1-1 in that it is provided with the adhesive layer 34.

The adhesive layer 34 will be described below. Since the configurations of the components (substrate 30, partition layer 31, cover layer 32, and channel section 33) other than the adhesive layer 34 are similar to those of the substrate 10, partition layer 11, cover layer 12, and channel section 3 of the microfluidic chip 1, explanation will be omitted.

If an adhesive is used for bonding between the partition layer and the cover layer, the adhesive may be applied onto the partition layer 31, as shown in Fig. 4, to form an adhesive layer 34 between the partition layer 31 and the cover layer 32. In this case, the fluid (e.g., liquid) passing through the channel section 33 contacts the adhesive layer 34. Therefore, the adhesive layer 34 may have resistance to the fluid introduced into the channel section 33. Provision of the adhesive layer 34 can strengthen the adhesion between the partition layer 31 and the cover layer 32 and can improve durability of the microfluidic chip 103.

### <First Example>

The following description relates to specific examples of the microfluidic chip according to the first embodiment of the present disclosure.

The first embodiment of the present disclosure should not be limited to the following examples.

### (Example 1)

First, a transparent photosensitive resin was applied onto a glass substrate to form a photosensitive resin layer. PDMS was used as the photosensitive resin. The photosensitive resin was applied to the glass substrate using a spin coater set to 1,100 rpm and 30 sec. The rotation speed and time were adjusted so that the film thickness would be 50 µm. Next, the photosensitive resin underwent heat treatment (pre-baking) on a hot plate for the purpose of removing residual solvent contained therein. Pre-baking was performed at 90°C for 20 minutes.

Next, the photosensitive resin layer on the glass substrate was exposed to draw a channel pattern. Specifically, the photosensitive resin was subjected to pattern exposure via a photomask having a microchannel pattern array. The photomask was formed of a light-shielding film with a double-layer structure of chromium and chromium oxide. A proximity exposure device was used for the exposure. The exposure device had a high-pressure mercury lamp as a light source, and the exposure wavelength was broadband including g-, h-, and i-lines. The exposure dose in the exposure for forming the channel pattern (first exposure step) was 100 mJ/cm².

Next, the exposed photosensitive resin layer was developed to form a channel pattern. Specifically, the photosensitive resin layer was developed for 60 seconds using an alkaline developer (TMAH 2.38%) to dissolve the unexposed portions and to pattern a channel structure.

Subsequently, shower cleaning was performed using ultra-pure water to remove the developer from the photosensitive resin layer on the substrate, followed by drying using a spin dryer.

Next, an exposure step (second exposure step) was performed to control the elastic modulus and Vickers hardness. The exposure dose in the second exposure step was 50 mJ/cm².

Next, the channel pattern was subjected to heat treatment (post-baking) in an oven at 100°C for 10 minutes. Thus, a channel section (channel pattern) was formed, being defined within the partition layer. The minimum width between opposing parts of the partition layer, i.e., the minimum width of the channel, was set to 50 µm.

After post-baking, the elastic modulus and Vickers hardness of the partition layer were measured. A microindentation tester (HM2000) was used for the measurement. Specifically, the elastic modulus and Vickers hardness were measured with an indentation load of 2 mN using a square pyramid indenter with a face angle of 136°. The elastic modulus, i.e., the hardness in the elastic deformation region, was measured from the slope of the measurement curve at unloading, and the Vickers hardness, i.e., the hardness in the plastic deformation region, was measured by estimating an indentation from the push amount of the indenter. The elastic modulus was measured to be 0.001 GPa (1 MPa). The Vickers hardness was measured to be 0.008 HV

Next, a surface modification treatment was performed on the bonding surfaces of the partition layer forming the channel pattern and a separately prepared cover layer. For the cover layer, PMMA was used, which was provided with holes for inlet and outlet of the channel in advance. As the surface modification treatment, a plasma treatment was performed. Plasma treatment was performed for 30 seconds at a pressure of 10 Pa, oxygen gas flow rate of 10 sccm, and RF power of 100 W.

Next, the partition layer forming the channel pattern was bonded to the cover layer using a thermocompression machine. Thermocompression was performed for 30 seconds at a temperature of 100°C and a compressive force of 5 kN. Thus, a microfluidic chip of the present example was obtained.

### (Example 2)

A urethane acrylate resin was used as a photosensitive resin applied to a glass substrate. The exposure dose in the second exposure step using a proximity exposure device was set to 100 mJ/cm². Other than this, a microfluidic chip of the present example was obtained as in Example 1.

In the microfluidic chip of the present example, the elastic modulus and Vickers hardness were measured as in Example 1 after post-baking. In the present example, the elastic modulus was measured to be 0.25 GPa. The Vickers hardness was measured to be 2 HV

### (Example 3)

A urethane acrylate resin similar to Example 2 was used as a photosensitive resin applied to a glass substrate. The exposure dose in the second exposure step using a proximity exposure device was set to 170 mJ/cm² and the post-baking temperature was set to 120°C. Other than this, a microfluidic chip of the present example was obtained as in Example 1.

In the microfluidic chip of the present example, the elastic modulus and Vickers hardness were measured as in Example 1 after post-baking. In the present example, the elastic modulus was measured to be 0.7 GPa. The Vickers hardness was measured to be 0.8 HV.

### (Example 4)

An acrylic resin was used as a photosensitive resin applied to a glass substrate. The post-baking temperature was set to 150°C. Other than this, a microfluidic chip of the present example was obtained as in Example 1.

In the present example, the elastic modulus was measured to be 1.5 GPa. The Vickers hardness was measured to be 1.3 HV

### (Example 5)

An acrylic resin similar to Example 4 was used as a photosensitive resin applied to a glass substrate. The exposure dose in the second exposure step using a proximity exposure device was set to 130 mJ/cm² and the post-baking temperature was set to 120°C. Other than this, a microfluidic chip of the present example was obtained as in Example 1.

In the present example, the elastic modulus was measured to be 2.5 GPa. The Vickers hardness was measured to be 20 HV

### (Example 6)

An acrylic resin as in Example 4 was used as a photosensitive resin applied to a glass substrate. The exposure dose in the second exposure step using a proximity exposure device was set to 170 mJ/cm² and the post-baking temperature was set to 150°C. Other than this, a microfluidic chip of the present example was obtained as in Example 1.

In the present example, the elastic modulus was measured to be 5 GPa. The Vickers hardness was measured to be 40 HV

### (Example 7)

An acrylic resin as in Example 4 was used as a photosensitive resin applied to a glass substrate. The exposure dose in the second exposure step using a proximity exposure device was set to 170 mJ/cm² and the post-baking temperature was set to 150°C. Other than this, a microfluidic chip of the present example was obtained as in Example 1.

In the present example, the elastic modulus was measured to be 5.1 GPa. The Vickers hardness was measured to be 42 HV

### (Example 8)

A negative liquid resin (negative liquid resist) of an epoxy resin was used as a photosensitive resin applied to a glass substrate. The exposure dose in the second exposure step using a proximity exposure device was set to 500 mJ/cm² and the post-baking temperature was set to 250°C. Other than this, a microfluidic chip of the present example was obtained as in Example 1.

In the present example, the elastic modulus was measured to be 10 GPa. The Vickers hardness was measured to be 80 HV

### (Comparative Example 1)

A microfluidic chip of the present comparative example was obtained as in Example 8, except that the post-baking temperature was set to 300°C.

In the present comparative example, the elastic modulus was measured to be 11.7 GPa. The Vickers hardness was measured to be 20.8 HV

### (Comparative Example 2)

A microfluidic chip of the present comparative example was obtained as in Comparative Example 1, except that the exposure dose in the second exposure step using a proximity exposure device was set to 600 mJ/cm².

In the present comparative example, the elastic modulus was measured to be 14.2 GPa. The Vickers hardness was measured to be 30.4 HV

### <Evaluation>

### (Bonding condition testing)

10 µL of a colored reaction solution was collected with a pipette and introduced into each of the microfluidic chips of Examples 1 to 8 and Comparative Examples 1 and 2 from the inlet of the channel, and the state of liquid transfer was microscopically observed.

Based on the observation, the bonding condition between the partition layer and the cover layer of each microfluidic chip was evaluated in two grades of Good and Poor according to the following criteria.

### <Criteria>

Good: Liquid leakage from the channel was not observed at the bonding portion between the partition layer and the cover layer.

Poor: Liquid leakage from the channel was observed at the bonding portion between the partition layer and the cover layer.

### (Bead passage testing)

The microfluidic chips of Examples 1 to 8 and Comparative Examples 1 and 2 were each subjected to bead passage testing as follows.

30 parts by mass of microbeads with a diameter of 40 µm were added to 100 parts by mass of a colored reaction solution and stirred. 10 µL of the reaction solution was collected using a pipette so that 10 or more microbeads are contained, and the collected reaction solution was microscopically observed to count the number of the microbeads in the solution. After that, the reaction solution in the pipette was introduced into the channel from the inlet of the microfluidic chip and transferred to the outlet. The reaction solution was recovered from the outlet and microscopically observed to again count the number of the microbeads therein.

Based on the recount, the bead passage condition (degree of retention due to channel deformation) in each microfluidic chip was evaluated in three grades of Good, Fair and Poor.

### <Criteria>

Good: The number of the microbeads in the solution recovered from the outlet was 90% or more of the number counted before transfer.

Fair: The number of the microbeads in the solution recovered from the outlet was 70% or more of the number counted before transfer.

Poor: The number of the microbeads in the solution recovered from the outlet was less than 70% of the number counted before transfer.

Evaluations of the examples and comparative examples in the First Example are shown in Table 1 together with the processing conditions of the microfluidic chips of the First Example.

**[Table 1]**

| | Processing conditions | | | | | Evaluation | | | |
|---|---|---|---|---|---|---|---|---|---|
| | Control of elastic modulus and Vickers hardness | | Exposure dose (mJ/cm²) | Post-baking temperature (°C) | Resin material of partition layer | Elastic modulus (GPa) | Vickers hardness (HV) | Bonding conditions (Liquid leakage prevention) | Bead passability (Channel modification) |
| | Post-baking | Exposure | | | | | | | |
| Ex. 1 | Good | Good | 50 | 100 | PDMS | 0.001 | 0.008 | Good | Fair |
| Ex. 2 | Good | Good | 100 | 100 | Urethane acrylate | 0.25 | 2.0 | Good | Good |
| Ex. 3 | Good | Good | 170 | 120 | Urethane acrylate | 0.7 | 0.8 | Good | Fair |
| Ex. 4 | Good | Good | 50 | 150 | Acrylic resin | 1.5 | 1.3 | Good | Fair |
| Ex. 5 | Good | Good | 130 | 120 | Acrylic resin | 2.5 | 20.0 | Good | Good |
| Ex. 6 | Good | Good | 170 | 150 | Acrylic resin | 5.0 | 40.0 | Good | Good |
| Ex. 7 | Good | Good | 170 | 150 | Acrylic resin | 5.1 | 42.0 | Good | Good |
| Ex. 8 | Good | Good | 500 | 250 | Epoxy resin | 10.0 | 80.0 | Good | Good |
| Comp. Ex. 1 | Good | Good | 500 | 300 | Epoxy resin | 11.7 | 20.8 | Poor | Good |
| Comp. Ex. 2 | Good | Good | 600 | 300 | Epoxy resin | 14.2 | 30.4 | Poor | Good |

As shown in Table 1, as a result of the bonding condition testing, the microfluidic chips of Examples 1 to 8 were all evaluated to be above a pass level (Good), i.e., there was no liquid leakage from the channel and liquid transfer was good. Also, as a result of the bead passage testing, the microfluidic chips of Examples 1 to 8 were all evaluated to be at least at a pass level (Fair), i.e., the influence of bead retention due to the channel being deformed, with the partition layer reduced in height (plastic deformation), was limited, and there was no practical problem. As a result of the bead passage testing, Examples 2 and 5 to 8 were evaluated to be Good. In other words, it was found that the microfluidic chips of Examples 1 to 8 could suppress occurrence of poor bonding between the resin layer forming the channel and the lid component, and could suppress deformation of the channel.

However, as a result of the bonding condition testing, the microfluidic chips of Comparative Examples 1 and 2 were both evaluated to be below the pass level (Poor). This is because, in Comparative Examples 1 and 2, the elastic modulus of the partition layer was excessively large (exceeding 10 GP), and therefore, the partition layer could not conform to slight irregularities at the bonding interface, which caused gaps when bonded to the cover layer, allowing liquid leakage to occur from the channel at the bonding portion between the partition layer and the cover layer.

Consequently, it was confirmed that the microfluidic chips of the First Example could suppress occurrence of poor bonding between the resin layer forming the channel and the lid component, and could suppress deformation of the channel, by controlling the elastic modulus and Vickers hardness of the partition layer.

Specifically, it was found that, as in the microfluidic chips of Examples 1 to 8, a partition layer having an elastic modulus in the range of 1 MPa or more and 10 PGa or less could suppress occurrence of poor bonding between the resin layer forming the channel and the lid component, and could suppress deformation of the channel. Also, it was found that a partition layer having an elastic modulus in the range of greater than 2 GPa and less than or equal to 10 GPa could also suppress occurrence of poor bonding between the resin layer forming the channel and the lid component, and could suppress deformation of the channel. Furthermore, it was found that, as in the microfluidic chip of Example 2, a partition layer having an elastic modulus in the range of 1 MPa or more and 2 PGa or less and a Vickers hardness of 2 HV or more could also suppress occurrence of poor bonding between the resin layer forming the channel and the lid component, and could suppress deformation of the channel.

The results of the bead passage testing of Examples 1, 3 and 4 were different from the results of Examples 2 and 5 to 8. This is assumed to be due to the fact that the Vickers hardness of the latter examples was 2HV or more. In other words, it is assumed that, with the Vickers hardness being 2 HV or more, plastic deformation of the partition layer was more reliably suppressed and this worked in favor of the evaluation for channel deformation suppression (bead passability).

For example, the first embodiment can have the following configurations.
(1) A microfluidic chip including
   a substrate;
   a partition layer that is configured by a resin material and provided on the substrate to form a channel; and
   a cover layer that is provided on the surface of the partition layer facing away from the substrate, wherein
   the partition layer has an elastic modulus in the range of 1 MPa or more and 10 GPa or less.
(2) A microfluidic chip including
   a substrate;
   a partition layer that is configured by a resin material and provided on the substrate to form a channel; and
   a cover layer that is provided on the surface of the partition layer facing away from the substrate, wherein
   the partition layer has an elastic modulus in the range of greater than 2 GPa and less than or equal to 10 GPa.
(3) A microfluidic chip including
   a substrate;
   a partition layer that is configured by a resin material and provided on the substrate to form a channel; and
   a cover layer that is provided on the surface of the partition layer facing away from the substrate, wherein
   the partition layer has an elastic modulus in the range of 1 MPa or more and 2 GPa or less; and
   the partition layer has a Vickers hardness of 2 HV or more.
(4) The microfluidic chip according to (1) or (2), wherein
   the partition layer has a Vickers hardness of 2 HV or more.
(5) The microfluidic chip according to any one of (1) to (4), wherein
   the cover layer has an elastic modulus greater than that of the partition layer.
(6) The microfluidic chip according to any one of (1) to (4), wherein
   the cover layer has an elastic modulus smaller than that of the partition layer.
(7) The microfluidic chip according to any one of (1) to (6), wherein
   the resin material forming the partition layer is a photosensitive resin having photosensitivity to light having a wavelength of 190 nm or more and 400 nm or less in the UV light region.
(8) A method of producing a microfluidic chip, including steps of
   applying a resin onto a substrate;
   exposing the applied resin;
   developing and cleaning the exposed resin to form a partition layer defining a channel on the substrate;
   performing a post-baking treatment on the partition layer; and
   bonding a cover layer to the surface of the partition layer facing away from the substrate, wherein
   the partition layer has an elastic modulus in the range of 1 MPa or more and 10 GPa or less.
(9) The method of producing a microfluidic chip according to (8), wherein
   the partition layer has a Vickers hardness of 2 HV or more.
(10) The method of producing a microfluidic chip according to (9), wherein
   the elastic modulus and the Vickers hardness of the partition layer are controlled by the post-baking treatment.
(11) The method of producing a microfluidic chip according to (9) or (10), wherein
   the elastic modulus and the Vickers hardness of the partition layer are controlled by an exposure dose of UV light in the step of exposing the resin.
(12) The method of producing a microfluidic chip according to any one of (8) to (11), wherein
   a photosensitive rein is exposed to light having a wavelength of 190 nm or more and 400 nm or less in the UV light region, in the step of exposing the resin.

### 2. Second embodiment

A second embodiment of the present disclosure will be described. The second embodiment of the present disclosure relates to a microfluidic chip and a method of producing a microfluidic chip.

As mentioned above, in µ-TAS, micrometer-sized channels (microchannels) formed on a substrate are often used. Such substrates are called chips, microchips, microfluidic chips, etc.

Such microfluidic chips based on the conventional art have been prepared, for example, by bonding multiple structural components together, such as glass, plastic, resin, and metal. In generally used methods, these structural components are bonded together, with intermediate components other than these structural components interposed therebetween. As the intermediate materials, adhesives have been used. In this case, after forming a channel on the surface of a first structural component (e.g., substrate), an adhesive is applied to the surface of the partition forming the channel, and a second structural component serving as a lid for the channel is bonded to the first structural component to form a microfluidic chip.

The conventional microfluidic chips, which are formed using a method of bonding structural components together via an adhesive, raise an issue that the adhesive components may elute into the solution passing through the microchannel and inhibit reactions of the solution.

Therefore, in recent years, a lid for microchannels is required to be formed without using an adhesive, i.e., omitting an adhesive, so that adhesive components are prevented from eluting into the channel.

The second embodiment of the present disclosure aims to provide a microfluidic chip and to provide a method of producing the microfluidic chip, in which adhesive components are prevented from eluting into the channel and inhibiting reactions of the solution in the channel.

In order to achieve the above aim, a microfluidic chip according to an aspect of the second embodiment includes a base section, a partition section forming a channel on the base section, and an upper lid section formed on the surface of the partition section facing away from the base section to serve as a lid for the channel, wherein no adhesive layer is provided between the partition section and the upper lid section.

In order to achieve the above aim, a microfluidic chip according to another aspect of the second embodiment includes a base section, a partition section forming a channel on the base section, and an upper lid section formed on the surface of the partition layer facing away from the substrate to serve as a lid for the channel, wherein the upper lid section and the partition section are joined together by welding.

In order to achieve the above aim, a microfluidic chip according to still another aspect of the second embodiment includes a channel and an upper lid section serving as a lid for the channel, wherein the material of the upper lid section is a resin having thermal fluidity, and the channel has a cross section with rounded corners.

A method of producing a microfluidic chip according to an aspect of the second embodiment includes steps of applying a photosensitive resin onto a base section; exposing the applied photosensitive resin; developing and cleaning the exposed photosensitive resin to form a partition section that defines a channel on the base section; and heat-treating the partition section and fluidizing the photosensitive resin to form an upper lid section for the channel.

A method of producing a microfluidic chip according to another aspect of the second embodiment includes steps of applying a first photosensitive resin onto a base section; applying a second photosensitive resin onto the applied first photosensitive resin; exposing the first photosensitive resin and the second photosensitive resin; developing and cleaning the exposed first photosensitive resin and the exposed second photosensitive resin to form a partition section that defines a channel on the base section; and heat-treating and fluidizing the second photosensitive resin on the partition section to form an upper lid section for the channel.

According to an aspect of the second embodiment of the present disclosure, there can be provided a microfluidic chip, in which adhesive components are prevented from eluting into the channel and inhibiting reactions of the solution in the channel.

A microfluidic chip according to the second embodiment of the present disclosure will be described. In the following description, the substrate side of the microfluidic chip may be referred to as lower, and the opposite side of the microfluidic chip from the substrate side (lid component side) may be referred to as upper.

As a result of diligent studies, the present inventors have found that, in a microfluidic chip, an upper lid can be provided on the partition section without using an adhesive, by forming an upper lid section serving as a lid for the channel section by heating and fluidizing a resin material. Thus, the present inventors have invented a microfluidic chip and a method of producing the microfluidic chip, in which adhesive components are prevented from eluting into the channel and inhibiting reactions of the solution in the channel.

Referring to the drawings, some modes (Embodiments 2-1 and 2-2) of the second embodiment of the present disclosure will be described.

### (2.1) Embodiment 2-1

### (2. 1. 1) Basic configuration of microfluidic chip

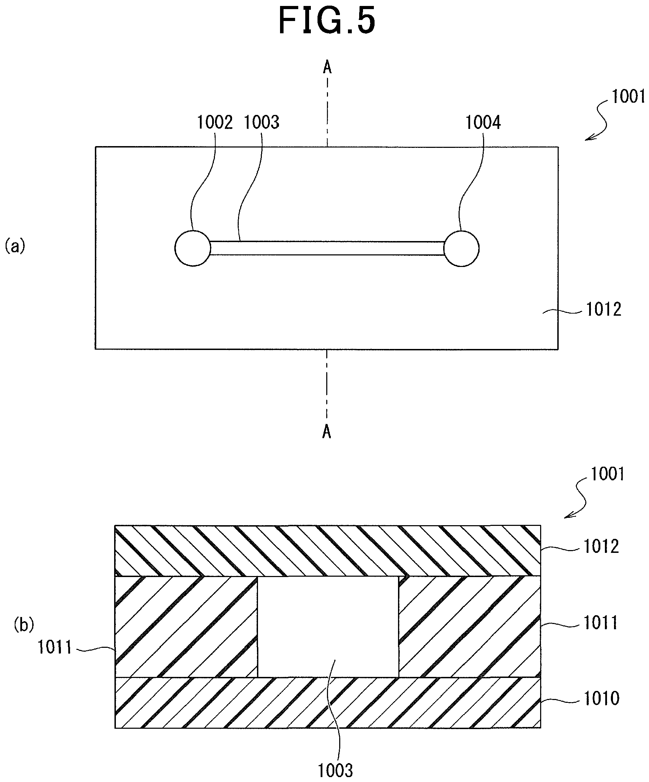

Fig. 5 is a set of schematic diagrams illustrating a configuration example of a microfluidic chip 1001 according to Embodiment 2-1 of the present disclosure (hereinafter referred to as the present embodiment). Specifically, Fig. 5(a) is a schematic plan view illustrating the microfluidic chip 1001 according to the present embodiment. Fig. 5(b) is a schematic cross-sectional view illustrating a cross section of the microfluidic chip 1001 taken along the line A-A of Fig. 5(a).

As shown in Fig. 5(a), the microfluidic chip 1001 includes an inlet 1002 for introducing a fluid (e.g., liquid), a channel section 1003 through which the fluid introduced from the inlet 1002 flows, and an outlet 1004 for discharging the fluid from the channel section 1003. In the microfluidic chip 1001, the upper part of the channel section 1003 is covered with an upper lid layer 1012, and the inlet 1002 and the outlet 1004 are through-holes provided in the upper lid layer 1012. Details of the upper lid layer 1012 will be described later.

Fig. 5(a) shows the channel section 1003 that can be seen through a transparent upper lid layer 1012.

The microfluidic chip 1001 may be provided with at least one or more inlets 1002 and outlets 1004, or may be provided with multiple of each. The microfluidic chip 1001 may be provided with multiple channel sections 1003, or may be designed such that the fluid introduced from the inlet 1002 can be merged or separated.

Details of the structural components of the channel section 1003 of the microfluidic chip 1001 will be described later. As shown in Fig. 5(b), the microfluidic chip 1001 includes a substrate (example of the base section) 1010, a partition layer (example of the partition section) 1011 forming a channel on the substrate 1010, and an upper lid layer (example of the upper lid section) 1012 formed on the surface of the partition layer 1011 facing away from the substrate 1010 to serve as a lid for the channel section 1003.

In the microfluidic chip 1001, the channel section 1003 through which the fluid introduced from the inlet 1002 flows is a region surrounded by the substrate 1010, partition layer 1011, and upper lid layer 1012. The channel section 1003 is defined within the partition layer 1011 provided on the substrate 1010 and is covered with the upper lid layer 1012, as a lid component, provided on the side opposite from the substrate 1010. In other words, the channel section 1003 is a space configured by the substrate 1010, partition layer 1011, and upper lid layer 1012. As mentioned above, a fluid is introduced into the channel section 1003 from the inlet 1002 (see Fig. 5(a)) provided to the upper lid layer 1012, and the fluid that has passed through the channel section 1003 is discharged from the outlet 1004.

The microfluidic chip 1001 of the present embodiment includes no adhesive layer between the partition layer 1011 and the upper lid layer 1012, details of which will be described later. In the present embodiment, the partition layer 1011 and the upper lid layer 1012 are joined together by welding. Herein, the adhesive layer refers to a layer containing an adhesive and used for bonding multiple structural components together. In the microfluidic chip 1001, the partition layer 1011 and the upper lid layer 1012 are joined together by welding (bonded together) without providing an adhesive layer therebetween, so that adhesive components are prevented from eluting into the channel and inhibiting reactions of the solution in the channel.

As shown in Fig. 5(b), the partition layer 1011 and the upper lid layer 1012 are separate bodies. Herein, separate bodies refers to the partition layer 1011 and the upper lid layer 1012, for example, being formed of different resin materials. In this case, as a resin material for forming the upper lid layer 1012, a material having favorable characteristics, such as having glass transition temperature or exposure sensitivity different from those of the partition layer 1011, can be adequately selected. The present disclosure is not limited to this, but the partition layer 1011 and the upper lid layer 1012 may be defined to be separate bodies based on these layers forming a laminated structure with an interface formed therebetween. In this case, the partition layer 1011 and the upper lid layer 1012 may be formed using the same resin material.

### (2.1.1.1) Substrate

The substrate 1010 serves as a base of the microfluidic chip 1001, and the channel section 1003 is configured by the partition layer 1011 provided on the substrate 1010. In other words, the substrate 1010 and the partition layer 1011 configure a body of the microfluidic chip 1001.

The substrate 1010 can be formed using either a translucent material or a non-translucent material. For example, if the state inside the channel section1003 (state of the fluid) is detected and observed using light, a material having good transparency to light can be used. As the translucent material, resins, glass, etc. can be used. Examples of the resin used as a translucent material for forming the substrate 1010 include acrylic resins, methacrylic resins, polypropylenes, polycarbonate resins, cycloolefin resins, polystyrene resins, polyester resins, urethane resins, silicone resins, and fluororesins, which are preferred from the perspective of suitability for forming the body of the microfluidic chip 1001.

For example, if the state inside the channel section 1003 (state of the fluid) is not required to be detected and observed using light, a non-translucent material may be used. Examples of non-translucent materials include silicon wafers and copper plates. The thickness of the substrate 1010 is not particularly limited, but is preferred to be in the range of 10 µm (0.01 mm) or more and 10 mm or less because some degree of rigidity is required in the channel formation process.

### (2.1.1.2) Partition layer

The partition layer 1011 is provided on the substrate to form the channel section 1003. The partition layer 1011 can be formed using a resin material. For example, a photosensitive resin can be used as the resin material of the partition layer 1011.

The photosensitive resin forming the partition layer 1011 is preferred to have photosensitivity to light having a wavelength of 190 nm or more and 400 nm or less in the UV light region. As the photosensitive resin, photoresists such as liquid resists and dry film resists can be used. These photosensitive resins may be of positive type with which the photosensitive area becomes soluble, or may be of negative type with which the photosensitive area becomes insoluble. Examples of the photosensitive resin composition suitable for forming the partition layer 1011 in the microfluidic chip 1001 include negative radical type photosensitive resins containing alkali-soluble polymers, addition polymerizable monomers, and photopolymerization initiators. Examples of the photosensitive resin materials include acrylic resins, epoxy resins, polyamide resins, polyimide resins, polyurethane resins, polyester resins, polyether resins, polyolefin resins, polycarbonate resins, polystyrene resins, norbornene resins, phenol novolak resins, and other photosensitive resins. These resins may be used singly, or may be used by mixing or copolymerizing a plurality of them.

In the present embodiment, the resin material used for the partition layer 1011 is not limited to photosensitive resins, but may be, for example, silicone rubber (PDMS: polydimethylsiloxane) or synthetic resins. Examples of the synthetic resins include polymethyl methacrylate resins (PMMA), polycarbonates (PC), polystyrene resins (PS), polypropylenes (PP), cycloolefin polymers (COP), and cycloolefin copolymers (COC). The resin material for the partition layer 1011 is preferred to be appropriately selected according to usage.

The thickness of the partition layer 1011, i.e., the height of the channel section 1003 on the substrate 1010, is not particularly limited, but is required to be larger than the substances (e.g., drugs, bacteria, cells, red blood cells, white blood cells, etc.) to be analyzed and tested which are contained in the fluid introduced into the channel section 1003. Therefore, the thickness of the partition layer 1011, i.e., the height (depth) of the channel section 1003, is preferred to be in the range of 1 µm or more and 500 µm or less, more preferred to be in the range of 10 µm or more and 100 µm, and even more preferred to be in the range of 40 µm or more and 60 µm or less.

Similarly, since the width of the channel section 1003 is required to be larger than the substances to be analyzed and tested, the width of the channel section 1003 defined within the partition layer 1011 is preferred to be in the range of 1 µm or more and 500 µm or less, more preferred to be in the range of 10 µm or more and 100 µm, and even more preferred to be in the range of 10 µm or more and 30 µm or less.

The channel length determined by the partition layer 1011 is required to ensure sufficient reaction time for the reaction solution, and therefore, is preferred to be in the range of 10 mm or more and 100 mm or less, more preferred to be in the range of 30 mm or more and 70 mm or less, and even more preferred to be in the range of 40 mm or more and 60 mm or less.

### (2.1.1.3) Upper lid layer

As shown in Figs. 5(a) and 5b), the upper lid layer 1012 of the microfluidic chip 1001 of the present embodiment is a lid component covering the channel section 1003. As mentioned above, the upper lid layer 1012 is provided on the surface of the partition layer 1011 facing away from the substrate 1010, and faces the substrate 1010 via the partition layer 1011. More specifically, as shown in Fig. 5(b), the side end portions of the upper lid layer 1012, as viewed in cross section, are supported by the partition layer 1011, the center region thereof faces the substrate 1010, and the center region defines the upper part of the channel section 1003.

The upper lid layer 1012 can be formed using either a translucent material or a non-translucent material. For example, if the state inside the channel is detected and observed using light, a material having good transparency to light can be used. A resin can be used as the translucent material. The resin used for forming the upper lid layer 1012 may be a photosensitive resin similarly to the partition layer 1011.

The photosensitive resin used for the upper lid layer 1012 is a resin having thermal fluidity. If a photosensitive resin having thermal fluidity is used as the material of the upper lid layer 1012, the upper lid layer 1012 can be formed in the microfluidic chip 1001 without using an adhesive. Thus, adhesive components are prevented from eluting into the channel and inhibiting reactions of the solution in the channel.

Conventionally, bonding of a partition layer to a lid component using an adhesive has required advanced equipment and technique, and this made the production method complicated. In the microfluidic chip 1001 according to the present embodiment, the upper lid layer 1012 can be formed on the partition layer 1011 more easily than in the conventional art, using a resin (photosensitive resin in the present example) having thermal fluidity as a material for the upper lid layer 1012. This can eliminate complexity of the production method.

The photosensitive resin having thermal fluidity is preferred to have a melt flow rate (MFR) in the range of 1 g/10 min or more and 100 g/10 min (230°C) or less. This can facilitate formation of the upper lid layer 1012 in the production process described later.

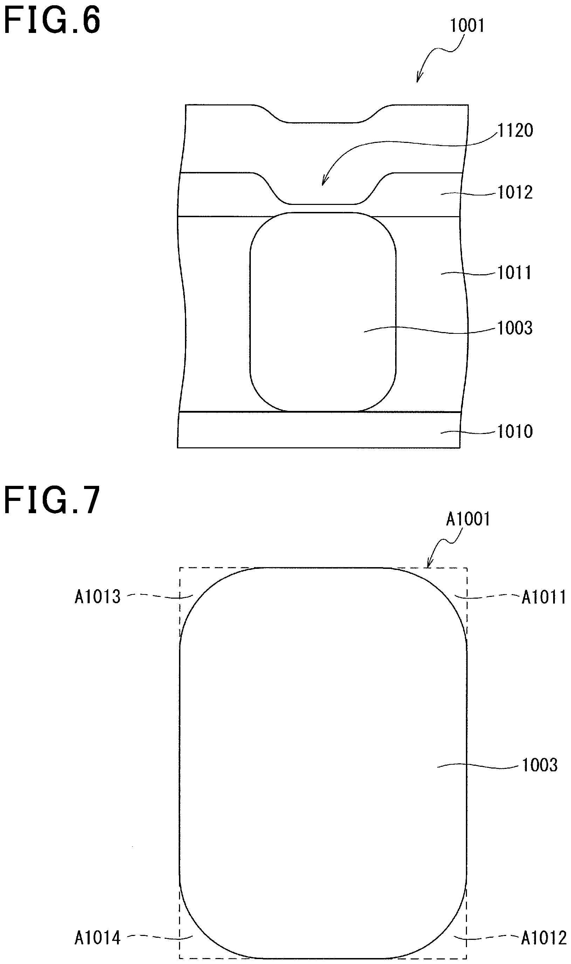

As will be described in detail later, the upper lid layer 1012 is formed by heat-treating and fluidizing (reflowing) a photosensitive resin having thermal fluidity formed on the partition layer 1011, and allowing it to flow onto the channel section 1003. Therefore, as shown in Fig. 6, the upper lid layer 1012 has a recess 1120. In other words, the upper lid layer 1012 has a recessed region. Specifically, the upper lid layer 1012 has a thickness which is reduced from the partition layer 1011 side toward the center of the channel section 1003, forming the recess 1120 as viewed in cross section.

The upper lid layer 1012 may be formed using a resin material with a glass transition temperature (Tg) lower than that of the partition layer 1011. For example, the glass transition temperature of the upper lid layer 1012 may be lower than that of the partition layer 1011 by 30°C to 50°C. In this case, the photosensitive resin forming the partition layer 1011 is hardly fluidized because the glass transition temperature (Tg) thereof is higher than that of the photosensitive resin forming the upper lid layer 1012. Accordingly, when forming the upper lid layer 1012, the partition layer 1011 can be suppressed from being fluidized and changing the channel pattern.

The glass transition temperature (Tg) of the upper lid layer 1012 is preferred to be in the range of 100°C or more and 300°C or less.

The upper lid layer 1012 is preferred to have an exposure sensitivity in the range of 5 µC/cm² or more and 50 µC/cm² or less.

The upper lid layer 1012 may have an exposure sensitivity different from that of the partition layer 1011. For example, the exposure sensitivity of the upper lid layer 1012 may be higher than that of the partition layer 1011 by the range of 5 µC/cm² or more and 20 µC/cm² or less.

For example, the exposure sensitivity of the upper lid layer 1012 may be lower than that of the partition layer 1011 by the range of 5 µC/cm² or more and 20 µC/cm² or less.

Thus, the upper lid layer 1012 may be configured to have an exposure sensitivity higher or lower than that of the partition layer 1011.

If the exposure sensitivity is different between the upper lid layer 1012 and the partition layer 1011, the exposure dose may be set to a value matching the photosensitive resin forming the partition layer 1011, so that the opening width of the photosensitive resin forming the partition layer 1011, i.e., the width of the channel pattern, can be made sufficiently large. Thus, when fluidizing the resin material of the upper lid layer during formation of the upper lid layer 1012, if fluidization has also occurred in the partition layer resin, the opening width of the photosensitive resin forming the partition layer 1011 can be made sufficiently large and a sufficient space can be retained as the channel section 1003.

### (2.1.1.4) Channel configuration

Fig. 7 is a diagram illustrating an example of a cross section of the channel section 1003 of the microfluidic chip 1001 according to the present embodiment. In the present embodiment, the cross section of the channel section 1003 is preferred to have rounded corners (e.g., the sides of the channel section 1003 in a cross section are connected by arcs). Thus, the transfer speed or flow rate of the fluid (e.g., reaction solution) in the channel section 1003 can be stabilized, and the substances to be tested can be suppressed from being retained at the corners.

Referring to Fig. 7, a cross section of the channel section 1003 in the present embodiment will be described. Fig. 7 is a schematic cross-sectional view illustrating an enlarged cross section of the channel section 1003 having rounded corners. As shown in Fig. 7, there is a difference in area between a virtual cross section A1001 when the cross-sectional shape of the channel section 1003 is rectangular and the cross section of the channel section 1003 having rounded corners. Specifically, as shown in Fig. 7, the four corners of the cross section of the channel section 1003 each have an arc shape. Therefore, the area of the cross section of the channel section 1003 is smaller than that of the virtual cross section A1001 by the sum of the areas of the virtual corner portions A1011, A1012, A1013 and A1014.

In the present embodiment, the area of the cross section of the channel section 1003 having rounded corners may be in the range of 95% or more and 98% or less of the surface area of the virtual cross section A1001. Fig. 7 shows the channel section 1003 having a rectangular cross section with rounded corners; however, without being limited to this, the cross section may have other shapes (polygonal shape with rounded corners) than a rectangular shape. In this case also, the area of the cross section of the channel section 1003 may be in the range of 95% or more and 98% or less of the area of the virtual cross section not having rounded corners.

The channel section 1003 is preferred to have a 10-point surface roughness Rz in the range of 0.001 µm or more and 0.03 µm or less. The 10-point surface roughness Rz of the channel section 1003 refers to the roughness of the side surfaces of parts of the partition layer 1011 facing the channel section 1003, and the surface of the substrate 1010 facing the channel section 1003.

If the 10-point surface roughness Rz is adequately designed within the above range, testing can be suitably performed according to the reaction solutions or the substances to be tested, and according to the desired testing conditions. For example, the surface roughness Rz of the channel section 1003 being in the range of 0.001 µm or more and 0.01 µm or less can reduce contact of the fluid (e.g., reaction solution) or substance to be tested introduced into the channel section 1003, with the inner surfaces of the channel section 1003 and can improve liquid transferability (e.g., liquid transfer speed or flow rate).

The surface roughness Rz of the channel section 1003 being greater than 0.02 µm and less than or equal to 0.03 µm can reduce the liquid transfer speed and can ensure time of stay in the channel section 1003, i.e., reaction time, of the fluids or substances to be tested introduced into the channel section 1003.

The surface roughness Rz of the channel section 1003 being greater than 0.01 µm and less than or equal to 0.02 µm can ensure both of an appropriate liquid transfer speed and reaction time of the fluids or substances to be tested in the channel section 1003.

The surface roughness of the channel section 1003 can be adequately controlled using a known etching method or other methods during production of the microfluidic chip 1001.

### (2.1.2) Method of producing microfluidic chip

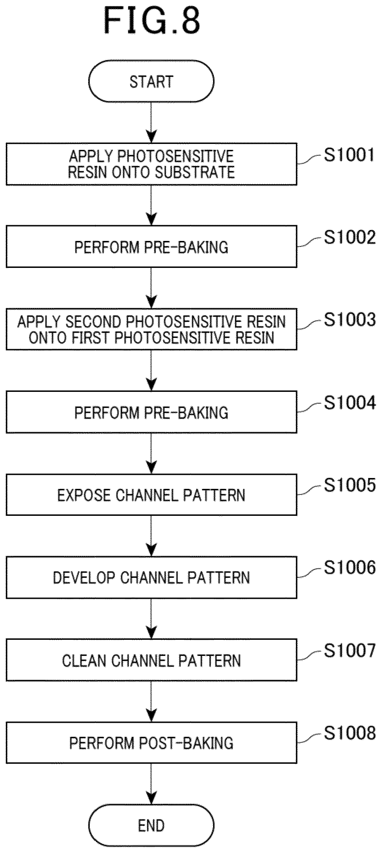

Next, a method of producing the microfluidic chip 1001 of the present embodiment will be described. Fig. 8 is a flowchart illustrating an example of a method of producing the microfluidic chip 1001 according to the present embodiment.

Description herein will be provided taking an example in which the partition layer 1011 is formed of a photosensitive resin.

### (Step S1001)

In the method of producing the microfluidic chip 1001 of the present embodiment, a resin for forming a partition layer 1011 (one example of the first photosensitive resin) is applied first onto a substrate 1010. Thus, a resin layer for forming a partition layer 1011 is provided on the substrate 1010. In the method of producing the microfluidic chip 1001 of the present embodiment, for example, a resin layer (first photosensitive resin layer) made of a photosensitive resin is formed on the sub strate 1010.

The photosensitive resin layer is formed on the substrate 1010, for example, by applying a photosensitive resin onto the substrate 1010. Examples of the coating method include spin coating, spray coating, and bar coating, among which, spin coating is preferred from the perspective of film thickness controllability. The photosensitive resin that can be applied to the substrate 1010 can be in various forms such as liquid, solid, gel-like, and film-like forms. Of these forms, liquid resists are preferred to be used for forming the photosensitive resin layer. Such a liquid resist can be positive or negative type, which can be adequately determined according to the characteristics of the channel pattern.

The resin (e.g., photosensitive resin) may be applied to the substrate 1010 such that the thickness of the resin layer (e.g., photosensitive resin layer), i.e., the thickness of the partition layer 1011, will be in the range of 1 µm or more and 500 µm or less.

### (Step S1002)

Following formation of the first photosensitive resin layer of the partition resin on the substrate 1010, heat treatment (pre-baking treatment) is performed for the purpose of removing solvent contained in the resin (e.g., photosensitive resin) applied to the substrate 1010. In the method of producing the microfluidic chip 1001 of the present embodiment, pre-baking treatment is not essential but may be adequately performed under optimal conditions (temperature and duration) according to the characteristics of the resin used. For example, if the resin layer on the substrate 1010 is formed of a photosensitive resin, pre-baking may be performed under optimal temperature and duration adequately determined according the characteristics of the photosensitive resin. For the purpose of improving adhesion between the substrate and the photosensitive resin, the substrate may undergo an HMDS treatment or may be provided with a thin film of a resin coating as necessary.

### (Step S1003)

Next, a resin for forming an upper lid layer 1012 (one example of the second photosensitive resin) is applied onto the first photosensitive resin layer. Thus, a resin layer for forming an upper lid layer 1012 is provided on the pre-baked partition resin. In the method of producing the microfluidic chip 1001 of the present embodiment, for example, a resin layer (second photosensitive resin layer) made of a photosensitive resin having thermal fluidity is formed on the substrate 1010. The second photosensitive resin layer can be formed on the substrate 1010 similarly to the first photosensitive resin layer formed at step S1001.

### (Step S1004)

Following formation of the second photosensitive resin layer of the upper lid resin on the first photosensitive resin layer of the partition resin, heat treatment (pre-baking treatment) is performed for the purpose of removing solvent contained in the upper lid resin applied to the first photosensitive resin layer. The pre-baking treatment of the present step is not essential but, similarly to the pre-baking treatment at step S1002, may be adequately performed under optimal conditions (temperature and duration) according to the characteristics of the resin used. For the purpose of improving adhesion between the first photosensitive resin layer and the second photosensitive resin, the substrate may undergo an HMDS treatment or may be provided with a thin resin coating as necessary. Thus, two photosensitive resin layers are formed on the substrate 1010.

As shown in steps S1001 to S1004, the two photosensitive resin layers (first and second photosensitive resin layers) for forming a partition layer 1011 and an upper lid layer 1012 are joined together by welding without using an adhesive. In other words, in the microfluidic chip 1001 of the present embodiment, the partition layer 1011 and the upper lid layer 1012 are joined together by welding. More specifically, in the microfluidic chip 1001 of the present embodiment, the partition layer 1011 and the upper lid layer 1012 can be joined together without using an intermediate component (e.g., adhesive). Thus, adhesive components can be prevented from eluting into the channel section 1003, and poor bonding that would have been induced by uneven thickness of the adhesive can be avoided.

The number of the photosensitive resin layers is not limited to two as in the present embodiment, but may be three or more.

### (Step S1005)

Next, the resins (e.g., first and second photosensitive resins) applied to the substrate 1010 are exposed. Specifically, a channel pattern is drawn by exposure on the photosensitive resins applied to the substrate 1010. Exposure may be performed, for example, using an exposure device that uses UV light as a light source, or using a laser drawing device. Among them, proximity exposure using UV light as a light source or exposure using a contact exposure device is preferred. If a proximity exposure device is used, exposure is performed via a photomask with a channel pattern array for the microfluidic chip 1001. The photomask may be a photomask with a light-shielding film that has a double-layer structure of chromium and chromium oxide.

If the photosensitive resins (first and second photosensitive resin layers) applied to the substrate 1010 are positive resists, the exposed regions are dissolved, forming the channel section 1003, and the photosensitive resin in the unexposed regions remain as parts of the partition layer 1011 and the upper lid layer 1012. If the photosensitive resins applied to the substrate 1010 are negative resists, the photosensitive resins in the exposed regions remain as parts of the partition layer 1011 and the upper lid layer 1012, and the unexposed regions are dissolved, forming the channel section 1003. In this way, in the method of producing the microfluidic chip 1001 of the present embodiment, the partition layer 1011 configuring the channel section 1003 can be formed on the substrate 1010 using photolithography.

If a chemically amplified resist or the like is used for forming a resin layer on the substrate 1010, heat treatment (post-exposure baking: PEB) may be further performed after exposure to promote catalytic reaction of the acid generated due to exposure.

### (Step S1006)

Next, the exposed photosensitive resins are developed to form a channel pattern.

The development is performed, for example, using a spray-, dip- or paddle-type or other types of developing device, by reaction of the photosensitive resin with a developer. Examples of the developer include sodium carbonate aqueous solution, tetramethylammonium hydroxide, potassium hydroxide, and organic solvents. The developer is not limited to these, but an optimal developer may be adequately used according to the characteristics of the photosensitive resins. The concentration or the time of development can be adequately adjusted to optimal conditions according to the characteristics of the photosensitive resins.

### (Step S1007)

Next, the resin layers (photosensitive resin layers) on the substrate 1010 are cleaned to completely remove the developer used for development. Cleaning may be performed, for example, using a spray-, shower-, immersion-type or other types of cleaning device. An optimal cleaning solution may be adequately used from among, for example, pure water, isopropyl alcohol, and the like to remove the developer used for development. Cleaning is followed by drying using a spin dryer, IPA vapor dryer, natural drying, etc.

At this stage also, the second photosensitive resin layer of the upper lid resin remains on the partition layer 1011.

### (Step S1008)

Next, the channel pattern, i.e., the partition layer 1011 forming the channel section 1003 and the second photosensitive resin layer, undergoes heat treatment (post-baking). With this post-baking treatment, residual moisture generated during development or cleaning is removed. Also, with this post-baking treatment, an upper lid layer 1012 serving as a lid for the channel section 1003 and defining the upper part of the channel section 1003 is formed. Specifically, this post-baking treatment can promote fluidization (reflow) of the upper lid resin, i.e., the photosensitive resin having fluidity, and form an upper lid layer 1012 for the channel section 1003. The post-baking treatment is performed with optimal temperature and duration determined according the characteristics of the upper lid resin. As shown in Fig. 5, the upper lid layer 1012 is a lid component covering the channel 1003 and thus is formed over the channel section 1003. The inlet 1002 and the outlet 1004 are open without being covered with the upper lid layer 1012.

For example, the post-baking treatment is performed using a hot plate, oven, etc. If the drying performed at the cleaning step S1007 is insufficient, the developer or moisture generated during cleaning may remain in the partition layer 1011. Solvent that has not been removed in the post-baking treatment may also remain in the partition layer 1011. These materials can be removed by performing post-baking treatment.

As described above, the method of producing the microfluidic chip 1001 according to the present embodiment includes steps of applying a partition resin onto a substrate 1010 (step S1001), applying an upper lid resin onto the applied partition resin (step S1003), exposing the applied partition resin and the applied upper lid resin (step S1005), developing and cleaning the exposed partition resin and upper lid resin to form a partition layer 1011 defining a channel section 1003 on the substrate 1010 (steps S1006 and S1007), and heat-treating and fluidizing the upper lid resin on the partition layer 1011 to form an upper lid layer 1012 for the channel section 1003 (step S1008).

Thus, the partition layer 1011 and the upper lid layer 1012 can be joined together by welding without using an adhesive layer, so that adhesive components are prevented from eluting into the channel section 1003 and inhibiting reactions of the solution in the channel.

### (2.1.3) Details of steps of producing partition layer and upper lid layer

In the method of producing the microfluidic chip 1001 of the present embodiment, production steps are adjusted according to the physical properties of the partition resin for forming the partition layer 1011 and the upper lid resin for forming the upper lid layer 1012. A specific example will be described below.

### (2.1.3.1) Formation of partition layer and upper lid layer using resins having different glass transition temperatures

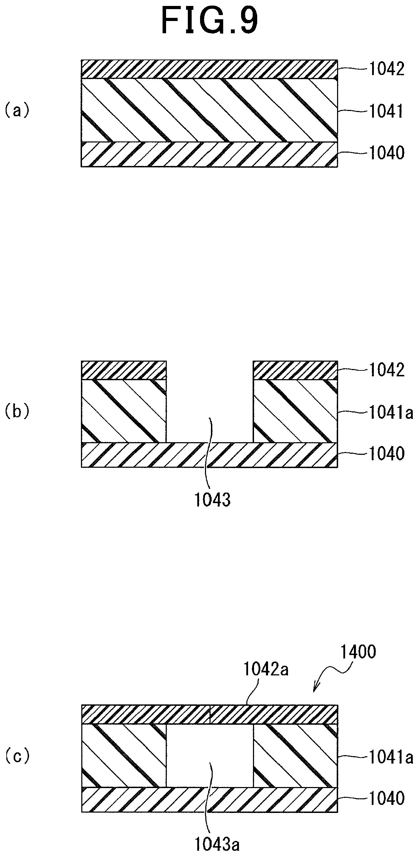

Referring to Fig. 9, a description will be given of forming the partition layer 1011 and the upper lid layer 1012 in the case where the partition resin and the upper lid resin have different glass transition temperatures. Fig. 9(a) is a schematic cross-sectional view illustrating a first photosensitive resin layer 1041 and a second photosensitive resin layer 1042 formed on a substrate 1040, Fig. 9(b) is a schematic cross-sectional view illustrating a channel pattern on the substrate 1040, and Fig. 9(c) is a schematic cross-sectional view illustrating a configuration of a microfluidic chip 1400 according to the present example.

In the present example, a description will be given of a method of producing a microfluidic chip under conditions in which the glass transition temperature (Tg) of the upper lid resin is lower than that of the partition resin [Glass transition temperature (Tg) of upper lid resin < Glass transition temperature (Tg) of partition resin].

As shown in Fig. 9(a), in the present example, a first photosensitive resin layer 1041 is formed by applying a partition photosensitive resin onto the substrate 1040 at the coating step S1001. The partition photosensitive resin is applied with a desired thickness onto the substrate 1040 using spin coating, for example. In the present example, a second photosensitive resin layer 1042 is formed by applying an upper lid photosensitive resin onto the first photosensitive resin layer 1041 at step S1003. In the present example, the upper lid photosensitive resin forming the second photosensitive resin layer 1042 has a glass transition temperature (Tg) lower than that of the partition photosensitive resin forming the first photosensitive resin layer 1041. The second photosensitive resin layer 1042 is applied with a desired thickness onto the first photosensitive resin layer 1041 using spin coating, similarly to the first photosensitive resin layer 1041.

In the present example, at the exposure step S1005, a channel pattern is drawn, via a photomask, on the first and second photosensitive resin layers 1041 and 1042 applied to the substrate 1040. For example, the present example uses a proximity exposure device using light with a wavelength of 350 nm or more and 400 nm or less in the UV light region as a light source. Next, at the development step S1006, a channel pattern 1043 is formed by developing the exposed first and second photosensitive resin layers 1041 and 1042. For example, a sodium carbonate solution may be sprayed as a developer. Next, at step S1007, the developed first and second photosensitive resin layers 1041 and 1042 are cleaned to completely remove the developer. For example, ultra-pure water may be sprayed for cleaning. Thus, as shown in Fig. 9(b), a partition layer 1041a is formed defining the channel pattern 1043.

Next, the microfluidic chip, in which the channel pattern 1043 has been formed, undergoes heat treatment (post-baking) at step S1008. In the present example, heat treatment is performed using a hot plate at a temperature near the glass transition temperature (Tg) of the second photosensitive resin layer 1042. In the present example, heat treatment promotes fluidization (reflow) of the second photosensitive resin layer 1042, i.e., the upper lid resin, and portions of the upper lid resin on opposing right and left parts of the partition layer 1011 flow toward the center of the channel pattern 1043. The portions of the upper lid resin that have flowed from parts of the partition layer 1041a are joined on the side opposite from the substrate 1040, i.e., on the upper side of the channel pattern 1043, to form an upper lid layer 1042a. Thus, as shown in Fig. 9(c), the upper part of the channel pattern 1043 is covered with the upper lid layer 1042a to form a channel section 1043a, thereby producing the microfluidic chip 1400.

As in the present example, if the glass transition temperature (Tg) of the first photosensitive layer 1041, i.e., the partition resin, forming the partition layer 1041a is higher than that of the second photosensitive resin layer 1042, i.e., the upper lid resin, the partition resin is not fluidized substantially even when the upper lid resin is fluidized (reflows) due to heat treatment. Accordingly, by satisfying the condition of [Glass transition temperature (Tg) of upper lid resin < Glass transition temperature (Tg) of partition resin], the upper lid layer 1042a can be easily formed by causing the upper lid resin to fluidize, without deforming the channel pattern due to the fluidization of the upper lid resin.

### (2.1.3.2) Formation of partition layer and upper lid layer using resins with different exposure sensitivities (1)

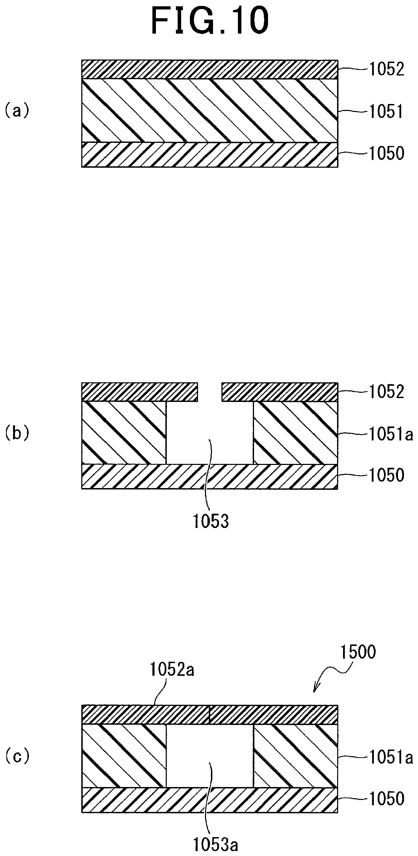

Referring to Fig. 10, a description will be given of forming the partition layer 1011 and the upper lid layer 1012 in the case where the partition resin and the upper lid resin have different exposure sensitivities. Fig. 10(a) is a schematic cross-sectional view illustrating a first photosensitive resin layer 1051 and a second photosensitive resin layer 1052 formed on a substrate 1050, Fig. 10(b) is a schematic cross-sectional view illustrating a channel pattern on the substrate 1050, and Fig. 10(c) is a schematic cross-sectional view illustrating a configuration of a microfluidic chip 1500 according to the present example.

In the present example, a description will be given of a method of producing a microfluidic chip under conditions in which the exposure sensitivity (C/cm²) of the upper lid resin is lower than that of the partition resin [Exposure sensitivity (C/cm²) of upper lid resin < Exposure sensitivity (C/cm²) of partition resin].

As shown in Fig. 10(a), in the present example, a first photosensitive resin layer 1051 is formed by applying a partition photosensitive resin onto the substrate 1050 at the coating step S1001. The partition photosensitive resin is applied with a desired thickness onto the substrate 1050 using spin coating, for example. The first photosensitive resin layer 1051, i.e., the partition resin, is a positive resist. In the present example, a second photosensitive resin layer 1052 is formed by applying an upper lid photosensitive resin onto the first photosensitive resin layer 1051 at step S1003. The second photosensitive resin layer 1052, i.e., the upper lid resin, is a positive resist. In other words, in the present example, positive resists are used as the partition photosensitive resin and the upper lid photosensitive resin.

In the present example, the upper lid photosensitive resin forming the second photosensitive resin layer 1052 has an exposure sensitivity (C/cm²) lower than that of the partition photosensitive resin forming the first photosensitive resin layer 1051. The second photosensitive resin layer 1052 is applied with a desired thickness onto the first photosensitive resin layer 1051 using spin coating, similarly to the first photosensitive resin layer 1051. For example, a chemically amplified resist may be used as the partition resin for forming the first photosensitive resin layer 1051, and a non-chemically amplified resist may be used as the upper lid resin for forming the second photosensitive resin layer 1052.

In the present example, at the exposure step S1005, a channel pattern is drawn, via a photomask, on the first and second photosensitive resin layers 1051 and 1052 applied to the substrate 1050. For example, the present example uses a proximity exposure device using light with a wavelength of 350 nm or more and 400 nm or less in the UV light region as a light source. An optimal exposure dose is determined herein for the first photosensitive resin layer 1051. As mentioned above, in the present example, the exposure sensitivity of the upper lid resin for forming the second photosensitive resin layer 1052 is lower than that of the partition resin for forming the first photosensitive resin layer 1051. Therefore, although reaction is insufficient in the second photosensitive resin layer 1052 with the exposure dose matching the first photosensitive resin layer 1051, there is no problem in pattern resolution.

Next, at the development step S1006, a channel pattern 1053 is formed by developing the exposed first and second photosensitive resin layers 1051 and 1052. For example, a sodium carbonate solution may be sprayed as a developer. Next, at step S1007, the developed first and second photosensitive resin layers 1051 and 1052 are cleaned to completely remove the developer. For example, ultra-pure water may be sprayed for cleaning. Thus, as shown in Fig. 10(b), a partition layer 1051a is formed defining the channel pattern 1053. In the present example, as mentioned above, the second photosensitive resin layer 1052 is made of a resin having an exposure sensitivity lower than that of the first photosensitive resin layer 1051. Therefore, as shown in Fig. 10, the opening width in the exposed region in the second photosensitive resin layer 1052 is smaller than the opening width of the partition layer 1051a formed of the first photosensitive resin layer 1051.

Next, the microfluidic chip, in which the channel pattern 1053 has been formed, undergoes heat treatment (post-baking) at step S1008. In the present example, heat treatment is performed using a hot plate at a temperature near the glass transition temperatures (Tg) of the first and second photosensitive resin layers 1051 and 1052. In the present example, heat treatment promotes fluidization (reflow) of the second photosensitive resin layer 1052, i.e., the upper lid resin, and portions of the upper lid resin on right and left parts of the partition layer 1051a flow toward the center of the channel pattern 1053. The portions of the upper lid resin that have flowed from parts of the partition layer 1051a are joined on the side opposite from the substrate 1050, i.e., on the upper side of the channel pattern 1053, to form an upper lid layer 1052a. Thus, as shown in Fig. 10(c), the upper part of the channel pattern 1053 is covered with the upper lid layer 1052a to form a channel section 1053a, thereby producing the microfluidic chip 1500.

In the present example, the glass transition temperature (Tg) of the first photosensitive resin layer 1051, i.e., the partition resin, forming the partition layer 1051a is equal to that of the second photosensitive resin layer 1052, i.e., upper lid resin, forming the upper lid layer 1052a. Therefore, when the upper lid resin is fluidized (reflows), the partition resin is also fluidized. However, in the present example, the first and second photosensitive resin layers 1051 and 1052 are each formed of a positive resist, and the exposure sensitivity of the second photosensitive resin layer 1052 is lower than that of the first photosensitive resin layer 1051. Therefore, as shown in Fig. 10(b), the opening width of the second photosensitive resin layer 1052 is formed to be smaller than the opening width of the partition layer 1051a formed of the first photosensitive resin layer 1051, and the opening width of the partition layer 1051a formed of the first photosensitive resin layer 1051 is formed to be sufficiently larger than the opening width of the second photosensitive resin layer 1052.

Accordingly, the width of the channel section 1053a is reduced by more than the opening width of the channel pattern 1053 is, due to occurrence of fluidization in the first photosensitive resin layer 1051 with a degree that is approximately the same as in the second photosensitive resin layer 1052; however, as shown in Fig. 10(c), the width of the channel section 1053a is sufficiently ensured.

Accordingly, if the first and second photosensitive resin layers 1051 and 1052 are each formed of a positive resist and if the condition of [Exposure sensitivity of upper lid resin < Exposure sensitivity of partition resin] is satisfied, the upper lid resin and the partition resin can be fluidized to easily form the upper lid layer 1052a, while sufficiently retaining the width of the channel section.

### (2.1.3.3) Formation of partition layer and upper lid layer using resins with different exposure sensitivities (2)

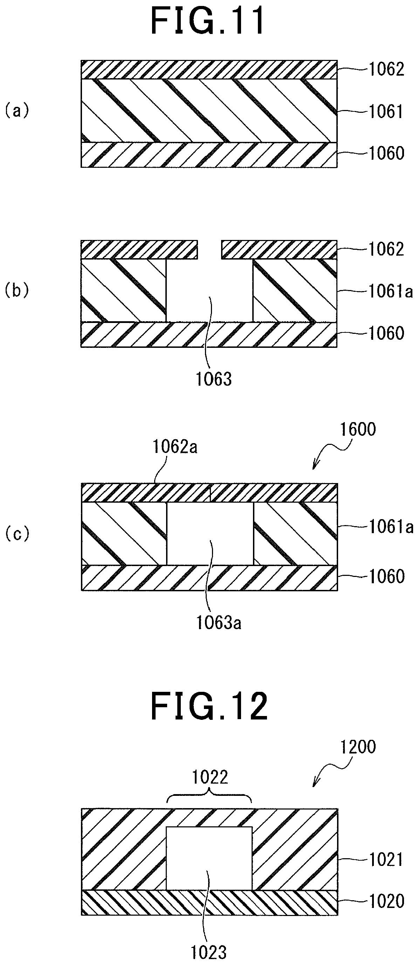

Referring to Fig. 11, a description will be given of forming the partition layer 1011 and the upper lid layer 1012 in the case where the partition resin and the upper lid resin have different exposure sensitivities. Fig. 11(a) is a schematic cross-sectional view illustrating a first photosensitive resin layer 1061 and a second photosensitive resin layer 1062 formed on a substrate 1060, Fig. 11(b) is a schematic cross-sectional view illustrating a channel pattern on the substrate 1060, and Fig. 11(c) is a schematic cross-sectional view illustrating a configuration of a microfluidic chip 1600 according to the present example.

In the present example, a description will be given of a method of producing a microfluidic chip under conditions in which the exposure sensitivity (C/cm²) of the upper lid resin is higher than that of the partition resin [Exposure sensitivity (C/cm²) of upper lid resin > Exposure sensitivity (C/cm²) of partition resin].

As shown in Fig. 11(a), in the present example, a first photosensitive resin layer 1061 is formed by applying a partition photosensitive resin onto the substrate 1060 at the coating step S1001. The partition photosensitive resin is applied with a desired thickness onto the substrate 1060 using spin coating, for example. The first photosensitive resin layer 1061, i.e., the partition resin, is a negative resist. In the present example, a second photosensitive resin layer 1062 is formed by applying an upper lid photosensitive resin onto the first photosensitive resin layer 1061 at step S1003. The second photosensitive resin layer 1062, i.e., the upper lid resin, is a negative resist. In other words, in the present example, negative resists are used as the partition photosensitive resin and the upper lid photosensitive resin.

In the present example, the upper lid photosensitive resin forming the second photosensitive resin layer 1062 has an exposure sensitivity (C/cm²) lower than that of the partition photosensitive resin forming the first photosensitive resin layer 1061. The second photosensitive resin layer 1062 is applied with a desired thickness onto the first photosensitive resin layer 1061 using spin coating, similarly to the first photosensitive resin layer 1061. For example, a non-chemically amplified resist may be used as the partition resin for forming the first photosensitive resin layer 1061, and a chemically amplified resist may be used as the upper lid resin for forming the second photosensitive resin layer 1062.

In the present example, at the exposure step S1005, a channel pattern is drawn, via a photomask, on the first and second photosensitive resin layers 1061 and 1062 applied to the substrate 1060. For example, the present example uses a proximity exposure device using light with a wavelength of 350 nm or more and 400 nm or less in the UV light region as a light source. An optimal exposure dose is determined herein for the first photosensitive resin layer 1061. As mentioned above, in the present example, the exposure sensitivity of the upper lid resin forming the second photosensitive resin layer 1062 is higher than that of the partition resin forming the first photosensitive resin layer 1061. Therefore, in the second photosensitive resin layer 1062, the exposure dose matching the first photosensitive resin layer 1061 is necessary and sufficient.

Next, at the development step S1006, a channel pattern 1063 is formed by developing the exposed first and second photosensitive resin layers 1061 and 1062. For example, a sodium carbonate solution may be sprayed as a developer. Next, at step S1007, the developed first and second photosensitive resin layers 1061 and 1062 are cleaned to completely remove the developer. For example, ultra-pure water may be sprayed for cleaning. Thus, as shown in Fig. 1 1(b), a partition layer 1061a is formed defining the channel pattern 1063. In the present example, the exposure sensitivity of the second photosensitive resin layer 1062 is higher than that of the first photosensitive resin layer 1061. Therefore, as shown in Fig. 11(b), after exposure, the region in the second photosensitive resin layer 1062 remaining as a pattern in the exposed portion becomes larger than the first photosensitive resin layer 1061. Consequently, as shown in Fig. 10, the opening width in the exposed region in the second photosensitive resin layer 1062 becomes smaller than the opening width of the partition layer 1061a formed of the first photosensitive resin layer 1061.

Next, the microfluidic chip, in which the channel pattern 1063 has been formed, undergoes heat treatment (post-baking) at step S1008. In the present example, heat treatment is performed using a hot plate at a temperature near the glass transition temperatures (Tg) of the first and second photosensitive resin layers 1061 and 1062. In the present example, heat treatment promotes fluidization (reflow) of the second photosensitive resin layer 1062, i.e., the upper lid resin, and portions of the upper lid resin on right and left parts of the partition layer 1061a flow toward the center of the channel pattern 1063. The portions of the upper lid resin that have flowed from parts of the partition layer 1061a are joined together on the side opposite from the substrate 1060, i.e., on the upper side of the channel pattern 1063, to form an upper lid layer 1062a. Thus, as shown in Fig. 11(c), the upper part of the channel pattern 1063 is covered with the upper lid layer 1062a to form a channel section 1063a, thereby producing the microfluidic chip 1600.

Similarly to the microfluidic chip 1500, in the microfluidic chip 1600 of the present example, the glass transition temperature (Tg) of the first photosensitive resin layer 1061, i.e., the partition resin, forming the partition layer 1061a is equal to that of the second photosensitive resin layer 1062, i.e., the upper lid resin, forming the upper lid layer 1062a. Therefore, when the upper lid resin is fluidized (reflows), the partition resin is also fluidized. In the present example, the first and second photosensitive resin layers 1061 and 1062 are each formed of a negative resist, and the exposure sensitivity of the second photosensitive resin layer 1062 is higher than that of the first photosensitive resin layer 1061. Therefore, as shown in Fig. 11(b), the opening width of the second photosensitive resin layer 1062 is formed to be smaller than the opening width of the partition layer 1061a formed of the first photosensitive resin layer 1061, and the opening width of the partition layer 1061a formed of the first photosensitive resin layer 1061 is formed to be sufficiently larger than the opening width of the second photosensitive resin layer 1062.

Accordingly, the width of the channel section 1063a is reduced by more than the opening width of the channel pattern 1063 is, due to occurrence of fluidization in the first photosensitive resin layer 1061 with a degree that is approximately the same as in the second photosensitive resin layer 1062; however, as shown in Fig. 11(c), the width of the channel section 1063a is sufficiently ensured.

Accordingly, if the first and second photosensitive resin layers 1061 and 1062 are each formed of a negative resist and if the condition of [Exposure sensitivity of upper lid resin > Exposure sensitivity of partition resin] is satisfied, the upper lid resin and the partition resin can be fluidized to easily form the upper lid layer 1062a, while sufficiently retaining the width of the channel section.

The description so far has been given of an example in which the partition layer and the upper lid layer are formed as separate bodies (multilayer configuration) in the microfluidic chip of the present embodiment. As described above, according to the production method of the present embodiment, the lid component (upper lid layer) covering the channel section of the microfluidic chip can be formed using existing photolithography techniques without using an intermediate layer such as an adhesive. Thus, adhesive components are prevented from eluting into the channel and inhibiting reactions of the solution in the channel.

Also, omission of an adhesive can avoid quality deterioration due to poor bonding which would have been caused by uneven film thickness of the adhesive. Furthermore, the production method can be simplified compared to the case where a partition layer and an upper lid layer are bonded together using an intermediate component such as an adhesive.

The present disclosure is not limited to this, but the partition layer and the upper lid layer may be configured using three or more layers. In this case also, the second photosensitive resin layer forming the upper lid layer may have a multilayer configuration. Photosensitive resins having thermal fluidity have characteristics such that
the higher the layer, the greater the fluidity of the resin upon heat treatment. Therefore, in the case of forming an upper lid layer using a second photosensitive layer with a multilayer configuration, parts of upper layers are joined together faster than the lower layers. Accordingly, in the upper lid layer formed of a second photosensitive resin layer with a multilayer configuration, the opening width becomes narrower the higher the layer, and parts of the uppermost layer or parts of the layers close to the uppermost layer are joined together.

### (2.1.4) Effects of Embodiment 2-1

The microfluidic chip 1001 described above has the following effects.
(1) The microfluidic chip 1001 according to the present embodiment includes a substrate 1010, a partition layer 1011 forming a channel section 1003 on the substrate 1010, and an upper lid layer 1012 formed on the surface of the partition layer 1011 facing away from the substrate 1010 to serve as a lid for the channel section 1003, wherein no adhesive layer is provided between the partition layer 1011 and the upper lid layer 1012.

Thus, in the microfluidic chip 1001, adhesive components are prevented from eluting into the channel and inhibiting reactions of the solution in the channel. Also, omission of an adhesive can avoid poor bonding which would have been caused by uneven film thickness of the adhesive.

(2) The microfluidic chip 1001 according to the present embodiment includes a substrate 1010, a partition layer 1011 forming a channel section 1003 on the substrate 1010, and an upper lid layer 1012 formed on the surface of the partition layer 1011 facing away from the substrate 1010 to serve as a lid for the channel section 1003, wherein the upper lid layer 1012 and the partition layer 1011 are joined together by welding.

Thus, in the microfluidic chip 1001, the upper lid layer 1012 and the partition layer 1011 are joined together without using an adhesive, so that adhesive components are prevented from eluting into the channel and inhibiting reactions of the solution in the channel. Also, omission of an adhesive can avoid poor bonding which would have been caused by uneven film thickness of the adhesive.

(3) The microfluidic chip 1001 according to the present embodiment includes a channel section 1003 and an upper lid layer 1012 serving as a lid for the channel section 1003, wherein the material of the upper lid layer 1012 is a resin having thermal fluidity, and the channel section 1003 has a cross section with rounded corners.

Thus, in the microfluidic chip 1001, the upper lid layer 1012 and the partition layer 1011 are joined together without using an adhesive, so that adhesive components are prevented from eluting into the channel and inhibiting reactions of the solution in the channel. Furthermore, the transfer speed or flow rate of the fluid (e.g., reaction solution) in the channel section 1003 can be stabilized, and the substances to be tested can be suppressed from being retained at the corners. Also, the upper lid layer 1012 and the partition layer 1011 can be joined together using existing photolithography techniques.

(4) In the microfluidic chip 1001 according to the present embodiment, the upper lid layer 1012 and the partition layer 1011 may be separate bodies.

Thus, a resin having favorable characteristics as an upper lid can be adequately selected.

(5) In the microfluidic chip 1001 according to the present embodiment, the partition layer 1011 and the upper lid layer 1012 may be formed of resin materials, and the resin materials may be photosensitive resins having photosensitivity to light with a wavelength of 190 nm or more and 400 nm or less in the UV light region.

Thus, the lid component (upper lid layer) of the microfluidic chip can be formed using reflow of the photosensitive resins without using an adhesive, so that adhesive components are prevented from eluting into the channel and inhibiting reactions of the solution in the channel.

(6) In the microfluidic chip 1001 according to the present embodiment, the upper lid layer 1012 may be a separate body from the partition layer 1011 and may have a glass transition temperature lower than that of the partition layer 1011.

Thus, when forming the upper lid layer 1012, the partition layer 1011 can be suppressed from being fluidized and changing the channel pattern.

(7) In the microfluidic chip 1001 according to the present embodiment, the upper lid layer 1012 may be a separate body from the partition layer 1011 and may be configured to have an exposure sensitivity higher or lower than that of the partition layer 1011.

Thus, when fluidizing the resin material of the upper lid layer during formation of the upper lid layer 1012, if fluidization has also occurred in the partition layer resin, the opening width of the photosensitive resin forming the partition layer 1011 can be made sufficiently large and a sufficient space can be retained as the channel section 1003.

(8) The method of producing the microfluidic chip 1001 according to the present embodiment includes steps of applying a photosensitive resin for a partition layer 1011 onto a substrate 1010, applying a photosensitive resin for an upper lid layer 1012 onto the applied photosensitive resin for a partition layer 1011, exposing the photosensitive resin for a partition layer 1011 and the photosensitive resin for an upper lid layer 1012, developing and cleaning the exposed photosensitive resin for a partition layer 1011 and the exposed photosensitive resin for an upper lid layer 1012 to form a partition layer 1011 defining a channel section 1003 on the substrate 1010, and heat-treating and fluidizing the photosensitive resin for an upper lid layer 1012 on the partition layer 1011 to form an upper lid layer 1012 for the channel section 1003.

Thus, there can be provided a microfluidic chip in which adhesive components are prevented from eluting into the channel and inhibiting reactions of the solution in the channel.

### (2.2) Embodiment 2-2

Referring to Fig. 12, a microfluidic chip according to Embodiment 2-2 of the present disclosure will be described. Fig. 12 is a cross-sectional view illustrating a configuration example of a microfluidic chip 1200 according to Embodiment 2-2 of the present disclosure.

The microfluidic chip 1200 includes a substrate 1020, a partition layer 1021 forming a channel section 1023 on the substrate 1020, and an upper lid layer 1022 formed of a part of the partition layer 1021. In other words, in the microfluidic chip 1200, the partition layer 1021 is formed integrally with the upper lid layer 1022. This is a point different from the microfluidic chips 1001, 1400, 1500 and 1600 of Embodiment 2-1.

### (2.2.1) Configuration of microfluidic chip 1200

The partition layer 1021 and the upper lid layer 1022 of the microfluidic chip 1200 will be described focusing on differences from the partition layer 1011 and the upper lid layer 1012 of Embodiment 2-1. Since the configurations of the components (substrate 1020 and channel section 1023) other than the upper lid layer 1022 are similar to those of the substrate 1010 and the channel section 1003 of the microfluidic chip 1001, explanation will be omitted.

The materials of the partition layer 1021 and the upper lid layer 1022 of the microfluidic chip 1200 may be similar to that of the partition layer 1011 of the microfluidic chip 1001.

Fig. 12 shows a configuration example of the microfluidic chip 1200 in which the partition layer 1021 is formed integrally with the upper lid layer 1022. In the microfluidic chip 1200, since the partition layer 1021 serves as the upper lid layer 1022, the production processes can be simplified.

### (2.2.2) Method of producing microfluidic chip 1200

An example of a method of producing the microfluidic chip 1200 will be described.

The method of producing the microfluidic chip 1200 can omit step S1003 (step of applying the second photosensitive resin) from the method of producing the microfluidic chip 1001 shown in Fig. 8. Thus, the production processes can be simplified.

Referring to Fig. 13, the method of producing the microfluidic chip 1200 will be described in more detail.

Fig. 13 is a set of diagrams illustrating processes in the method of producing the microfluidic chip 1200 according to the present embodiment. In the method of producing the microfluidic chip 1200, a channel pattern is formed as in the steps S1001, S1002 and S1004 to S1007 of the method of producing the microfluidic chip 1001 shown in Fig. 8, except that only one layer of a photosensitive resin is formed on the substrate 1020. Therefore, explanation for the steps S1001, S1002 and S1004 to S1007 will be omitted.

Fig. 13(a) is a schematic plan view illustrating a channel pattern 1220 when producing the microfluidic chip 1200 according to the present embodiment. The partition layer 1021 is provided with an inlet 1032 for introducing a liquid, a channel section 1023 through which the liquid flows, and an outlet 1034 for discharging the liquid. Explanation for the inlet 1032 and the outlet 1034 will be omitted because they have configurations similar to those of the inlet 1002 and the outlet 1004 of the microfluidic chip 1001 according to Embodiment 2-1 described above.

Fig. 13(b) is a cross-sectional view taken along the line B-B of Fig. 13(a). The partition layer 1021 is formed on the substrate 1020 as a base. The region surrounded by the substrate 1020 and the partition layer 1021 is a region 1032a in the channel pattern 1220 serving as the inlet 1032 for introducing a fluid (e.g., reaction solution). In order to introduce a fluid, no upper lid layer 1022 is formed on the upper part of the inlet 1032.

Fig. 13(c) is a cross-sectional view taken along the line C-C of Fig. 13(a). The partition layer 1021 is formed on the substrate 1020 as a base component, and the region surrounded by the substrate 1020 and the partition layer 1021 is a region 1023a in the channel pattern 1220 defining the channel section 1023 through which a fluid flows. The region 1023a has an opening width which is formed to be smaller than that of the region 1032a serving as the inlet 1032.

The channel pattern 1220 formed is subjected to heat treatment (post-baking). Heat treatment is performed, for example, using a hot plate, oven, etc. Post-baking is performed for the purpose of fluidizing (reflowing) the partition resin (partition layer 1021), by heating the resin up to the glass transition temperature (Tg) thereof. This is a point different from the heat treatment in the method of producing the microfluidic chip 1001 according to Embodiment 2-1. Fluidization (reflow) of a photosensitive resin is characterized by the tendency that the photosensitive resin is fluidized more on the side opposite from the substrate 1020, i.e., on the upper part of the channel pattern 1220, depending on the characteristics of the resin. Due to fluidization of the upper part of the channel pattern 1220, parts of the partition layer 1021 are joined together to function as an upper lid component of the channel pattern 1220, i.e., as the upper lid layer 1022 covering the region 1023a of the channel pattern 1220. Thus, an upper lid layer 1022 integrated with the partition layer 1021 is formed, producing a microfluidic chip 1200 of the present embodiment.

Fig. 13(d) is a schematic plan view illustrating the microfluidic chip 1200 after heat treatment (post-baking). Due to heat treatment, the partition layer 1021 is fluidized to form an upper lid layer 1022, as a result of which a channel section 1023 is defined as shown in Fig. 13(f) described later. Although not shown in Fig. 13(d), the channel section 1023 can be seen through a transparent upper lid layer 102. The inlet 1032 and the outlet 1034 are reduced in size due to fluidization of the partition layer 1021; however, since they are designed in advance to have a larger size to compensate for size reduction, they are not closed by the upper lid layer 1022 and remain as through-holes. This is similar to the production of the microfluidic chip 1001 according to Embodiment 2-1.

Fig. 13(e) is a schematic cross-sectional view illustrating the microfluidic chip 1200 taken along the line B-B of Fig. 13(d). Due to post-baking on a hot plate 1025, the resin on the upper part of the partition layer 1021 flows toward the center of the inlet 1032 in the width direction; however, the inlet 1032 is not closed and the opening end remains in communication with the outside. In other words, the function of the inlet 1032 for introducing a fluid is maintained.

Fig. 13(f) is a schematic cross-sectional view illustrating the microfluidic chip 1200 taken along the line C-C of Fig. 13(d). Due to post-baking on the hot plate 1025, portions of the resin on one side of the partition layer 1021 facing away from the substrate 1020 (on the upper side of the partition layer 1021) flow from right and left and are joined together near the center of the channel section 1023 in the width direction to form the upper lid layer 1022. Thus, according to the present embodiment, the lid component (upper lid layer) covering the channel section of the microfluidic chip can be formed using existing photolithography techniques without using an adhesive. Thus, adhesive components are prevented from eluting into the channel and inhibiting reactions of the solution in the channel.

Also, omission of an adhesive can avoid quality deterioration due to poor bonding which would have been caused by uneven film thickness of the adhesive. Furthermore, the production method can be simplified compared to the case where a partition layer and an upper lid layer are bonded together using an intermediate component such as an adhesive. In addition, poor bonding can be more reliably suppressed by integrating the partition layer 1021 with the upper lid layer 1022.

As described above, the method of producing the microfluidic chip 1200 according to the present embodiment includes steps of applying a partition resin onto a substrate 1010 (step S1001), exposing the partition resin (step S1005), developing and cleaning the exposed partition resin to form a partition layer 1021 defining a channel section 1023 on the substrate 1020 (steps S1006 and S1007), and heat-treating the partition layer 1021 and fluidizing the partition resin to form an upper lid layer 1022.

Thus, the upper lid layer 1022 can be joined to and integrated with the partition layer 1021 by welding as a part thereof, without using an adhesive, and therefore, adhesive components are prevented from eluting into the channel section 1023 and inhibiting reactions of the solution in the channel.

### (2.2.3) Effects of Embodiment 2-2

The microfluidic chip 1200 described above has the following effects.
(1) The microfluidic chip 1200 according to the present embodiment may be integrated with the partition layer 1021.
   Thus, poor bonding between the partition layer 1021 and the upper lid layer 1022 can be more reliably suppressed.
(2) The method of producing the microfluidic chip 1200 according to the present embodiment includes steps of applying a partition resin onto a substrate 1010 (step S1001), exposing the partition resin, developing and cleaning the exposed partition resin to form a partition layer 1021 defining a channel section 1023 on the substrate 1020, and heat-treating the partition layer 1021 and fluidizing the partition resin to form an upper lid layer 1022.

Thus, the upper lid layer 1022 can be joined to and integrated with the partition layer 1021 by welding as a part thereof, without using an adhesive, and therefore, adhesive components are prevented from eluting into the channel section 1023 and inhibiting reactions of the solution in the channel.

### <Second Example>

The following description relates to specific examples of the microfluidic chip and the method of producing the same, according to the second embodiment of the present disclosure. The second embodiment of the present disclosure should not be limited to the following examples.

### (Example 2-1)

A microfluidic chip and a method of producing the same according to Embodiment 2-1 will be described.

First, a partition layer photosensitive resin was applied onto a glass substrate to form a first photosensitive resin layer. The partition layer photosensitive resin used herein was a negative transparent liquid resin (negative liquid resist) containing an epoxy resin. The glass transition temperature (Tg) of the photosensitive resin (negative liquid resist) was 160°C. The negative liquid resist was applied onto the glass substrate using a spin coater set to 1,100 rpm and 30 sec. The rotation speed and time of the spin coater were controlled so that the thickness of the first photosensitive resin layer would be 50 µm.

Next, the partition layer photosensitive resin (negative liquid resist) underwent heat treatment (pre-baking) on a hot plate, for the purpose of removing residual solvent contained therein. Pre-baking was performed at 90°C for 20 minutes.

Next, an upper lid photosensitive resin was applied onto the first photosensitive resin layer to form a second photosensitive resin layer. The upper lid photosensitive resin was similar to the negative liquid resist mentioned above, except for having a glass transition temperature (Tg) (110°C) which was lower than that of the partition layer photosensitive resin by 50°C. Pre-baking was performed under conditions similar to the partition layer photosensitive resin to remove residual solvent contained in the upper lid photosensitive resin.

Next, the photosensitive resin layers (first and second photosensitive resin layers) on the glass substrate were exposed to draw a channel pattern. Specifically, the photosensitive resin was subjected to pattern exposure via a photomask having a microchannel pattern array. The photomask was formed of a light-shielding film with a double-layer structure of chromium and chromium oxide. A proximity exposure device was used for the exposure. The exposure device had a high-pressure mercury lamp as a light source, with a cut filter that was an i-line filter inserted for exposure. The exposure dose was 170 mJ/cm².

Next, the exposed photosensitive resin layers were developed to form a channel pattern. Specifically, the photosensitive resin layers were developed for 60 seconds using an alkaline developer (TMAH 2.38%) to dissolve the unexposed portions and to pattern a channel structure.

Subsequently, shower cleaning was performed using ultra-pure water to remove the developer from the photosensitive resin layers on the substrate, followed by drying using a spin dryer. At this stage, the upper side of the channel section of the channel pattern opposite from the glass substrate was open. Also, the second photosensitive resin layer remained on the partition layer.

Next, the channel pattern was subjected to heat treatment (post-baking) in an oven at 110°C for 30 minutes. In this case, due to reflow of the photosensitive resin, the upper parts of the channel pattern, i.e., parts of the second photosensitive resin layer, flowed toward the center of the channel section, and opposing parts of the second photosensitive resin layer on the partition layer were joined together. Thus, an upper lid layer was formed, thereby obtaining a microfluidic chip of the present example. At both ends of the channel section, an inlet and an outlet, as through-holes, were formed without being closed by the upper lid layer.

In the microfluidic chip of the present example prepared as described above, the partition layer and the upper lid layer are joined together by welding without using an adhesive, and the upper lid layer is formed by fluidizing the upper lid layer resin (second photosensitive resin layer) during post-baking. Thus, adhesive components are prevented from eluting into the channel and inhibiting reactions of the solution in the channel.

10 µL of a colored reaction solution was collected with a pipette and introduced into the microfluidic chip of the present example from the inlet of the channel, and the state of liquid transfer was microscopically observed. The introduced reaction solution smoothly passed through the channel without leakage, and liquid transfer was good. In this way, the fluid passed smoothly through the microfluidic chip of the present example without leakage, and thus it was confirmed that the microfluidic chip was imparted with basic characteristics of a microfluidic chip.

### (Example 2-2)

A microfluidic chip and a method of producing the same according to Example 2-2 will be described.

A microfluidic chip according to Example 2-2 was prepared as in Example 2-1, except that the partition layer was formed integrally with the upper lid layer.

Specifically, first, as in Example 2-1, a partition layer photosensitive resin was applied onto a glass substrate to form a first photosensitive resin layer, followed by pre-baking.

Next, the first photosensitive resin layer on the glass substrate was exposed under conditions similar to Example 2-1 to draw a channel pattern.

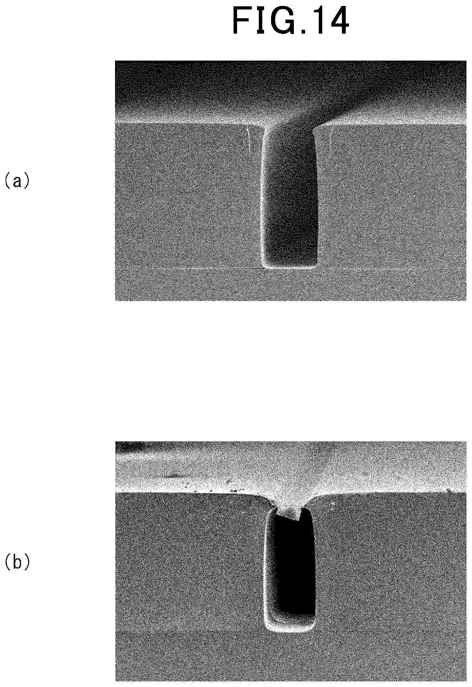

Next, the exposed first photosensitive resin layer was developed under conditions similar to Example 2-1, followed by removing the developer and drying using a spin dryer, thereby forming a channel pattern. Fig. 14(a) shows an example of a cross-sectional SEM image of the channel pattern at this point.

In Fig. 14(a), the recess sandwiched between opposing parts of the partition layer is the channel section of the channel pattern. As shown in Fig. 14(a), in the channel section of the channel pattern after removal of the developer and drying, the upper side thereof opposite from the glass substrate is open. The opening on the upper side of the channel section is similar in Example 2-1.

Next, the channel pattern was subjected to heat treatment (post-baking) in an oven at 160°C for 30 minutes. In this case, due to reflow of the photosensitive resin, the upper parts of the channel pattern, i.e., the upper parts of the partition layer, flowed toward the center of the channel section, and portions of the photosensitive resin (negative liquid resist) on opposing parts of the partition layer were joined together. Thus, a microfluidic chip of the present example was obtained.

Fig. 14(b) shows an example of a cross-sectional SEM image of the microfluidic chip after post-baking. As shown in Fig. 14(b), it was confirmed that the upper parts of the channel pattern after post-baking, i.e., portions of the photosensitive resin on parts of the partition layer, were fluidized and joined together near the center of the channel section to form an upper lid layer.

As shown in Fig. 14(b), it was confirmed that the upper lid layer formed due to fluidization of the partition layer had a reduced thickness toward the center of the channel section to form a recess. As shown in Fig. 14(b), it was confirmed that the cross section of the channel section had rounded corners. These points are similar to Example 2-1 in which the partition layer and the upper lid layer are separate bodies.

At both ends of the channel section, an inlet and an outlet, as through-holes, were formed without being closed by the upper lid layer.

In the microfluidic chip of the present example prepared as described above, an upper lid layer is formed due to the upper parts of the partition layer being fluidized by post-baking, and there is no adhesive used between the partition layer and the upper lid layer. Thus, adhesive components are prevented from eluting into the channel and inhibiting reactions of the solution in the channel.

Liquid transfer testing was performed for the microfluidic chip of the present example as in Example 2-1. The reaction solution introduced in the testing smoothly passed through the channel without leakage, and liquid transfer was good. In this way, the fluid passed smoothly through the microfluidic chip of the present example without leakage, and thus it was confirmed that the microfluidic chip was imparted with basic characteristics of a microfluidic chip.

For example, the second embodiment can have the following configurations.
(1) A microfluidic chip including
   a base section;
   a partition section forming a channel on the base section; and
   an upper lid section formed on the surface of the partition section facing away from the base section, wherein
   no adhesive layer is provided between the partition section and the upper lid section.
(2) A microfluidic chip including
   a base section;
   a partition section forming a channel on the base section; and
   an upper lid section formed on the surface of the partition section facing away from the base section to serve as a lid for the channel, wherein
   the upper lid section and the partition section are joined together by welding.
(3) A microfluidic chip including
   a channel; and
   an upper lid section serving as a lid for the channel, wherein
   the upper lid section is made of a material that is a resin having thermal fluidity; and
   the channel has a cross section having rounded corners.
(4) The microfluidic chip according to any one of (1) to (3), wherein
   the upper lid section is formed separately from or integrally with a partition section forming the channel.
(5) The microfluidic chip according to any one of (1) to (4), wherein
   the upper lid section has a recess.
(6) The microfluidic chip according to any one of (1) to (5), wherein
   the partition section forming the channel and the upper lid section are each made of a resin material; and
   the resin material is a photosensitive resin having photosensitivity to light with a wavelength of 190 nm or more and 400 nm or less in the UV light region.
(7) The microfluidic chip according to any one of (1) to (6), wherein
   the upper lid section is a separate body from the partition section forming the channel and has a glass transition temperature lower than that of the partition section.
(8) The microfluidic chip according to any one of (1) to (7), wherein
   the upper lid section is a separate body from the partition section forming the channel and has an exposure sensitivity higher or lower than that of the partition section.
(9) A method of producing a microfluidic chip, including steps of
   applying a photosensitive resin onto a base section;
   exposing the applied photosensitive resin;
   developing and cleaning the exposed photosensitive resin to form a partition section defining a channel on the base section; and
   heat-treating the partition section and fluidizing the photosensitive resin to form an upper lid section for the channel.
(10) A method of producing a microfluidic chip, including steps of
   applying a first photosensitive resin onto a base section;
   applying a second photosensitive resin onto the applied first photosensitive resin;
   exposing the first photosensitive resin and the second photosensitive resin;
   developing and cleaning the exposed photosensitive resin and the exposed second photosensitive resin to form a partition section defining a channel on the base section; and
   heat-treating and fluidizing the second photosensitive resin on the partition section to form an upper lid section for the channel.

### 3. Third embodiment

A third embodiment of the present disclosure will be described. The third embodiment of the present disclosure relates to a microfluidic chip and a method of producing a microfluidic chip.

As mentioned above, in µ-TAS, micrometer-sized channels (microchannels) formed on a substrate are often used. Such substrates are called chips, microchips, microfluidic chips, etc.

Such microfluidic chips based on the conventional art have been prepared, for example, by bonding multiple structural components together, such as glass, plastic, resin, and metal. In generally used methods, these structural components are bonded together, with intermediate components other than these structural components interposed therebetween. As the intermediate materials, adhesives have been used. In this case, after forming channels on the surface of a first structural component (e.g., the surface of a substrate), an adhesive is applied to the surface of a partition section forming the channels, and a second structural component (lid component) serving as a lid of the channels is bonded to the first structural component to form a microfluidic chip.

In the conventional microfluidic chips in which structural components are bonded together via an adhesive, no adhesive is applied to channel sections of the microfluidic chips.

However, in recent years, the surface area of a channel section has increased as the channel pattern structure has become more complicated, and this has inevitably reduced the surface area for applying an adhesive to bond the partition section and the lid component together, i.e., the region (bonding region) used for bonding the partition section and the lid component. Accordingly, such conventional bonding methods have raised an issue of reducing bonding strength due to insufficient amounts of adhesive applied, and resultantly causing poor bonding.

The third embodiment of the present disclosure aims to provide a microfluidic chip and a method of producing the microfluidic chip, which can increase the area of the bonding region and can improve the bonding strength between the partition section and the lid component.

In order to achieve the above aim, a microfluidic chip according to an aspect of the third embodiment includes a base section, a partition section forming a channel on the base section, a cover section that is located on one side of the partition section facing away from the base section to serve as a lid for the channel, and an intermediate component located between the cover section and the channel.

A method of producing a microfluidic chip according to an aspect of the third embodiment includes steps of applying a photosensitive resin onto a base section, exposing the applied photosensitive resin, developing and cleaning the exposed photosensitive resin to form a partition section defining a channel on the base section, heat-treating the partition section and fluidizing the photosensitive resin to form an intermediate component overlapping at least part of the channel in plan view, and bonding a cover section to one side of the partition section facing away from the base section, via the intermediate component.

A method of producing a microfluidic chip according to another aspect of the third embodiment includes steps of applying a first photosensitive resin onto a base section, applying a second photosensitive resin onto the applied first photosensitive resin, exposing the first photosensitive resin and the second photosensitive resin, developing and cleaning the exposed first photosensitive resin and the exposed second photosensitive resin to form a partition section defining a channel on the base section, heat-treating and fluidizing the second photosensitive resin on the partition section to form an intermediate component overlapping at least part of the channel in plan view, and bonding a cover section to one side of the partition section facing away from the base section, via the intermediate component.

According to an aspect of the third embodiment, there can be provided a microfluidic chip that can increase the area of the bonding region and can improve the bonding strength between the partition section and the lid component.

A microfluidic chip according to the third embodiment of the present disclosure will be described. In the following description, the substrate side of the microfluidic chip may be referred to as lower, and the opposite side of the microfluidic chip from the substrate side (lid component side) may be referred to as upper.

As a result of diligent studies, the present inventors have found that, in a microfluidic chip, the area of the bonding region for bonding a partition section forming a channel to a lid component can be increased by providing an intermediate component between the partition section and the lid component. Thus, the present inventors have invented a microfluidic chip and a method of producing the microfluidic chip, which can improve an adhesive strength between a partition section and a lid component.

Referring to the drawings, some modes (Embodiments 3-1, 3-2 and 3-3) of the third embodiment will be described.

### (3.1) Embodiment 3-1

### (3.1.1) Basic configuration of microfluidic chip

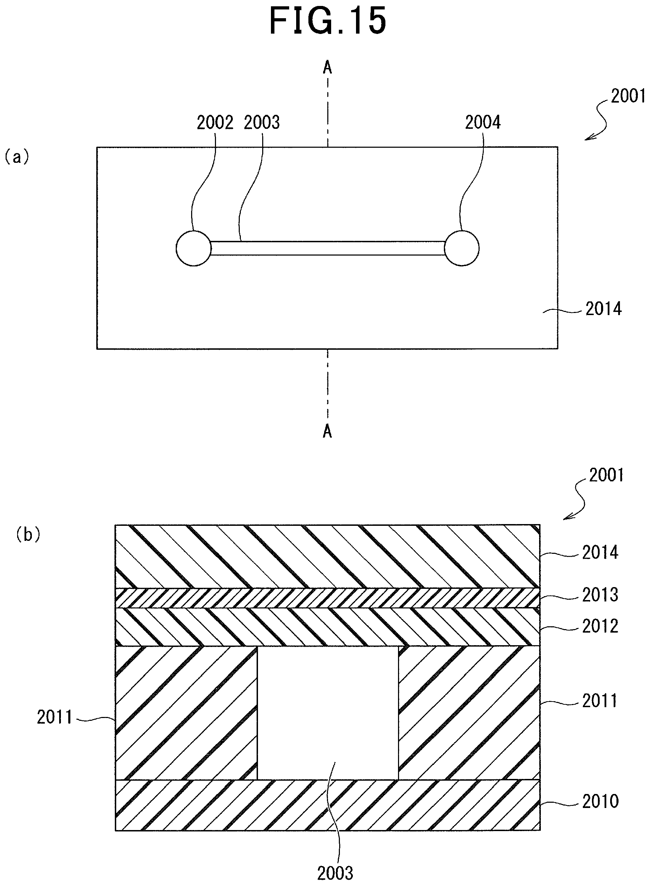

Fig. 15 is a set of schematic diagrams illustrating a configuration example of a microfluidic chip 2001 according to Embodiment 3-1 of the present disclosure (hereinafter referred to as the present embodiment). Specifically, Fig. 15(a) is a schematic plan view illustrating the microfluidic chip 2001 according to the present embodiment. Fig. 15(b) is a schematic cross-sectional view illustrating a cross section of the microfluidic chip 2001 taken along the line A-A of Fig. 15(a).

As shown in Fig. 15(a), the microfluidic chip 2001 includes an inlet 2002 for introducing a fluid (e.g., liquid), a channel section 2003 through which the fluid introduced from the inlet 2002 flows, and an outlet 2004 for discharging the fluid from the channel section 2003. In the microfluidic chip 2001, the upper part of the channel section 2003 is covered with a cover layer 2014, and the inlet 2002 and the outlet 2004 are through-holes formed through the cover layer 2014 and other layers laminated on the channel side of the cover layer 2014. Details of the cover layer 2014 will be described later.

Fig. 15(a) shows the channel section 2003 that can be seen through a transparent cover layer 2014.

The microfluidic chip 2001 may be provided with at least one or more inlets 2002 and outlets 2004, or may be provided with multiple of each. The microfluidic chip 2001 may be provided with multiple channel sections 2003, or may be designed such that the fluid introduced from the inlet 2002 can be merged or separated.

Details of the structural components of the channel section 2003 of the microfluidic chip 2001 will be described later. As shown in Fig. 15(b), the microfluidic chip 2001 includes a substrate (example of the base section) 2010, a partition layer (example of the partition section) 2011 forming a channel on the substrate 2010, a cover layer (example of the cover section) 14 located on one side of the partition layer 2011 facing away from the substrate 2010 to serve as a lid for the channel section 2003, and an intermediate layer (example of the intermediate component) 2012 located between the cover layer 2014 and the channel section 2003.

In the microfluidic chip 2001, the channel section 2003 through which the fluid introduced from the inlet 2002 flows is a region surrounded by the substrate 2010, the partition layer 2011, and the intermediate layer 2012. The channel section 2003 is defined within the partition layer 2011 provided on the substrate 2010 and is covered with the intermediate layer 2012 serving as a lid component on the side opposite from the substrate 2010. The intermediate layer 2012 is configured to define the channel together with the partition layer 2011 and corresponds to a simple upper lid of the channel section 2003. Therefore, the intermediate layer 2012 may also be referred to as an upper lid layer. In other words, the channel section 2003 is a space configured by the substrate 2010, partition layer 2011, and intermediate layer 2012. On the upper part of the channel section 2003, the cover layer 2014 is provided via the intermediate layer 2012 and an adhesive layer 2013 described later. A fluid is introduced into the channel section 2003 from the inlet 2002 (see Fig. 15(a)), which passes through the cover layer 2014, the adhesive layer 2013 laminated on the channel section 2003 side of the cover layer 2014, and the intermediate layer 2012, and the fluid that has passed through the channel section 2003 is discharged from the outlet 2004. The outlet 2004 is also a through-hole passing through the cover layer 2014, the adhesive layer 2013, and the intermediate layer 2012.

The microfluidic chip 2001 of the present embodiment includes no adhesive layer between the partition layer 2011 and the intermediate layer 2012, details of which will be described later. In the present embodiment, the partition layer 2011 and the intermediate layer 2012 are joined together by welding. Herein, the adhesive layer refers to a layer containing an adhesive and used for bonding multiple structural components together. In the microfluidic chip 2001, the partition layer 2011 and the intermediate layer 2012 are joined (bonded) together by welding without providing an adhesive layer therebetween, so that adhesive components are prevented from eluting into the channel and inhibiting reactions of the solution in the channel.

As shown in Fig. 15(b), the partition layer 2011 and the intermediate layer 2012 are separate bodies. Herein, separate bodies refers to the partition layer 2011 and the intermediate layer 2012, for example, being formed of different resin materials. In this case, as a resin material for forming the intermediate layer 2012, a material having favorable characteristics as an upper lid of the channel section 2003, i.e., as an intermediate component, such as having glass transition temperature or exposure sensitivity different from those of the partition layer 2011, can be adequately selected. The present disclosure is not limited to this, but the partition layer 2011 and the intermediate layer 2012 may be defined to be separate bodies based on these layers forming a laminated structure with an interface formed therebetween. In this case, the partition layer 2011 and the intermediate layer 2012 may be formed using the same resin material.

### (3.1.1.1) Substrate

The substrate 2010 serves as a base of the microfluidic chip 2001, and the channel section 2003 is configured by the partition layer 2011 provided on the substrate 2010. In other words, the substrate 2010 and the partition layer 2011 configure a body of the microfluidic chip 2001.

The substrate 2010 can be formed using either a translucent material or a non-translucent material. For example, if the state inside the channel section 2003 (state of the fluid) is detected and observed using light, a material having good transparency to light can be used. As the translucent material, resins, glass, etc. can be used. Examples of the resin used as a translucent material for forming the substrate 2010 include acrylic resins, methacrylic resins, polypropylenes, polycarbonate resins, cycloolefin resins, polystyrene resins, polyester resins, urethane resins, silicone resins, and fluororesins, which are preferred from the perspective of suitability for forming the body of the microfluidic chip 2001.

For example, if the state inside the channel section 2003 (state of the fluid) is not required to be detected and observed using light, a non-translucent material may be used. Examples of non-translucent materials include silicon wafers and copper plates. The thickness of the substrate 2010 is not particularly limited, but is preferred to be in the range of 10 µm (0.01 mm) or more and 10 mm or less because some degree of rigidity is required in the channel formation process.

### (3.1.1.2) Partition layer

The partition layer 2011 is provided on the substrate to configure the channel section 2003. The partition layer 2011 can be formed using a resin material. For example, a photosensitive resin can be used as the resin material of the partition layer 2011.

The photosensitive resin forming the partition layer 2011 is preferred to have photosensitivity to light having a wavelength of 190 nm or more and 400 nm or less in the UV light region. As the photosensitive resin, photoresists such as liquid resists and dry film resists can be used. These photosensitive resins may be of positive type with which the photosensitive area becomes soluble, or may be of negative type with which the photosensitive area becomes insoluble. Examples of the photosensitive resin composition suitable for forming the partition layer 2011 in the microfluidic chip 2001 include negative radical type photosensitive resins containing alkalisoluble polymers, addition polymerizable monomers, and photopolymerization initiators. Examples of the photosensitive resin materials include acrylic resins, epoxy resins, polyamide resins, polyimide resins, polyurethane resins, polyester resins, polyether resins, polyolefin resins, polycarbonate resins, polystyrene resins, norbornene resins, phenol novolak resins, and other photosensitive resins. These resins may be used singly, or may be used by mixing or copolymerizing a plurality of them.

In the present embodiment, the resin material used for the partition layer 2011 is not limited to photosensitive resins, but may be, for example, silicone rubber (PDMS: polydimethylsiloxane) or synthetic resins. Examples of the synthetic resins include polymethyl methacrylate resins (PMMA), polycarbonates (PC), polystyrene resins (PS), polypropylenes (PP), cycloolefin polymers (COP), and cycloolefin copolymers (COC). The resin material for the partition layer 2011 is preferred to be appropriately selected according to usage.

The thickness of the partition layer 2011, i.e., the height of the channel section 2003, on the substrate 2010 is not particularly limited, but is required to be larger than the substances (e.g., drugs, bacteria, cells, red blood cells, white blood cells, etc.) to be analyzed and tested which are contained in the fluid introduced into the channel section 2003. Therefore, the thickness of the partition layer 2011, i.e., the height (depth) of the channel section 2003, is preferred to be in the range of 1 µm or more and 500 µm or less, more preferred to be in the range of 10 µm or more and 100 µm, and even more preferred to be in the range of 40 µm or more and 60 µm or less.

Similarly, since the width of the channel section 2003 is required to be larger than the substances to be analyzed and tested, the width of the channel section 2003 defined within the partition layer 2011 is preferred to be in the range of 1 µm or more and 500 µm or less, more preferred to be in the range of 10 µm or more and 100 µm, and even more preferred to be in the range of 10 µm or more and 30 µm or less.

The channel length determined by the partition layer 2011 is required to ensure sufficient reaction time for the reaction solution, and therefore, is preferred to be in the range of 10 mm or more and 100 mm or less, more preferred to be in the range of 30 mm or more and 70 mm or less, and even more preferred to be in the range of 40 mm or more and 60 mm or less.

### (3.1.1.3) Intermediate layer

As shown in Fig. 15(b), the intermediate layer 2012 in the microfluidic chip 2001 of the present embodiment is a lid component covering the channel section 2003. More specifically, in the present embodiment, the intermediate layer 2012 overlaps the entirety of the channel section 2003 in plan view. As mentioned above, the intermediate layer 2012 is provided to the surface of the partition layer 2011 facing away from the substrate 2010, and faces the substrate 2010 via the partition layer 2011. More specifically, as shown in Fig. 15(b), the side end portions of the intermediate layer 2012, as viewed in cross section, are supported by the partition layer 2011, the center region thereof faces the substrate 2010, and the center region defines the upper part of the channel section 2003. The microfluidic chip 2001 of the present embodiment is provided with the intermediate layer 2012 between the cover layer 2014 as a lid component, and the channel section 2003 and the partition layer 2011, and therefore, the physical strength is improved so that deformation of the channel section 2003 and liquid leakage and the like due to the deformation can be reduced. In other words, the intermediate layer 2012 corresponds to a reinforcement component.

The intermediate layer 2012 can be formed using either a translucent material or a non-translucent material. For example, if the state inside the channel is detected and observed using light, a material having good transparency to light can be used. A resin can be used as the translucent material. The resin used for forming the intermediate layer 2012 may be a photosensitive resin similarly to the partition layer 2011.

The photosensitive resin used for the intermediate layer 2012 is a resin having thermal fluidity. If a photosensitive resin having thermal fluidity is used as the material of the intermediate layer 2012, the intermediate layer 2012 can be formed in the microfluidic chip 2001 without using an adhesive. Thus, adhesive components are prevented from eluting into the channel and inhibiting reactions of the solution in the channel.

In the microfluidic chip 2001 according to the present embodiment, the intermediate layer 2012 can be formed on the partition layer 2011 more easily than in the conventional art, using a resin (photosensitive resin in the present example) having thermal fluidity as a material for the intermediate layer 2012. This can eliminate complexity of the production method.

The photosensitive resin having thermal fluidity is preferred to have a melt flow rate (MFR) in the range of 1 g/10 min or more and 100 g/10 min (230°C) or less. This can facilitate formation of the intermediate layer 2012 in the process of production described later.

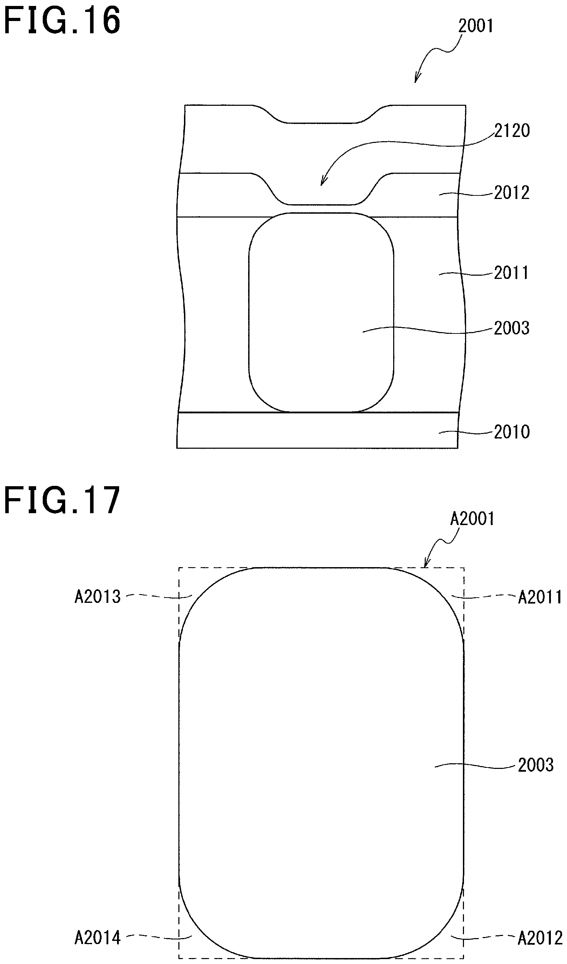

As will be described in detail later, the intermediate layer 2012 is formed by heat-treating and fluidizing (reflowing) a photosensitive resin having thermal fluidity formed on the partition layer 2011, and allowing it to flow onto the channel section 2003. Therefore, as shown in Fig. 16, the intermediate layer 2012 has a recess 2120. In other words, the intermediate layer 2012 has a recessed region. Specifically, the intermediate layer 2012 has a thickness which is reduced from the partition layer 2011 side toward the center of the channel section 2003, forming the recess 2120 as viewed in cross section.

The intermediate layer 2012 may be formed using a resin material with a glass transition temperature (Tg) lower than that of the partition layer 2011. For example, the glass transition temperature of the intermediate layer 2012 may be lower than that of the partition layer 2011 by 30°C to 50°C. In this case, the photosensitive resin forming the partition layer 2011 is hardly fluidized because the glass transition temperature (Tg) thereof is higher than that of the photosensitive resin forming the intermediate layer 2012. Accordingly, when forming the intermediate layer 2012, the partition layer 2011 is suppressed from being fluidized and changing the channel pattern.

The glass transition temperature (Tg) of the intermediate layer 2012 is preferred to be in the range of 100°C or more and 300°C or less.

The intermediate layer 2012 is preferred to have an exposure sensitivity in the range of 5 µC/cm² or more and 50 µC/cm² or less.

The intermediate layer 2012 may have an exposure sensitivity different from that of the partition layer 2011. For example, the exposure sensitivity of the intermediate layer 2012 may be higher than that of the partition layer 2011 by the range of 5 µC/cm² or more and 20 µC/cm² or less.

For example, the exposure sensitivity of the intermediate layer 2012 may be lower than that of the partition layer 2011 by the range of 5 µC/cm² or more and 20 µC/cm² or less.

Thus, the intermediate layer 2012 may be configured to have an exposure sensitivity higher or lower than that of the partition layer 2011.

If the exposure sensitivity is different between the intermediate layer 2012 and the partition layer 2011, the exposure dose may be set to a value matching the photosensitive resin forming the partition layer 2011, so that the opening width of the photosensitive resin forming the partition layer 2011, i.e., the width of the channel pattern, can be made sufficiently large. Thus, when fluidizing the resin material of the intermediate layer during formation of the intermediate layer 2012, if fluidization has also occurred in the partition layer resin, the opening width of the photosensitive resin forming the partition layer 2011 can be made sufficiently large and a sufficient space can be retained as the channel section 2003.

### (3.1.1.4) Channel configuration

Fig. 17 is a diagram illustrating an example of a cross section of the channel section 2003 of the microfluidic chip 2001 according to the present embodiment. In the present embodiment, the cross section of the channel section 2003 is preferred to have rounded corners (e.g., the sides of the channel section 2003 in a cross section are connected by arcs). Thus, the transfer speed or flow rate of the fluid (e.g., reaction solution) in the channel section 2003 can be stabilized, and the substances to be tested can be suppressed from being retained at the corners.

Referring to Fig. 17, a cross section of the channel section 2003 in the present embodiment will be described. Fig. 17 is a schematic cross-sectional view illustrating an enlarged cross section of the channel section 2003 having rounded corners. As shown in Fig. 17, there is a difference in area between a virtual cross section A2001 when the cross-sectional shape of the channel section 2003 is rectangular and the cross section of the channel section 2003 having rounded corners. Specifically, as shown in Fig. 17, the four corners of the cross section of the channel section 2003 each have an arc shape. Therefore, the area of the cross section of the channel section 2003 is smaller than that of the virtual cross section A2001 by the sum of the areas of the virtual corner portions A2011, A2012, A2013 and A2014.

In the present embodiment, the area of the cross section of the channel section 2003 having rounded corners may be in the range of 95% or more and 98% or less of the surface area of the virtual cross section A2001. Fig. 17 shows the channel section 2003 having a rectangular cross section with rounded corners; however, without being limited to this, the cross section may have shapes other than a rectangular shape (polygonal shape with rounded corners). In this case also, the area of the cross section of the channel section 2003 may be in the range of 95% or more and 98% or less of the area of the virtual cross section not having rounded corners.

The channel section 2003 is preferred to have a 10-point surface roughness Rz in the range of 0.001 µm or more and 0.03 µm or less. The 10-point surface roughness Rz of the channel section 2003 refers to the roughness of the side surfaces of parts of the partition layer 2011 on the channel section 2003 side, and the surface of the substrate 2010 on the channel section 2003 side.

If the 10-point surface roughness Rz is adequately designed within the above range, testing can be suitably performed according to the reaction solutions or the substances to be tested, and according to the desired testing conditions. For example, the surface roughness Rz of the channel section 2003 being in the range of 0.001 µm or more and 0.01 µm or less can reduce contact of the fluid (e.g., reaction solution) or substance to be tested introduced into the channel section 2003, with the inner surfaces of the channel section 2003 and can improve liquid transferability (e.g., liquid transfer speed or flow rate).

The surface roughness Rz of the channel section 2003 being greater than 0.02 µm and less than or equal to 0.03 µm can reduce the liquid transfer speed and can ensure time of stay in the channel section 2003, i.e., reaction time, of the fluids or substances to be tested introduced into the channel section 2003.

The surface roughness Rz of the channel section 2003 being greater than 0.01 µm and less than or equal to 0.02 µm can ensure both of an appropriate liquid transfer speed and reaction time of the fluids or substances to be tested in the channel section 2003.

The surface roughness of the channel section 2003 can be adequately controlled using a known etching method or the like during production of the microfluidic chip 2001.

### (3.1.1.5) Adhesive layer

Referring back to Fig. 15(b), in the microfluidic chip 2001 of the present embodiment, the adhesive layer 2013 is provided on the upper surface of the intermediate layer 2012, i.e., the surface facing away from the channel. The adhesive layer 2013 is used for bonding (adhering) between the intermediate layer 2012 and the cover layer 2014. In other words, the intermediate layer 2012 and the cover layer 2014 are bonded together via the adhesive layer 2013. The adhesive layer 2013 is an adhesive applied onto the intermediate layer 2012. The adhesive used for the adhesive layer 2013 may be adequately determined based on the affinity for the materials forming the intermediate layer 2012 and the cover layer 2014 or other factors, and is not particularly limited as long as the intermediate layer 2012 and the cover layer 2014 can be bonded together. Examples of the adhesive that can be used in the present embodiment include acrylic resin adhesives, urethane resin adhesives, and epoxy resin adhesives. A material having transparency is preferred to be used for the adhesive layer 2013. Thus, visibility can be improved when detecting and observing the state inside the channel via the intermediate layer 2012 and the cover layer 2014.

In the present embodiment, the adhesive layer 2013 is applied onto the intermediate layer 2012. Thus, adhesive components are prevented from eluting into the channel and inhibiting reactions of the solution in the channel.

In the conventional art, it has been required to apply exactly the right amount of adhesive to regions except for the through-holes (inlet and outlet) provided to the cover layer 2014, and grooves, i.e., channels, defined within the partition layer 2011, for example. With the pattern structures of microfluidic chips becoming more complicated in recent years, this method requires highly skilled workers to apply exactly the right amount of adhesive and also requires devices and techniques for achieving high coating skills. This has raised an issue of complicated production methods.

In the present embodiment, with the adhesive layer 2013 provided on the intermediate layer 2012, an adhesive can be evenly applied to the upper side of the partition layer 2011 and the channel section 2003, i.e., to the entire upper regions of the microfluidic chip 2001, regardless of the surface area of the channel pattern. Therefore, the cover layer 2014, as a lid component, can be easily bonded without requiring devices or techniques of particularly high level.

### (3.1.1.6) Cover layer

As shown in Fig. 15(b), the cover layer 2014 in the microfluidic chip 2001 of the present embodiment is a lid component covering the channel section 2003. As mentioned above, the cover layer 2014 is provided on one side of the partition layer 2011 facing away from the substrate 2010, and bonded to the partition layer 2011 via the intermediate layer 2012 which is part of the partition layer 2011. More specifically, as shown in Fig. 15(b), the cover layer 2014 is bonded to the intermediate layer 2012 using the adhesive layer 2013 and is indirectly bonded to the partition layer 2011 as viewed in cross section.

The cover layer 2014 can be formed using either a translucent material or a non-translucent material. For example, if the state inside the channel is detected and observed using light, a material having good transparency to light can be used. As the translucent material, resins, glass, etc. can be used. Examples of the resin for forming the cover layer 2014 include acrylic resins, methacrylic resins, polypropylenes, polycarbonate resins, cycloolefin resins, polystyrene resins, polyester resins, urethane resins, silicone resins, and fluororesins, which are preferred from the perspective of suitability for forming the body of the microfluidic chip 2001. The thickness of the cover layer 2014 is not particularly limited but is preferred to be in the range of 10 µm or more and 10 mm or less from the perspective of providing through-holes corresponding to the inlet 2002 and the outlet 2004 of the cover layer 2014. It is preferred that, before being bonded to the intermediate layer 2012, the cover layer 2014 is provided with holes in advance corresponding to the inlet 2002 for introducing a fluid (liquid) and the outlet 2004 for discharging the fluid.

As mentioned above, the cover layer 2014 is bonded to the adhesive layer 2013. The adhesive layer 2013 is evenly applied to the intermediate layer 2012 which is formed over substantially the entirety of the upper part of the microfluidic chip 2001. Therefore, in the microfluidic chip 2001 of the present embodiment, a large contact surface area, i.e., large bonding region, can be ensured between the adhesive layer 2013 on the intermediate layer 2012 and the cover layer 2014. Thus, the microfluidic chip 2001 of the present embodiment can suppress occurrence of poor bonding due to deterioration in adhesive strength, and can facilitate bonding of the cover layer 2014 to the partition layer 2011 via the intermediate layer 2012 by bonding the cover layer 2014 to the intermediate layer 2012 using the adhesive layer 2013.

The microfluidic chip 2001 of the present embodiment includes the intermediate layer 2012 serving as a simple upper lid for the channel section 2003; however, in the case of using only the intermediate layer 2012 as a lid component without providing the cover layer 2014, the strength (physical strength) as a microfluidic chip may have room for improvement, although practical durability can be achieved. In this regard, formation of the cover layer 2014 can further improve the strength as a microfluidic chip.

In this way, the microfluidic chip 2001 according to the present embodiment includes a substrate 2010, a partition layer 2011 forming a channel section 2003 on the substrate 2010, a cover layer 2014 located on one side of the partition layer 2011 facing away from the substrate 2010 to serve as a lid for the channel section, and an intermediate component located between the cover layer 2014 and the channel section 2003. Thus, the microfluidic chip 2001 of the present embodiment can increase the area of the bonding region for bonding the partition layer 2011 to the cover layer 2014, as a lid component, and can improve the adhesive strength between the partition section and the lid component.

### (3.1.2) Method of producing microfluidic chip

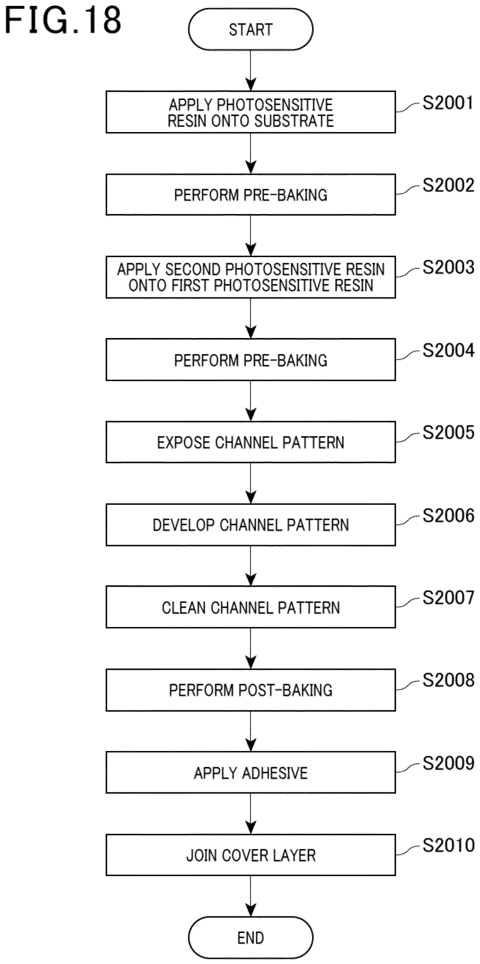

Next, a method of producing the microfluidic chip 2001 of the present embodiment will be described. Fig. 18 is a flowchart illustrating an example of a method of producing the microfluidic chip 2001 according to the present embodiment.

Description herein will be provided taking an example in which the partition layer 2011 is formed of a photosensitive resin.

### (Step S2001)

In the method of producing the microfluidic chip 2001 of the present embodiment, a resin for forming a partition layer 2011 (one example of the first photosensitive resin) is applied first onto a substrate 2010. Thus, a resin layer for forming a partition layer 2011 is provided on the substrate 2010. In the method of producing the microfluidic chip 2001 of the present embodiment, for example, a resin layer (first photosensitive resin layer) made of a photosensitive resin is formed on the substrate 2010.

The photosensitive resin layer is formed on the substrate 2010, for example, by applying a photosensitive resin onto the substrate 2010. Examples of the coating method include spin coating, spray coating, and bar coating, among which, spin coating is preferred from the perspective of film thickness controllability. The photosensitive resin that can be applied to the substrate 2010 can be in various forms such as liquid, solid, gel-like, and film-like forms. Of these forms, liquid resists are preferred to be used for forming the photosensitive resin layer. Such a liquid resist can be positive or negative type, which can be adequately determined according to the characteristics of the channel pattern.

The resin (e.g., photosensitive resin) may be applied to the substrate 2010 such that the thickness of the resin layer (e.g., photosensitive resin layer), i.e., the thickness of the partition layer 2011, will be in the range of 1 µm or more and 500 µm or less.

### (Step S2002)

Following formation of the first photosensitive resin layer of the partition resin on the substrate 2010, heat treatment (pre-baking treatment) is performed for the purpose of removing solvent contained in the resin (e.g., photosensitive resin) applied to the substrate 2010. In the method of producing the microfluidic chip 2001 of the present embodiment, pre-baking treatment is not essential but may be adequately performed under optimal conditions (temperature and duration) according to the characteristics of the resin used. For example, if the resin layer on the substrate 2010 is formed of a photosensitive resin, pre-baking may be performed under optimal temperature and duration adequately determined according the characteristics of the photosensitive resin. For the purpose of improving adhesion between the substrate and the photosensitive resin, the substrate may undergo an HMDS treatment or may be provided with a thin resin coating as necessary.

### (Step S2003)

Next, a resin for forming an intermediate layer 2012 (one example of the second photosensitive resin) is applied onto the first photosensitive resin layer. Thus, a resin layer for forming an intermediate layer 2012 is provided on the pre-baked partition resin. In the method of producing the microfluidic chip 2001 of the present embodiment, for example, a resin layer (second photosensitive resin layer) made of a photosensitive resin having thermal fluidity is formed on the substrate 2010. The second photosensitive resin layer can be formed on the substrate 2010 similarly to the first photosensitive resin layer formed at step S2001.

### (Step S2004)

Following formation of the second photosensitive resin layer of the upper lid resin on the first photosensitive resin layer of the partition resin, heat treatment (pre-baking treatment) is performed for the purpose of removing solvent contained in the intermediate layer resin applied to the first photosensitive resin layer. The pre-baking treatment of the present step is not essential but, similarly to the pre-baking treatment at step S2002, may be adequately performed under optimal conditions (temperature and duration) according to the characteristics of the resin used. For the purpose of improving adhesion between the first photosensitive resin layer and the second photosensitive resin, the substrate may undergo an HMDS treatment or may be provided with a thin resin coating as necessary. Thus, two photosensitive resin layers are formed on the substrate 2010.

As shown in steps S2001 to S2004, the two photosensitive resin layers (first and second photosensitive resin layers) for forming a partition layer 2011 and an intermediate layer 2012 are joined together by welding without using an adhesive. In other words, in the microfluidic chip 2001 of the present embodiment, the partition layer 2011 and the intermediate layer 2012 are joined together by welding. More specifically, in the microfluidic chip 2001 of the present embodiment, the partition layer 2011 and the intermediate layer 2012 can be joined together without using an adhesive layer (e.g., adhesive). Thus, adhesive components are prevented from eluting into the channel section 2003, and poor bonding that would have been induced by uneven thickness of the adhesive can be avoided.

The number of the photosensitive resin layers is not limited to two as in the present embodiment, but may be three or more. With the configuration described above, the intermediate layer 2012 can be said to be part of the partition layer 2011.

### (Step S2005)

Next, the resins (e.g., first and second photosensitive resins) applied to the substrate 2010 are exposed. Specifically, a channel pattern is drawn by exposure on the photosensitive resins applied to the substrate 2010. Exposure may be performed, for example, using an exposure device that uses UV light as a light source, or using a laser drawing device. Among them, proximity exposure using UV light as a light source or exposure using a contact exposure device is preferred. If a proximity exposure device is used, exposure is performed via a photomask with a channel pattern array for the microfluidic chip 2001. The photomask may be a photomask with a light-shielding film that has a double-layer structure of chromium and chromium oxide.

If the photosensitive resins (first and second photosensitive resin layers) applied to the substrate 2010 are positive resists, the exposed regions are dissolved, forming the channel section 2003, and the photosensitive resins in the unexposed regions remain as parts of the partition layer 2011 and the intermediate layer 2012. If the photosensitive resins applied to the substrate 2010 are negative resists, the photosensitive resins in the exposed regions remain as parts of the partition layer 2011 and the intermediate layer 2012, and the unexposed regions are dissolved, forming the channel section 2003. In this way, in the method of producing the microfluidic chip 2001 of the present embodiment, the partition layer 2011 configuring the channel section 2003 can be formed on the substrate 2010 using photolithography.

If a chemically amplified resist or the like is used for forming a resin layer on the substrate 2010, heat treatment (post-exposure baking: PEB) may be further performed after exposure to promote catalytic reaction of the acid generated due to exposure.

### (Step S2006)

Next, the exposed photosensitive resins are developed to form a channel pattern.

The development is performed, for example, using a spray-, dip- or paddle-type or other types of developing device, by reaction of the photosensitive resin with a developer. Examples of the developer include sodium carbonate aqueous solution, tetramethylammonium hydroxide, potassium hydroxide, and organic solvents. The developer is not limited to these, but an optimal developer may be adequately used according to the characteristics of the photosensitive resins. The concentration or the time of development can be adequately adjusted to optimal conditions according to the characteristics of the photosensitive resins.

### (Step S2007)

Next, the resin layers (photosensitive resin layers) on the substrate 2010 are cleaned to completely remove the developer used for development. Cleaning may be performed, for example, using a spray-, shower-, immersion-type or other types of cleaning device. An optimal cleaning solution may be adequately used from among, for example, pure water, isopropyl alcohol, and the like to remove the developer used for development. Cleaning is followed by drying using a spin dryer, IPA vapor dryer, natural drying, etc.

At this stage also, the second photosensitive resin layer of the intermediate layer resin remains on the partition layer 2011.

### (Step S2008)

Next, the channel pattern, i.e., the partition layer 2011 forming the channel section 2003 and the second photosensitive resin layer, undergoes heat treatment (post-baking). With this post-baking treatment, residual moisture generated during development or cleaning is removed. Also, with this post-baking treatment, an intermediate layer 2012 is formed to serve as a lid for the channel section 2003 and to define the upper part of the channel section 2003, being overlapped with the channel section 2003 in plan view. Specifically, this post-baking treatment can promote fluidization (reflow) of the intermediate layer resin, i.e., the photosensitive resin having fluidity, and form an intermediate layer 2012 for the channel section 2003. The post-baking treatment is performed under optimal temperature and duration determined according the characteristics of the intermediate layer resin. As shown in Fig. 15, the intermediate layer 2012 is a lid component covering the channel 2003 and thus is formed over the channel section 2003. The inlet 2002 and the outlet 2004 are open without being covered with the intermediate layer 2012.

For example, the post-baking treatment is performed using a hot plate, oven, etc. If the drying performed at the cleaning step S2007 is insufficient, the developer or moisture generated during cleaning may remain in the partition layer 2011. Solvent that has not been removed in the pre-baking treatment may also remain in the partition layer 2011. These materials can be removed by performing post-baking treatment.

In the present embodiment, an intermediate layer 2012 serving as a simple upper lid can be formed in this way. This can increase the contact area between the adhesive layer 2013 and the cover layer 2014, i.e., the bonding region for bonding the cover layer 2014 and the partition layer 2011.

### (Step S2009)

Next, an adhesive is applied onto the intermediate layer 2012 to form an adhesive layer 2013. The adhesive is evenly applied to the region other than the inlet 2002 and outlet 2004 which are through-holes as entrance and exit of the channel section 2003.

### (Step S2010)

Next, a cover layer is arranged on the adhesive layer 2013, and the intermediate layer 2012 and the cover layer 2014 are bonded together. Bonding the cover layer 2014 to the intermediate layer 2012, the strength as a microfluidic chip can be improved. Also, consequently, the cover layer 2014 is bonded to one side of the partition layer 2011 facing away from the substrate 2010 via the intermediate layer 2012 to serve as a lid component for the microfluidic chip 2001.

It is preferred that the cover layer 2014 is provided, in advance, with holes corresponding to the inlet 2002 and the outlet 2004 (see Fig. 15(a)) before being bonded to the intermediate layer 2012. This makes it possible to suppress dust or contamination issues, compared to the case of forming holes after bonding of the intermediate layer 2012.

In the present disclosure, the method of bonding between the cover layer 2014 and the intermediate 2012 is not limited to the method using an adhesive (adhesive layer 2013). For example, the cover layer 2014 and the intermediate 2012 may be bonded together using thermocompression using a thermocompression machine or heat rolling machine.

Other than the method using thermocompression, the method of bonding the cover layer 2014 and the intermediate 2012 may be a bonding method in which the bonding surfaces of the intermediate layer 2012 and the cover layer 2014 undergo surface modification treatment.

As a bonding method by performing surface modification treatment, a plasma treatment, corona treatment, excimer laser treatment, or the like can be mentioned. In this case, an optimal treatment may be adequately selected for improving reactivity on the surface of the intermediate layer 2012, according to the affinity and adhesion compatibility between the intermediate layer 2012 and the cover layer 2014. If bonding is performed using a surface modification treatment, step S2009 may be omitted, and the intermediate layer 2012 and the cover layer 2014 may be bonded together at step S2010.

As described above, the method of producing the microfluidic chip 2001 according to the present embodiment includes steps of applying a partition resin onto a substrate 2010 (step S2001), applying an intermediate layer resin onto the applied partition resin (step S2003), exposing the partition resin and the intermediate layer resin (step S2005), developing and cleaning the exposed partition resin and the exposed intermediate layer resin to form a partition layer 2011 defining a channel section 2003 on the substrate 2010 (steps S2006 and S2007), heat-treating and fluidizing the intermediate layer resin on the partition layer 2011 to form an intermediate layer 2012 overlapping the channel section 2003 in plan view (step S2008), and bonding a cover layer 2014 on one side of the partition layer 2011 facing away from the substrate 2010 via the intermediate layer 2012 (step S2010).

Thus, the area of the bonding region for bonding the partition layer 2011 to the cover layer 2014, as a lid component, can be increased, and the adhesive strength between the partition section and the lid component can be improved. Furthermore, the partition layer 2011 and the intermediate layer 2012 can be joined together by welding without using an adhesive, so that adhesive components are prevented from eluting into the channel section 2003 and inhibiting reactions of the solution in the channel.

### (3.1.3) Details of steps of producing partition layer and intermediate layer

In the method of producing the microfluidic chip 2001 of the present embodiment, production steps are adjusted according to the physical properties of the partition resin for forming the partition layer 2011 and the intermediate layer resin for forming the intermediate layer 2012. A specific example will be described below.

### (3.1.3.1) Formation of partition layer and intermediate layer using resins having different glass transition temperatures

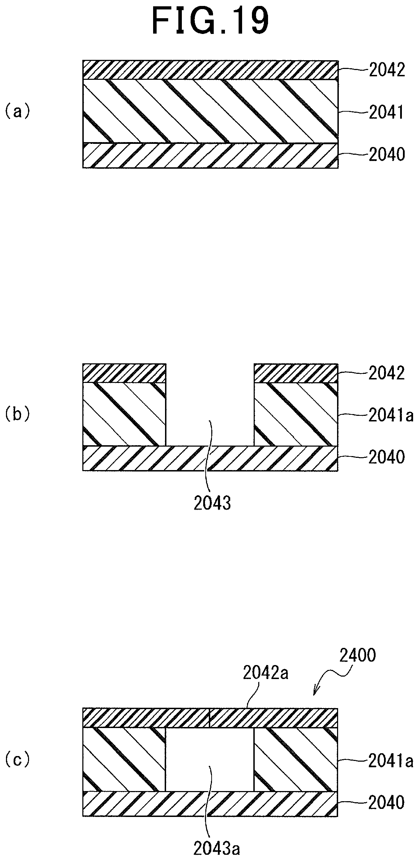

Referring to Fig. 19, a description will be given of forming the partition layer 2011 and the intermediate layer 2012 in the case where the partition resin and the intermediate layer resin have different glass transition temperatures. Fig. 19(a) is a schematic cross-sectional view illustrating a first photosensitive resin layer 2041 and a second photosensitive resin layer 2042 formed on a substrate 2040, Fig. 19(b) is a schematic cross-sectional view illustrating a channel pattern on the substrate 2040, and Fig. 19(c) is a schematic cross-sectional view illustrating a configuration of a microfluidic chip 2400 according to the present example.

In the present example, a description will be given of a method of producing a microfluidic chip under conditions in which the glass transition temperature (Tg) of the intermediate layer resin is lower than that of the partition resin [Glass transition temperature (Tg) of intermediate layer resin < Glass transition temperature (Tg) of partition resin].

As shown in Fig. 19(a), in the present example, a first photosensitive resin layer 2041 is formed by applying a partition photosensitive resin onto the substrate 2040 at the coating step S2001. The partition photosensitive resin is applied to the substrate 2040 with a desired thickness using spin coating, for example. In the present example, a second photosensitive resin layer 2042 is formed by applying an intermediate layer photosensitive resin onto the first photosensitive resin layer 2041 at step S2003. In the present example, the intermediate layer photosensitive resin forming the second photosensitive resin layer 2042 has a glass transition temperature (Tg) lower than that of the partition photosensitive resin forming the first photosensitive resin layer 2041. The second photosensitive resin layer 2042 is applied with a desired thickness onto the first photosensitive resin layer 2041 using spin coating, similarly to the first photosensitive resin layer 2041.

In the present example, at the exposure step S2005, a channel pattern is drawn, via a photomask, on the first and second photosensitive resin layers 2041 and 2042 applied to the substrate 2040. For example, the present example uses a proximity exposure device using light with a wavelength of 350 nm or more and 400 nm or less in the UV light region as a light source. Next, at the development step S2006, a channel pattern 2043 is formed by developing the exposed first and second photosensitive resin layers 2041 and 2042. For example, a sodium carbonate solution may be sprayed as a developer. Next, at step S2007, the developed first and second photosensitive resin layers 2041 and 2042 are cleaned to completely remove the developer. For example, ultra-pure water may be sprayed for cleaning. Thus, as shown in Fig. 19(b), a partition layer 2041a is formed defining the channel pattern 2043.

Next, the microfluidic chip, in which the channel pattern 2043 has been formed, undergoes heat treatment (post-baking) at step S2008. In the present example, heat treatment is performed using a hot plate at a temperature near the glass transition temperature (Tg) of the second photosensitive resin layer 2042. In the present example, heat treatment promotes fluidization (reflow) of the second photosensitive resin layer 2042, i.e., the intermediate layer resin, and portions of the intermediate layer resin on opposing right and left parts of the partition layer 2011 flow toward the center of the channel pattern 2043. The portions of the intermediate layer resin that have flowed from parts of the partition layer 2041a are joined together on the side opposite from the substrate 2040, i.e., on the upper side of the channel pattern 2043, to form an intermediate layer 2042a. Thus, as shown in Fig. 19(c), the upper part of the channel pattern 2043 is covered with the intermediate layer 2042a to form a channel section 2043a, thereby producing the microfluidic chip 2400.

As in the present example, if the glass transition temperature (Tg) of the first photosensitive layer 2041, i.e., the partition resin, forming the partition layer 2041a is higher than that of the second photosensitive resin layer 2042, i.e., the intermediate layer resin, the partition resin is not fluidized substantially even when the intermediate layer resin is fluidized (reflows) due to heat treatment. Accordingly, by satisfying the condition of [Glass transition temperature (Tg) of intermediate layer resin < Glass transition temperature (Tg) of partition resin], the intermediate layer 2042a can be easily formed by causing the intermediate layer resin to fluidize, without deforming the channel pattern due to fluidization of the intermediate layer resin.

### (3.1.3.2) Formation of partition layer and intermediate layer using resins with different exposure sensitivities (1)

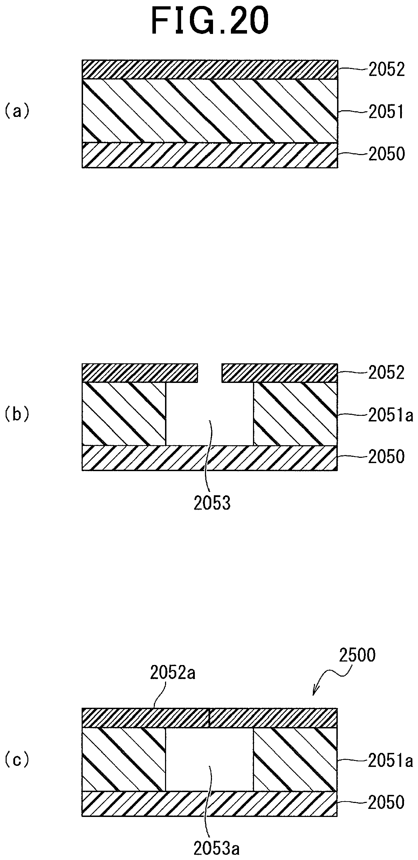

Referring to Fig. 20, a description will be given of forming the partition layer 2011 and the intermediate layer 2012 in the case where the partition resin and the intermediate layer resin have different exposure sensitivities. Fig. 20(a) is a schematic cross-sectional view illustrating a first photosensitive resin layer 2051 and a second photosensitive resin layer 2052 formed on a substrate 2050, Fig. 20(b) is a schematic cross-sectional view illustrating a channel pattern on the substrate 2050, and Fig. 20(c) is a schematic cross-sectional view illustrating a configuration of a microfluidic chip 2500 according to the present example.

In the present example, a description will be given of a method of producing a microfluidic chip under conditions in which the exposure sensitivity (C/cm²) of the intermediate layer resin is lower than that of the partition resin [Exposure sensitivity (C/cm²) of intermediate layer resin < Exposure sensitivity (C/cm²) of partition resin].

As shown in Fig. 20(a), in the present example, a first photosensitive resin layer 2051 is formed by applying a partition photosensitive resin onto the substrate 2050 at the coating step S2001. The partition photosensitive resin is applied to the substrate 2050 with a desired thickness using spin coating, for example. The first photosensitive resin layer 2051, i.e., the partition resin, is a positive resist. In the present example, a second photosensitive resin layer 2052 is formed by applying an intermediate layer photosensitive resin onto the first photosensitive resin layer 2051 at step S2003. The second photosensitive resin layer 2052, i.e., the intermediate layer resin, is a positive resist. In other words, in the present example, positive resists are used as the partition photosensitive resin and the intermediate layer photosensitive resin.

In the present example, the intermediate layer photosensitive resin forming the second photosensitive resin layer 2052 has an exposure sensitivity (C/cm²) lower than that of the partition photosensitive resin forming the first photosensitive resin layer 2051. The second photosensitive resin layer 2052 is applied with a desired thickness onto the first photosensitive resin layer 2051 using spin coating, similarly to the first photosensitive resin layer 2051. For example, a chemically amplified resist may be used as the partition resin for forming the first photosensitive resin layer 2051, and a non-chemically amplified resist may be used as the intermediate layer resin for forming the second photosensitive resin layer 2052.

In the present example, at the exposure step S2005, a channel pattern is drawn, via a photomask, on the first and second photosensitive resin layers 2051 and 2052 applied to the substrate 2050. For example, the present example uses a proximity exposure device using light with a wavelength of 350 nm or more and 400 nm or less in the UV light region as a light source. An optimal exposure dose is determined herein for the first photosensitive resin layer 2051. As mentioned above, in the present example, the exposure sensitivity of the intermediate layer resin forming the second photosensitive resin layer 2052 is lower than that of the partition resin forming the first photosensitive resin layer 2051. Therefore, although reaction is insufficient in the second photosensitive resin layer 2052 with the exposure dose matching the first photosensitive resin layer 2051, there is no problem in pattern resolution.

Next, at the development step S2006, a channel pattern 2053 is formed by developing the exposed first and second photosensitive resin layers 2051 and 2052. For example, a sodium carbonate solution may be sprayed as a developer. Next, at step S2007, the developed first and second photosensitive resin layers 2051 and 2052 are cleaned to completely remove the developer. For example, ultra-pure water may be sprayed for cleaning. Thus, as shown in Fig. 20(b), a partition layer 2051a is formed defining the channel pattern 2053. In the present example, as mentioned above, the second photosensitive resin layer 2052 is made of a resin having an exposure sensitivity lower than that of the first photosensitive resin layer 2051. Therefore, as shown in Fig. 20, the opening width in the exposed region in the second photosensitive resin layer 2052 is smaller than the opening width of the partition layer 2051a formed of the first photosensitive resin layer 2051.

Next, the microfluidic chip, in which the channel pattern 2053 has been formed, undergoes heat treatment (post-baking) at step S2008. In the present example, heat treatment is performed using a hot plate at a temperature near the glass transition temperatures (Tg) of the first and second photosensitive resin layers 2051 and 2052. In the present example, heat treatment promotes fluidization (reflow) of the second photosensitive resin layer 2052, i.e., the intermediate layer resin, and portions of the intermediate layer resin on right and left parts of the partition layer 2051a flow toward the center of the channel pattern 2053. The portions of the intermediate layer resin that have flowed from parts of the partition layer 2051a are joined together on the side opposite from the substrate 2050, i.e., on the upper side of the channel pattern 2053, to form an intermediate layer 2052a. Thus, as shown in Fig. 20(c), the upper part of the channel pattern 2053 is covered with the intermediate layer 2052a to form a channel section 2053a, thereby producing the microfluidic chip 2500.

In the present example, the glass transition temperature (Tg) of the first photosensitive resin layer 2051, i.e., the partition resin, forming the partition layer 2051a is equal to that of the second photosensitive resin layer 2052, i.e., the intermediate layer resin, forming the intermediate layer 2052a. Therefore, when the intermediate layer is fluidized (reflows), the partition resin is also fluidized. However, in the present example, the first and second photosensitive resin layers 2051 and 2052 are each formed of a positive resist, and the exposure sensitivity of the second photosensitive resin layer 2052 is lower than that of the first photosensitive resin layer 2051. Therefore, as shown in Fig. 20(b), the opening width of the second photosensitive resin layer 2052 is formed to be smaller than the opening width of the partition layer 2051a formed of the first photosensitive resin layer 2051, and the opening width of the partition layer 2051a formed of the first photosensitive resin layer 2051 is formed to be sufficiently larger than the opening width of the second photosensitive resin layer 2052.

Accordingly, the width of the channel section 2053a is reduced by more than the opening width of the channel pattern 2053 is, due to occurrence of fluidization in the first photosensitive resin layer 2051 with a degree that is approximately the same as in the second photosensitive resin layer 2052; however, as shown in Fig. 20(c), the width of the channel section 2053a is sufficiently ensured.

Accordingly, if the first and second photosensitive resin layers 2051 and 2052 are each formed of a positive resist and if the condition of [Exposure sensitivity of intermediate layer resin < Exposure sensitivity of partition resin] is satisfied, the intermediate layer resin and the partition resin can be fluidized to easily form the intermediate layer 2052a, while sufficiently retaining the width of the channel section.

### (3.1.3.3) Formation of partition layer and intermediate layer using resins with different exposure sensitivities (2)

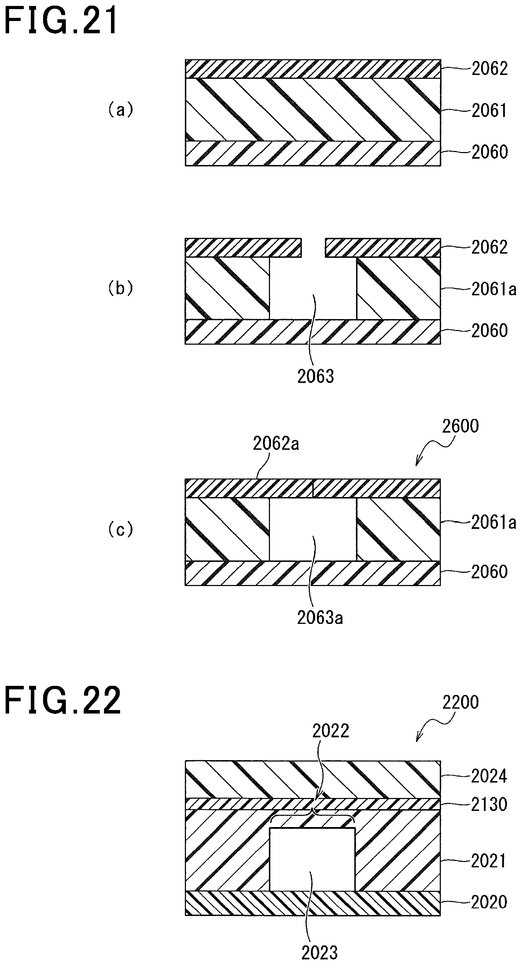

Referring to Fig. 21, a description will be given of forming the partition layer 2011 and the intermediate layer 2012 in the case where the partition resin and the intermediate layer resin have different exposure sensitivities. Fig. 21(a) is a schematic cross-sectional view illustrating a first photosensitive resin layer 2061 and a second photosensitive resin layer 2062 formed on a substrate 2060, Fig. 21(b) is a schematic cross-sectional view illustrating a channel pattern on the substrate 2060, and Fig. 21(c) is a schematic cross-sectional view illustrating a configuration of a microfluidic chip 2600 according to the present example.

In the present example, a description will be given of a method of producing a microfluidic chip under conditions in which the exposure sensitivity (C/cm²) of the intermediate layer resin is higher than that of the partition resin [Exposure sensitivity (C/cm²) of intermediate layer resin > Exposure sensitivity (C/cm²) of partition resin].

As shown in Fig. 21(a), in the present example, a first photosensitive resin layer 2061 is formed by applying a partition photosensitive resin onto the substrate 2060 at the coating step S2001. The partition photosensitive resin is applied to the substrate 2060 with a desired thickness using spin coating, for example. The first photosensitive resin layer 2061, i.e., the partition resin, is a negative resist. In the present example, a second photosensitive resin layer 2062 is formed by applying an intermediate layer photosensitive resin onto the first photosensitive resin layer 2061 at step S2003. The second photosensitive resin layer 2062, i.e., the intermediate layer resin, is a negative resist. In other words, in the present example, negative resists are used as the partition photosensitive resin and the intermediate layer photosensitive resin.

In the present example, the intermediate layer photosensitive resin forming the second photosensitive resin layer 2062 has an exposure sensitivity (C/cm²) lower than that of the partition photosensitive resin forming the first photosensitive resin layer 2061. The second photosensitive resin layer 2062 is applied with a desired thickness onto the first photosensitive resin layer 2061 using spin coating, similarly to the first photosensitive resin layer 2061. For example, a non-chemically amplified resist may be used as the partition resin for forming the first photosensitive resin layer 2061, and a chemically amplified resist may be used as the intermediate layer resin for forming the second photosensitive resin layer 2062.

In the present example, at the exposure step S2005, a channel pattern is drawn, via a photomask, on the first and second photosensitive resin layers 2061 and 2062 applied to the substrate 2060. For example, the present example uses a proximity exposure device using light with a wavelength of 350 nm or more and 400 nm or less in the UV light region as a light source. An optimal exposure dose is determined herein for the first photosensitive resin layer 2061. As mentioned above, in the present example, the exposure sensitivity of the intermediate layer resin forming the second photosensitive resin layer 2062 is higher than that of the partition resin forming the first photosensitive resin layer 2061. Therefore, in the second photosensitive resin layer 2062, the exposure dose matching the first photosensitive resin layer 2061 is necessary and sufficient.

Next, at the development step S2006, a channel pattern 2063 is formed by developing the exposed first and second photosensitive resin layers 2061 and 2062. For example, a sodium carbonate solution may be sprayed as a developer. Next, at step S2007, the developed first and second photosensitive resin layers 2061 and 2062 are cleaned to completely remove the developer. For example, ultra-pure water may be sprayed for cleaning. Thus, as shown in Fig. 21(b), a partition layer 2061a is formed defining the channel pattern 2063. In the present example, the exposure sensitivity of the second photosensitive resin layer 2062 is higher than that of the first photosensitive resin layer 2061. Therefore, as shown in Fig. 21(b), after exposure, the region in the second photosensitive resin layer 2062 remaining as a pattern in the exposed portion becomes larger than the first photosensitive resin layer 2061. Consequently, as shown in Fig. 21(b), the opening width in the exposed region in the second photosensitive resin layer 2062 becomes smaller than the opening width of the partition layer 2061a formed of the first photosensitive resin layer 2061.

Next, the microfluidic chip, in which the channel pattern 2063 has been formed, undergoes heat treatment (post-baking) at step S2008. In the present example, heat treatment is performed using a hot plate at a temperature near the glass transition temperatures (Tg) of the first and second photosensitive resin layers 2061 and 2062. In the present example, heat treatment promotes fluidization (reflow) of the second photosensitive resin layer 2062, i.e., the intermediate layer resin, and portions of the intermediate layer resin on right and left parts of the partition layer 2061a flow toward the center of the channel pattern 2063. The portions of the intermediate layer resin that have flowed from parts of the partition layer 2061a are joined together on the side opposite from the substrate 2060, i.e., on the upper side of the channel pattern 2063, to form an intermediate layer 2062a. Thus, as shown in Fig. 21(c), the upper part of the channel pattern 2063 is covered with the intermediate layer 2062a to form a channel section 2063a, thereby producing the microfluidic chip 2600.

Similarly to the microfluidic chip 2500, in the microfluidic chip 2600 of the present example, the glass transition temperature (Tg) of the first photosensitive resin layer 2061, i.e., the partition resin, forming the partition layer 2061a is equal to that of the second photosensitive resin layer 2062, i.e., the intermediate layer resin, forming the intermediate layer 2062a. Therefore, when the intermediate layer is fluidized (reflows), the partition resin is also fluidized. In the present example, the first and second photosensitive resin layers 2061 and 2062 are each formed of a negative resist, and the exposure sensitivity of the second photosensitive resin layer 2062 is higher than that of the first photosensitive resin layer 2061. Therefore, as shown in Fig. 21(b), the opening width of the second photosensitive resin layer 2062 is formed to be smaller than the opening width of the partition layer 2061a formed of the first photosensitive resin layer 2061, and the opening width of the partition layer 2061a formed of the first photosensitive resin layer 2061 is formed to be sufficiently larger than the opening width of the second photosensitive resin layer 2062.

Accordingly, the width of the channel section 2063a is reduced by more than the opening width of the channel pattern 2063 is, due to occurrence of fluidization in the first photosensitive resin layer 2061 with a degree that is approximately the same as in the second photosensitive resin layer 2062; however, as shown in Fig. 21(c), the width of the channel section 2063a is sufficiently ensured.

Accordingly, if the first and second photosensitive resin layers 2061 and 2062 are each formed of a negative resist and if the condition of [Exposure sensitivity of intermediate layer resin > Exposure sensitivity of partition resin] is satisfied, the intermediate layer resin and the partition resin can be fluidized to easily form the intermediate layer 2062a, while sufficiently retaining the width of the channel section.

The description so far has been given of an example in which the partition layer and the intermediate layer are formed as separate bodies (multilayer configuration) in the microfluidic chip of the present embodiment. As described above, according to the production method of the present embodiment, the intermediate layer 2012, which serves as a region for bonding the partition section (partition layer 2011) to the lid component (cover layer 2014) covering the channel section of the microfluidic chip, can be formed using existing photolithography techniques without using an adhesive layer such as an adhesive. Thus, the area of the bonding region can be increased, and the adhesive strength between the partition layer 2011 and the cover layer 2014 can be improved. Furthermore, adhesive components are prevented from eluting into the channel and inhibiting reactions of the solution in the channel.

Also, omission of an adhesive can avoid quality deterioration due to poor bonding which would have been caused by uneven film thickness of the adhesive. In addition, the production method can be simplified compared to the case where a cover layer, as a lid component, is bonded to a partition layer without providing an intermediate layer therebetween, or the case where a partition layer and an intermediate layer are bonded together using an adhesive layer such as an adhesive.

The present disclosure is not limited to this, but the partition layer and the intermediate layer may be configured using three or more layers. In this case also, the second photosensitive resin layer forming the intermediate layer may have a multilayer configuration. Photosensitive resins having thermal fluidity have characteristics such that
the higher the layer, the greater the fluidity of the resin upon heat treatment. Therefore, in the case of forming an intermediate layer using a second photosensitive layer with a multilayer configuration, portions of upper layers are joined together faster than the lower layers. Accordingly, in the intermediate layer formed of a second photosensitive resin layer with a multilayer configuration, the opening width becomes narrower the higher the layer, and parts of the uppermost layer or parts of the layers close to the uppermost layer are joined together.

### (3.1.4) Effects of third embodiment

The microfluidic chip 2001 described above has the following effects.

In the present embodiment, the microfluidic chips 2400, 2500 and 2600 have configurations similar to the configuration of the microfluidic chip 2001. Also, the substrates 2040, 2050 and 2060 of these microfluidic chips are similar to the substrate 2010, the partition layers 2041a, 2051a and 2061a are similar to the partition layer 2011, the intermediate layers 2042a, 2052a and 2062a are similar to the intermediate layer 2012, and the channel sections 2043a, 2053a and 2063a are similar to the channel section 2003.

(1) The microfluidic chip 2001 according to the present embodiment includes a substrate 2010, a partition layer 2011 forming a channel section 2003 on the substrate 2010, a cover layer 2014 located on one side of the partition layer 2011 facing away from the substrate 2010 to serve as a lid for the channel section 2003, and an intermediate layer 2012 located between the cover layer 2014 and the channel section 2003.

Thus, the area of the region (bonding region) for bonding the partition section to the lid component can be increased, and the adhesive strength between the partition section and the lid component can be improved.

(2) In the microfluidic chip 2001 according to the present embodiment, the intermediate layer 2012 may be part of the partition layer 2011, and the cover layer 2014 may be bonded to the partition layer 2011 via the intermediate layer 2012.

Thus, the adhesive strength between the partition section and the lid component can be further improved.

(3) In the microfluidic chip 2001 according to the present embodiment, the intermediate layer 2012 may overlap part of the channel section 2003 in plan view.

Thus, the area of the bonding region can be reliably increased compared to the conventional art.

(4) In the microfluidic chip 2001 according to the present embodiment, the intermediate layer 2012 may overlap the entirety of the channel section 2003 in plan view.

Thus, the area of the bonding region can be reliably increased compared to the conventional art. Furthermore, the lid component can be easily bonded without requiring devices or techniques of particularly high level.

(5) In the microfluidic chip 2001 according to the present embodiment, if the intermediate layer 2012 overlaps the entirety of the channel section 2003 in plan view, the intermediate layer 2012 and the cover layer 2014 may be bonded together via the adhesive layer 2013.

Thus, the lid component can be bonded easily. Furthermore, if the intermediate layer 2012 overlaps the entirety of the channel section 2003 in plan view, the adhesive components forming the adhesive layer 2013 are prevented from eluting into the channel section 2003 and inhibiting reactions of the solution in the channel.

(6) In the microfluidic chip 2001 according to the present embodiment, no adhesive layer 2013 may be provided between the partition layer 2011 and the intermediate layer 2012.

Thus, forming the intermediate layer 2012 without using an adhesive, adhesive components are prevented from eluting into the channel section 2003 and inhibiting reactions of the solution in the channel.

(7) In the microfluidic chip 2001 according to the present embodiment, the intermediate layer 2012 and the partition layer 2011 may be joined together by welding.

Thus, forming the intermediate layer 2012 without using an adhesive, adhesive components are prevented from eluting into the channel section 2003 and inhibiting reactions of the solution in the channel.

(8) In the microfluidic chip 2001 according to the present embodiment, the material of the intermediate layer 2012 may be a resin having thermal fluidity, and the channel section 2003 may have a cross section with rounded corners.

Thus, the intermediate layer 2012 and the partition layer 2011 can be joined together using existing photolithography techniques. Furthermore, the transfer speed or flow rate of the fluid (e.g., reaction solution) in the channel section 2003 can be stabilized and the substances to be tested can be suppressed from being retained at the corners.

(9) In the microfluidic chip 2001 according to the present embodiment, the intermediate layer 2012 and the partition layer 2011 may be separate bodies.

Thus, a resin having favorable characteristics as an intermediate component can be adequately selected.

(10) In the microfluidic chip 2001 according to the present embodiment, the partition layer 2011 and the intermediate layer 2012 may be formed of resin materials, and the resin materials may be photosensitive resins having photosensitivity to light with a wavelength of 190 nm or more and 400 nm or less in the UV light region.

Thus, the intermediate layer 2012 of the microfluidic chip can be formed using reflow of the photosensitive resins without using an adhesive, so that adhesive components are prevented from eluting into the channel and inhibiting reactions of the solution in the channel.

(11) In the microfluidic chip 2001 according to the present embodiment, the intermediate layer 2012 may be a separate body from the partition layer 2011 and may have a glass transition temperature lower than that of the partition layer 2011.

Thus, when forming the intermediate layer 2012, the partition layer 2011 can be suppressed from being fluidized and changing the channel pattern.

(12) In the microfluidic chip 2001 according to the present embodiment, the intermediate layer 2012 may be a separate body from the partition layer 2011 and may be configured to have an exposure sensitivity higher or lower than that of the partition layer 2011.

Thus, when fluidizing the resin material of the intermediate layer during formation of the intermediate layer 2012, if fluidization has also occurred in the partition layer resin, the opening width of the photosensitive resin forming the partition layer 2011 can be made sufficiently large and a sufficient space can be retained as the channel section 2003.

(13) The method of producing the microfluidic chip 2001 according to the present embodiment includes steps of applying a photosensitive resin for a partition layer 2011 onto a substrate 2010, applying a photosensitive resin for an intermediate layer 2012 onto the applied photosensitive resin for a partition layer 2011, exposing the photosensitive resin for a partition layer 2011 and the photosensitive resin for an intermediate layer 2012, developing and cleaning the exposed photosensitive resin for a partition layer 2011 and the exposed photosensitive resin for an intermediate layer 2012 to form a partition layer 2011 defining a channel section 2003 on the substrate 2010, heat-treating and fluidizing the photosensitive resin for an intermediate layer 2012 on the partition layer 2011 to form an intermediate layer 2012 overlapping at least part of the channel section 2003 in plan view, and bonding a cover layer 2014 on one side of the partition layer 2011 facing away from the substrate 2010 via the intermediate layer 2012.

Thus, there can be provided a microfluidic chip that can increase the area of the bonding region and can improve the bonding strength between the partition section and the lid component.

### (3.2) Embodiment 3-2

Referring to Fig. 22, a microfluidic chip according to Embodiment 3-2 of the present disclosure will be described. Fig. 22 is a cross-sectional view illustrating a configuration example of a microfluidic chip 2200 according to Embodiment 3-2 of the present disclosure.

The microfluidic chip 2200 includes a substrate 2020, a partition layer 2021 forming a channel section 2023 on the substrate 2020, a cover layer (one example of the cover section) 2024 located on one side of the partition layer 2021 facing away from the substrate 2020 to serve as a lid for the channel section 2023, and an intermediate layer 2022 formed of part of the partition layer 2021 and located between the cover layer 2024 and the channel section 2023. Also, the microfluidic chip 2200 is provided with an adhesive layer 2130 for bonding the cover layer 2024 to the partition layer 2021 and the intermediate layer 2022. In other words, in the microfluidic chip 2200, the partition layer 2021 is formed integrally with the intermediate layer 2022. This is a point different from the microfluidic chips 2001, 2400, 2500 and 2600 of Embodiment 3-1.

### (3.2.1) Configuration of microfluidic chip 2200

The partition layer 2021 and the intermediate layer 2022 of the microfluidic chip 2200 will be described focusing on differences from the partition layer 2011 and the intermediate layer 2012 of Embodiment 3-1. Since the configurations of the components (substrate 2020, partition layer 2130, cover layer 2024, and channel section 2023) other than the intermediate layer 2022 are similar to those of the substrate 2010, adhesive layer 2013, cover layer 2014, and channel section 2003 of the microfluidic chip 2001, explanation will be omitted.

The materials of the partition layer 2021 and the intermediate layer 2022 of the microfluidic chip 2200 may be similar to that of the partition layer 2011 of the microfluidic chip 2001.

Fig. 22 shows a configuration example of the microfluidic chip 2200 in which the partition layer 2021 is formed integrally with the intermediate layer 2022. In the microfluidic chip 2200, since the partition layer 2021 serves as the intermediate layer 2022, the production processes can be simplified.

### (3.2.2) Method of producing microfluidic chip 2200

An example of a method of producing the microfluidic chip 2200 will be described.

The method of producing the microfluidic chip 2200 can omit step S2003 (step of applying the second photosensitive resin) from the method of producing the microfluidic chip 2001 shown in Fig. 18. Thus, the production processes can be simplified.

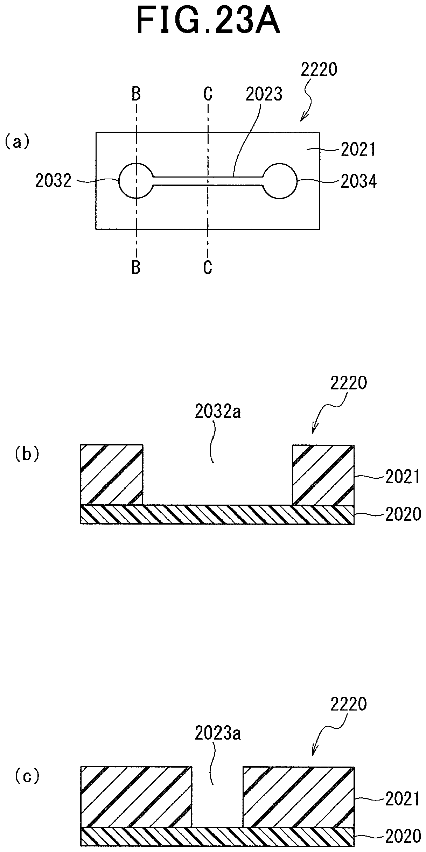

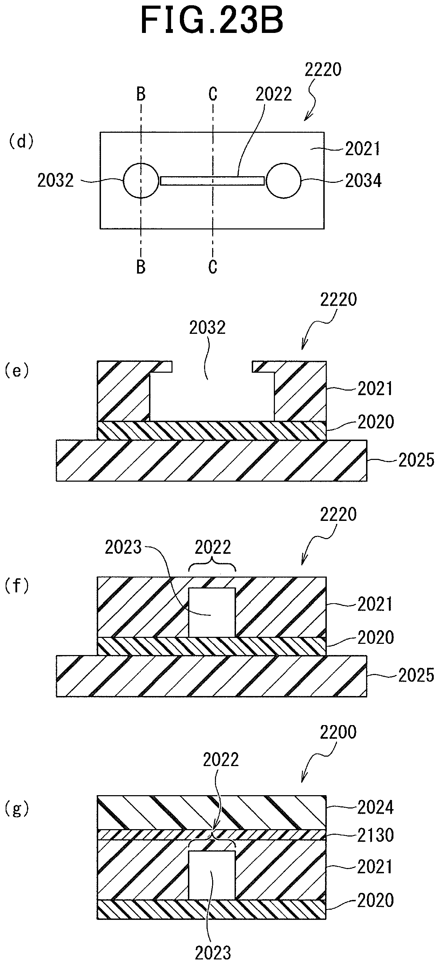

Referring to Figs. 23A and 23B, the method of producing the microfluidic chip 2200 will be described in more detail.

Figs. 23A and 23B are schematic diagrams illustrating processes in the method of producing the microfluidic chip 2200 according to the present embodiment. In the method of producing the microfluidic chip 2200, a channel pattern is formed as in the steps S2001, S2002 and S2005 to S2010 of the method of producing the microfluidic chip 2001 shown in Fig. 18, except that only one layer of a photosensitive resin is formed on the substrate 2020. Therefore, explanation for the steps S2001, S2002 and S2005 to S2010 will be omitted.

Fig. 23A(a) is a schematic plan view illustrating a channel pattern 2220 when producing the microfluidic chip 2200 according to the present embodiment. The partition layer 2021 is provided with an inlet 2032 for introducing a liquid, a channel section 2023 through which the liquid flows, and an outlet 2034 for discharging the liquid. Explanation for the inlet 2032 and the outlet 2034 will be omitted because they have configurations similar to those of the inlet 2002 and the outlet 2004 of the microfluidic chip 2001 of Embodiment 3-1 described above.

Fig. 23A(b) is a cross-sectional view taken along the line B-B of Fig. 23A(a). The partition layer 2021 is formed on the substrate 2020 as a base. The region surrounded by the substrate 2020 and the partition layer 2021 is a region 2032a in the channel pattern 2220 serving as the inlet 2032 for introducing a fluid (e.g., reaction solution). In order to introduce a fluid, no intermediate layer 2022, adhesive layer 2130, or cover layer 2024 is formed on the upper part of the inlet 2032.

Fig. 23A(c) is a cross-sectional view taken along the line C-C of Fig. 23A(a). The partition layer 2021 is formed on the substrate 2020 as a base component, and the region surrounded by the substrate 2020 and the partition layer 2021 is a region 2023a in the channel pattern 2220 defining the channel section 2023 through which a fluid flows. The region 2023a has an opening width which is formed to be smaller than that of the region 2032a serving as the inlet 2032.

The channel pattern 2220 formed undergoes heat treatment (post-baking). Heat treatment is performed, for example, using a hot plate, oven, etc. Post-baking is performed for the purpose of fluidizing (reflowing) the partition resin (partition layer 2021) by heating the resin up to the glass transition temperature (Tg) thereof. This is a point different from the heat treatment in the method of producing the microfluidic chip 2001 of Embodiment 3-1. Fluidization (reflow) of a photosensitive resin is characterized by the tendency that the photosensitive resin is fluidized more on the side opposite from the substrate 2020, i.e., on the upper part of the channel pattern 2220, depending on the characteristics of the resin. Due to fluidization of the upper part of the channel pattern 2220, parts of the partition layer 2021 are joined together to function as an intermediate component of the channel pattern 2220, i.e., as the intermediate layer 2022 covering the region 2023a of the channel pattern 2220. Thus, an intermediate layer 2022 integrated with the partition layer 2021 is formed.

Fig. 23B(d) is a schematic plan view illustrating the channel pattern 2220 after heat treatment (post-baking). Due to heat treatment, the partition layer 2021 is fluidized to form an intermediate layer 2022, as a result of which a channel section 2023 is defined as shown in Fig. 23A(f) described later. Although not shown in Fig. 23A(d), the channel section 2023 can be seen through a transparent intermediate layer 2022. The inlet 2032 and the outlet 2034 are reduced in size due to fluidization of the partition layer 2021; however, since they are designed in advance to have a larger size to compensate for size reduction, they are not closed by the intermediate layer 2022 and remain as through-holes. This is similar to the production of the microfluidic chip 2001 of Embodiment 3-1.

Fig. 23B(e) is a schematic cross-sectional view illustrating the channel pattern 2220 taken along the line B-B of Fig. 23B(d). Due to post-baking on a hot plate 2025, the resin on the upper part of the partition layer 2021 flows toward the center of the inlet 2032 in the width direction; however, the inlet 2032 is not closed and the opening end remains in communication with the outside. In other words, the function of the inlet 2032 for introducing a fluid is maintained.

Fig. 23B(f) is a schematic cross-sectional view illustrating the channel pattern 2220 taken along the line C-C of Fig. 23B(d). Due to post-baking on the hot plate 2025, portions of the resin on one side of the partition layer 2021 facing away from the substrate 2020 (on the upper side of the partition layer 2021) flow from right and left and are joined together near the center of the channel section 2023 in the width direction to form the intermediate layer 2022. In other words, the intermediate layer 2022 overlaps the channel section 2023 in plan view. In this way, according to the present embodiment, the intermediate layer 2022, as a simple lid component, covering the channel section of the microfluidic chip can be formed using existing photolithography techniques without using an adhesive. Thus, adhesive components are prevented from eluting into the channel and inhibiting reactions of the solution in the channel.

Also, omission of an adhesive can avoid quality deterioration due to poor bonding which would have been caused by uneven film thickness of the adhesive. Furthermore, the production method can be simplified compared to the case where a partition layer and an intermediate layer are bonded together using an adhesive layer such as an adhesive. In addition, poor bonding can be more reliably suppressed by integrating the partition layer 2021 with the intermediate layer 2022. The upper part of the partition layer 2021 and the intermediate layer 2022 may serve as an intermediate layer as a region for bonding between the partition layer 2021 and the cover layer 2024.

Fig. 23B(g) is a cross-sectional view illustrating the microfluidic chip 2200 in which the partition layer 2021 and the intermediate layer 2022 are bonded to the cover layer 2024. An adhesive layer 2130 is applied to the surfaces of the partition layer 2021 and the intermediate layer 2022 facing away from the channel section 2023, and a cover layer 2024 is bonded to the intermediate layer as a bonding region (partition layer 2021 and the intermediate layer 2022) via the adhesive layer 2130. Formation of the cover layer 2014 on the intermediate layer 2022, as a simple upper lid, can further improve the physical strength of the microfluidic chip 2200.

In this way, the microfluidic chip 2200 according to the present embodiment includes a substrate 2020, a partition layer 2021 forming a channel section 2023 on the substrate 2020, a cover layer 2024 located on one side of the partition layer 2021 facing away from the substrate 2020 to serve as a lid for the channel section 2023, and an intermediate layer 2022 located between the cover layer 2024 and the channel section 2023. Thus, the area of the bonding region can be increased to improve the adhesive strength between the partition layer 2021, as the partition section, and the cover layer 2024, as a lid component.

As described above, the method of producing the microfluidic chip 2200 according to the present embodiment includes steps of applying a partition resin onto a substrate 2020 (step S2001), exposing the partition resin (step S2005), developing and cleaning the exposed partition resin to form a partition layer 2021 defining a channel section 2023 on the substrate 2020 (steps S2006 and S2007), heat-treating the partition layer 2021 and fluidizing the partition resin to form an intermediate layer 2022 overlapping at least part of the channel in plan view (step S2008), and bonding a cover layer 2024 on one side of the partition layer 2021 facing away from the substrate 2020 via the intermediate layer 2022 (step S2010).

Thus, the intermediate layer 2022 can be joined to and integrated with the partition layer 2021 as a part thereof by welding, without using an adhesive, and therefore, adhesive components are prevented from eluting into the channel section 2023 and inhibiting reactions of the solution in the channel.

### (3.3) Embodiment 3-3

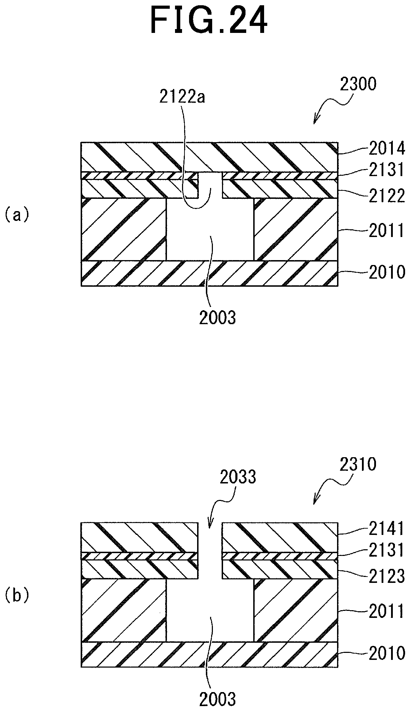

Referring to Fig. 24, a microfluidic chip according to Embodiment 3-3 of the present disclosure will be described. Fig. 24(a) is a cross-sectional view illustrating a configuration example of a microfluidic chip according to Embodiment 3-3 of the present disclosure.

A microfluidic chip 2300 includes a substrate 2010, a partition layer 2011 forming a channel section 2003 on the substrate 2010, a cover layer (one example of the cover section) 2014 located on one side of the partition layer 2011 facing away from the substrate 2010 to serve as a lid for the channel section 2003, and an intermediate layer 2122 located between the cover layer 2014 and the channel section 2003 and overlapping part of the channel section 2003 in plan view. Also, the microfluidic chip 2300 is provided with an adhesive layer 2131 for bonding the cover layer 2014 to the partition layer 2011 and the intermediate layer 2022. Although not shown in Fig. 24(a), the microfluidic chip 2300 includes an inlet and an outlet (with configurations similar to those of the inlet 2002 and the outlet 2004 shown in Fig. 15(a)).

The intermediate layer as a bonding region (intermediate layer 2122) for bonding the partition section to the lid component in the present disclosure is not limited to have a configuration of overlapping the entirety of the channel section (e.g., channel section 2003) in plan view, but may have a configuration of overlapping part of the channel section in plan view. With this configuration also, the area of the bonding region is increased compared to the case where only the upper partition section is used as a bonding region as in the conventional art, and therefore, the adhesive strength can be improved between the partition section and the lid component. In other words, in post-baking treatment (step S2008), the intermediate layer 2122 may be formed overlapping at least part of the channel section 2003 in plan view.

In the present example, the intermediate layer 2122 is provided with a gap 2122a in a region thereof overlapping the center region of the channel section 2003 in plan view. In other words, in the microfluidic chip 2300, the intermediate layer 2122 overlaps part of the channel section 2003, not the entirety thereof, in plan view. This is a point different from the microfluidic chips 2001, 2400, 2500 and 2600 of Embodiment 3-1.

Specifically, in the post-baking treatment, when the intermediate layer resin is promoted in fluidization (reflow) and portions thereof on right and left parts of the partition layer 2011 flow toward the center of the channel section 2003, heating may be stopped before these portions of the intermediate layer resin flowing from parts of the partition layer 2011 are joined together. Thus, time used for the post-baking treatment can be reduced when producing the microfluidic chip 2300. Also, in the microfluidic chip 2300, the adhesive layer 2131 is formed by applying an adhesive onto the intermediate layer 2122 except for the region of the gap 2122a. Thus, adhesive components are prevented from eluting into the channel and inhibiting reactions of the solution in the channel.

The gap 2122a may also be provided to the intermediate layer of a microfluidic chip (e.g., the microfluidic chip 2200 shown in Fig. 22) in which the partition layer is formed integrally with the intermediate layer. In other words, in the post-baking treatment (step S2008), the intermediate layer 2022 may be formed overlapping at least part of the channel section 2003 in plan view. In this case, in the post-baking treatment, heating may be stopped before portions of the resin on opposing parts of the partition layer 2021 are fluidized and joined together. Also, in this case, the adhesive layer 2130 may be formed by applying an adhesive onto the intermediate layer 2022 except for the gap portion.

Fig. 24(b) is a cross-sectional view illustrating another configuration example of a microfluidic chip according to the present embodiment.

The microfluidic chip 2310 includes a substrate 2010, a partition layer 2011 forming a channel section 2003 on the substrate 2010, a cover layer (one example of the cover section) 2141 located on one side of the partition layer 2011 facing away from the substrate 2010 to serve as a lid for the channel section 2003, and an intermediate layer 2123 located between the cover layer 2141 and the channel section 2003 and overlapping part of the channel section 2003 in plan view. Also, the microfluidic chip 2310 is provided with an adhesive layer 2131 for bonding a cover layer 2141 to the partition layer 2011 and the intermediate layer 2123. Although not shown in Fig. 24(b), the microfluidic chip 2310 includes an inlet and an outlet (with configurations similar to those of the inlet 2002 and the outlet 2004 shown in Fig. 15(a)).

The cover layer 2141 and the intermediate layer 2123 of the microfluidic chip 2310 have a region overlapping the channel section 2003 in plan view where a through-hole 2033 is provided. The through-hole 2033 passes through the cover layer 2141, the adhesive layer 2131, and the intermediate layer 2123 in the thickness direction to serve as an air escape route. Therefore, in the microfluidic chip 2310 having the through-hole 2033, air entrapment can be suppressed when, for example, bonding the intermediate layer 2123 and the cover layer 2141 together and thus bonding can be facilitated.

The through-hole 2033 serving as an air entrapment prevention mechanism may be provided to any of the cover layer 2141, adhesive layer 2131, and intermediate layer 2123, as long as it is provided at a position overlapping the channel section 2003 in plan view (region facing the channel section 2003), i.e., as long as it is provided along the fluid transfer direction in the channel section 2003. The through-hole 2033 may be adequately arranged at a portion where a highest air entrapment prevention effect can be exerted. A single through-hole 2033 or multiple through-holes 2033 may be provided. If multiple through-holes 2033 are provided, the distance from one through-hole 2033 to the subsequent through-hole 2033, i.e., the arrangement interval (pitch) between the multiple through-holes 2033, is preferred to be in the range of 5 µm or more and 1,000 µm or less. With the arrangement interval of the above range, air entrapment can be more efficiently avoided when bonding the intermediate layer 2123 to the cover layer 2141.

As shown in Fig. 24(b), in the present embodiment, the width of the through-hole 2033 passing through the intermediate layer 2123, adhesive layer 2131, and cover layer 2141 in the thickness direction is constant. The present disclosure is not limited to this. For example, the diameter of the opening end of the through-hole 2033 on the channel section 2003 side may be different from the diameter of the opening end on the side opposite from the channel section 2003 (cover layer 2141 side) and the width may change in the thickness direction accordingly.

Alternatively, the opening end of the through-hole 2033 on the channel section 2003 side may have a diameter equal to that of the opening end on the cover layer 2141 side, and the width may vary in the middle portion (portion between the opening end on the channel section 2003 side and the opening end on the cover layer 2141 side) in the thickness direction. In this case, for example, in the cross section shown in Fig. 24(b), the through-hole 2033 may have a shape tapered toward the middle portion, or a shape thickened toward the middle portion, as viewed in cross section.

The shape of the through-hole 2033 (shape of the opening end) in plan view may be a circular shape. The circular shape may include a perfect circle, ellipse, oval, and oblong circle. If the opening end of the through-hole 2033 is a circle or ellipse, the diameter (maximum diameter) is preferred to be in the range of 1 µm or more and 100 µm or less. Thus, air entrapment when bonding the intermediate layer 2123 to the cover layer 2141 can be more efficiently suppressed.

The shape of the through-hole 2033 (shape of the opening end) in plan view may be a polygonal shape. In this case, the polygonal shape may have rounded corners, with the sides connected by arcs. If the opening end of the through-hole 2033 has a polygonal shape, the through-hole 2033 is preferred to have a width in the range of 1 µm or more and 100 µm or less in the longitudinal direction of the channel section 2003. Thus, air entrapment when bonding the intermediate layer 2123 to the cover layer 2141 can be efficiently suppressed. It should be noted that the size of the through-hole 2033 may be determined considering the entire size of the microfluidic chip or the width, length, etc. of the channel.

The through-hole 2033 in the cover layer 2141 may be formed, for example, when forming the inlet and the outlet, or may be formed, for example, separately from the inlet and the outlet using existing photolithography techniques.

The through-hole 2033 in the intermediate layer 2123 may be formed simultaneously with the intermediate layer 2122 by controlling the photomask used when forming the channel pattern. In other words, the through-hole 2033 serving as an air entrapment prevention mechanism can be formed in the intermediate layer 2122 using ordinary photolithography techniques. In this case, similarly to the inlet and the outlet, the intermediate layer 2022 may be formed outside the region corresponding to the through-hole 2033 in the photolithography processing.

As in the microfluidic chip 2310, the through-hole 2033 may be provided to the cover layer (cover layer 2024), adhesive layer 2130, and the intermediate layer (intermediate layer 2022) of a microfluidic chip (e.g., the microfluidic chip 2200 shown in Fig. 22) in which the partition layer is formed integrally with the intermediate layer.

### <Third Example>

The following description relates to specific examples of the microfluidic chip and the method of producing the same, according to the third embodiment of the present disclosure. The third embodiment of the present disclosure should not be limited to the following examples.

### (Example 3-1)

A microfluidic chip and a method of producing the same according to Embodiment 3-1 will be described.

First, a partition layer photosensitive resin was applied onto a glass substrate to form a first photosensitive resin layer. The partition layer photosensitive resin used herein was a negative transparent liquid resin (negative liquid resist) containing an epoxy resin. The glass transition temperature (Tg) of the photosensitive resin (negative liquid resist) was 160°C. The negative liquid resist was applied onto the glass substrate using a spin coater set to 1,100 rpm and 30 sec. The rotation speed and time of the spin coater were controlled so that the thickness of the first photosensitive resin layer would be 50 µm.

Next, the partition layer photosensitive resin (negative liquid resist) underwent heat treatment (pre-baking) on a hot plate, for the purpose of removing residual solvent contained therein. Pre-baking was performed at 90°C for 20 minutes.

Next, an intermediate layer photosensitive resin was applied onto the first photosensitive resin layer to form a second photosensitive resin layer. The intermediate layer photosensitive resin was similar to the negative liquid resist mentioned above, except for having a glass transition temperature (Tg) (110°C) which was lower than that of the partition layer photosensitive resin by 50°C. Pre-baking was performed under conditions similar to the partition layer photosensitive resin to remove the residual solvent contained in the intermediate layer photosensitive resin.

Next, the photosensitive resin layers (first and second photosensitive resin layers) on the glass substrate were exposed to draw a channel pattern. Specifically, the photosensitive resin was subjected to pattern exposure via a photomask having a microchannel pattern array. The photomask was formed of a light-shielding film with a double-layer structure of chromium and chromium oxide. A proximity exposure device was used for the exposure. The exposure device had a high-pressure mercury lamp as a light source, with a cut filter that was an i-line filter inserted for exposure. The exposure dose was 170 mJ/cm².

Next, the exposed photosensitive resin layers were developed to form a channel pattern. Specifically, the photosensitive resin layers were developed for 60 seconds using an alkaline developer (TMAH 2.38%) to dissolve the unexposed portions and to pattern a channel structure.

Subsequently, shower cleaning was performed using ultra-pure water to remove the developer from the photosensitive resin layers on the substrate, followed by drying using a spin dryer. At this stage, the upper side of the channel section of the channel pattern opposite from the glass substrate was open. Also, the second photosensitive resin layer remained on the partition layer.

Next, the channel pattern was subjected to heat treatment (post-baking) in an oven at 160°C for 30 minutes. In this case, due to reflow of the photosensitive resin, the upper parts of the channel pattern, i.e., parts of the second photosensitive resin layer, flowed toward the center of the channel section, and opposing parts of the second photosensitive resin layer on the partition layer were joined together. Thus, an intermediate layer was formed and a microfluidic chip of the present example was obtained. At both ends of the channel section, an inlet and an outlet, as through-holes, were formed without being closed by the intermediate layer.

Next, an adhesive was applied onto the intermediate layer to form an adhesive layer. An acrylic resin adhesive was used as the adhesive.
Next, a cover layer was bonded to the adhesive layer to prepare a microfluidic chip. For the cover layer, PMMA provided with holes for inlet and outlet of the channel in advance was used.

The microfluidic chip according to the present example prepared as described above includes an intermediate layer between a partition layer and a cover layer. Therefore, the area of the bonding region for bonding the partition layer to the cover layer, as a lid component, can be increased, and the adhesive strength between the partition section and the lid component can be improved. Due to provision of the intermediate layer, the adhesive components forming the adhesive layer are prevented from eluting into the channel and inhibiting reactions of the solution in the channel. Furthermore, the microfluidic chip of the present example can improve the physical strength by providing a cover layer on the intermediate layer. In the microfluidic chip of the present example, the partition layer and the intermediate layer are joined together by welding without using an adhesive, and the intermediate layer is formed by fluidizing the intermediate layer resin (second photosensitive resin layer) during post-baking. Thus, adhesive components are prevented from eluting into the channel and inhibiting reactions of the solution in the channel.

10 µL of a colored reaction solution was collected with a pipette and introduced into the microfluidic chip of the present example from the inlet of the channel, and the state of liquid transfer was microscopically observed. The introduced reaction solution smoothly passed through the channel without leakage, and liquid transfer was good. In this way, the fluid passed smoothly through the microfluidic chip of the present example without leakage, and thus it was confirmed that the microfluidic chip was imparted with basic characteristics of a microfluidic chip.

### (Example 3-2)

A microfluidic chip and a method of producing the same according to Embodiment 3-2 will be described.

A microfluidic chip according to Example 3-2 was prepared as in Example 3-1, except that the partition layer was formed integrally with the upper lid layer.

Specifically, first, as in Example 3-1, a partition layer photosensitive resin was applied onto a glass substrate to form a 3-1 photosensitive resin layer, followed by pre-baking.

Next, the first photosensitive resin layer on the glass substrate was exposed under conditions similar to Example 3-1 to draw a channel pattern.

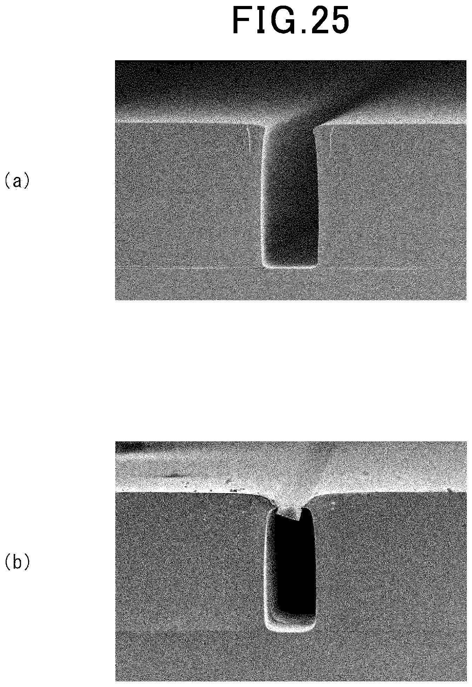

Next, the exposed first photosensitive resin layer was developed under conditions similar to Example 3-1, followed by removing the developer and drying using a spin dryer, thereby forming a channel pattern. Fig. 25(a) shows an example of a cross-sectional SEM image of the channel pattern at this point.

In Fig. 25(a), the recess sandwiched between opposing parts of the partition layer is the channel section of the channel pattern. As shown in Fig. 25(a), in the channel section of the channel pattern after removal of the developer and drying, the upper side thereof opposite from the glass substrate is open. The opening on the upper side of the channel section is similar to Example 3-1.

Next, the channel pattern underwent post-baking under conditions as in Example 3-1 (baked in an oven at 160°C for 30minutes). In this case, due to reflow of the photosensitive resin, the upper parts of the channel pattern, i.e., the upper parts of the partition layer, flowed toward the center of the channel section, and portions of the photosensitive resin (negative liquid resist) on opposing parts of the partition layer were joined together.

Fig. 25(b) shows an example of a cross-sectional SEM image of the microfluidic chip after post-baking. As shown in Fig. 25(b), it was confirmed that the upper parts of the channel pattern after post-baking, i.e., portions of the photosensitive resin on parts of the partition layer, were fluidized and joined together near the center of the channel section to form an intermediate layer.

As shown in Fig. 25(b), it was confirmed that the intermediate layer formed due to fluidization of the partition layer had a reduced thickness toward the center of the channel section to form a recess. As shown in Fig. 25(b), it was confirmed that the cross section of the channel section had rounded corners. These points are similar to Example 3-1 in which the partition layer and the intermediate layer are separate bodies.

At both ends of the channel section, an inlet and an outlet, as through-holes, were formed without being closed by the intermediate layer.

Next, as in Example 3-1, an adhesive layer was formed and a cover layer was bonded onto the adhesive layer. Thus, a microfluidic chip of the present example was obtained.

The microfluidic chip according to the present example prepared as described above is provided with an intermediate component including a partition layer and an intermediate layer. Therefore, the area of the bonding region for bonding the partition layer to the cover layer, as a lid component, can be increased, and the adhesive strength between the partition section and the lid component can be improved. Due to provision of the intermediate component, the adhesive components forming the adhesive layer are prevented from eluting into the channel and inhibiting reactions of the solution in the channel. Furthermore, the microfluidic chip of the present example can improve the physical strength by providing a cover layer on the intermediate layer. In the microfluidic chip of the present example, an intermediate layer is formed due to the upper parts of the partition layer being fluidized by post-baking, and there is no adhesive used between the partition layer and the intermediate layer. Thus, adhesive components are prevented from eluting into the channel and inhibiting reactions of the solution in the channel.

Liquid transfer testing was performed for the microfluidic chip of the present example as in Example 3-1. The reaction solution introduced in the testing smoothly passed through the channel without leakage, and liquid transfer was good. In this way, the fluid passed smoothly through the microfluidic chip of the present example without leakage, and thus it was confirmed that the microfluidic chip was imparted with basic characteristics of a microfluidic chip.

For example, the third embodiment can have the following configurations.
(1) A microfluidic chip including
   a base section;
   a partition section forming a channel on the base section;
   a cover section located on one side of the partition section facing away from the base section to serve as a lid for the channel and; and
   an intermediate component located between the cover section and the channel.
(2) The microfluidic chip according to (1), wherein
   the intermediate component is part of the partition section; and
   the cover section is bonded to the partition section via the intermediate component.
(3) The microfluidic chip according to (1) or (2), wherein
   the intermediate component overlaps part of the channel in plan view.
(4) The microfluidic chip according to (1) or (2), wherein
   the intermediate component overlaps the entirety of the channel in plan view.
(5) The microfluidic chip according to (4), wherein
   the intermediate component and the cover section are bonded together via an adhesive layer.
(6) The microfluidic chip according to any one of (1) to (5), wherein
   no adhesive layer is provided between the partition section and the intermediate component.
(7) The microfluidic chip according to any one of (1) to (5), wherein
   the intermediate component and the partition section are joined together by welding.
(8) The microfluidic chip according to any one of (1) to (5), wherein
   the intermediate component is a resin having thermal fluidity; and
   the channel has a cross section with rounded corners.
(9) The microfluidic chip according to any one of (1) to (8), wherein
   the intermediate component and the partition section are separate from or integrated with each other.
(10) The microfluidic chip according to any one of (1) to (9), wherein
   the intermediate component includes a recess.
(11) The microfluidic chip according to any one of (1) to (10), wherein
   the partition section and the intermediate component are each formed of a resin material; and
   the resin material is a photosensitive resin having photosensitivity to light with a wavelength of 190 nm or more and 400 nm or less in the UV light region.
(12) The microfluidic chip according to any one of (1) to (11), wherein
   the intermediate component is a separate body from the partition section and has a glass transition temperature lower than that of the partition section.
(13) The microfluidic chip according to any one of (1) to (12), wherein
   the intermediate component is a separate body from the partition section and has an exposure sensitivity higher or lower than that of the partition section.
(14) A method of producing a microfluidic chip, including steps of
   applying a photosensitive resin onto a base section;
   exposing the applied photosensitive resin;
   developing and cleaning the exposed photosensitive resin to form a partition section defining a channel on the base section;
   heat-treating the partition section and fluidizing the photosensitive resin to form an intermediate component overlapping at least part of the channel in plan view; and
   bonding a cover layer to one side of the partition section facing away from the base section via the intermediate component.
(15) A method of producing a microfluidic chip, including steps of
   applying a first photosensitive resin onto a base section;
   applying a second photosensitive resin onto the applied first photosensitive resin;
   exposing the first photosensitive resin and the second photosensitive resin;
   developing and cleaning the exposed first photosensitive resin and the exposed second photosensitive resin to form a partition section defining a channel on the base section;
   heat-treating and fluidizing the second photosensitive resin on the partition section to form an intermediate component overlapping at least part of the channel in plan view; and
   bonding a cover layer to one side of the partition section facing away from the base section via the intermediate component.

### 4. Fourth embodiment

A fourth embodiment of the present disclosure will be described. The fourth embodiment of the present disclosure relates to a microfluidic chip and a method of producing a microfluidic chip.

As mentioned above, in µ-TAS, micrometer-sized channels (microchannels) formed on a substrate are often used. Such substrates are called chips, microchips, microfluidic chips, etc.

In microfluidic chips, bubbles may be generated in reaction solutions passing through the microchannels. Such bubbles may be generated due to air entrapment when injecting a fluid such as a reaction solution into the microfluidic chip, boiling when heating the reaction solution, or air entrapment due to uneven liquid flow in the microchannels, or generated from the reaction solution, or due to other causes.

Generation of such bubbles may make the liquid transfer in the microchannels unstable or may inhibit reactions of the solution.

In a proposed method of removing bubbles in microchannels based on the conventional art, for example, a bubble separation section with a mesh member is attached to the channels to remove bubbles generated due to air entrapment in the fluid of the microchannels.

For example, in another proposed method of preparing a microfluidic chip, the structural components are bonded together using an adhesive.

In the microfluidic chip production method based on the conventional art, it has been common to bond the structural components together using adhesives. However, in the case where the bubble separation section mentioned above is attached to microchannels using an adhesive, there has been an issue that the adhesive components may elute into the solution passing through the microchannels and inhibit reactions of the solution.

The fourth embodiment of the present disclosure aims to provide a microfluidic chip and a method of producing the microfluidic chip, which can remove bubbles generated in the microchannels and can prevent adhesive components from eluting into the channel and inhibiting reactions of the solution in the channels.

In order to achieve the above aim, a microfluidic chip according to an aspect of the fourth embodiment of the present disclosure includes a base section, a partition section forming a channel on the base section, and an upper lid section formed on the surface of the partition section facing away from the base section to serve as a lid for the channel, wherein the upper lid section is provided with a bubble removal section that removes bubbles generated in the channel, and no adhesive layer is provided between the partition section and the upper lid section.

In order to achieve the above aim, a microfluidic chip according to another aspect of the fourth embodiment of the present disclosure includes a base section, a partition section forming a channel on the base section, and an upper lid section formed on the surface of the partition section facing away from the base section to serve as a lid for the channel, wherein the upper lid section is provided with a bubble removal section that removes bubbles generated in the channel, and the upper lid section and the partition section are joined together by welding.

In order to achieve the above aim, a microfluidic chip according to still another aspect of the fourth embodiment of the present disclosure includes a channel and an upper lid section serving as a lid for the channel, wherein the material of the upper lid section is a resin having thermal fluidity, and the upper lid section is provided with a bubble removal section for removing bubbles generated in the channel.

A method of producing a microfluidic chip according to an aspect of the fourth embodiment of the present disclosure includes steps of applying a photosensitive resin onto a base section, exposing the applied photosensitive resin, developing and cleaning the exposed photosensitive resin to form a partition section defining a channel on the base section, and heat-treating the partition section and fluidizing the photosensitive resin to form an upper lid section for the channel and to provide a through-hole as a bubble removal section in the upper lid section to remove bubbles generated in the channel.

A method of producing a microfluidic chip according to another aspect of the fourth embodiment of the present disclosure includes steps of applying a first photosensitive resin onto a base section, applying a second photosensitive resin onto the applied first photosensitive resin, exposing the first photosensitive resin and the second photosensitive resin, developing and cleaning the exposed first photosensitive resin and the exposed second photosensitive resin to form a partition section defining a channel on the base section, and heat-treating and fluidizing the second photosensitive resin on the partition section to form an upper lid section for the channel and to provide a through-hole as a bubble removal section in the upper lid section to remove bubbles generated in the channel.

According to these aspects of the fourth embodiment of the present disclosure, there can be provided a microfluidic chip that can remove bubbles generated in the microchannel and can prevent adhesive components from eluting into the channel and inhibiting reactions of the solution in the channel.

A microfluidic chip according to the fourth embodiment of the present disclosure will be described. In the following description, the substrate side of the microfluidic chip may be referred to as lower, and the opposite side of the microfluidic chip from the substrate side (lid component side) may be referred to as upper.

As a result of diligent studies, the present inventors have found that, if an upper lid section serving as a lid for the channel section is formed in a microfluidic chip by heating and fluidizing a resin material, an upper lid can be provided on the partition section without using an adhesive. Similarly, the present inventors have found that fluidization of a resin material can provide a bubble removable configuration to the upper lid section. Thus, the present inventors have invented a microfluidic chip and a method of producing the microfluidic chip, which can efficiently remove bubbles contained in the microchannel and can prevent adhesive components from eluting into the channel and inhibiting reactions of the solution in the channel.

Referring to the drawings, some modes (Embodiments 4-1, 4-2 and 4-3) of the fourth embodiment will be described.

### (4.1) Embodiment 4-1

### (4.1.1) Basic configuration of microfluidic chip

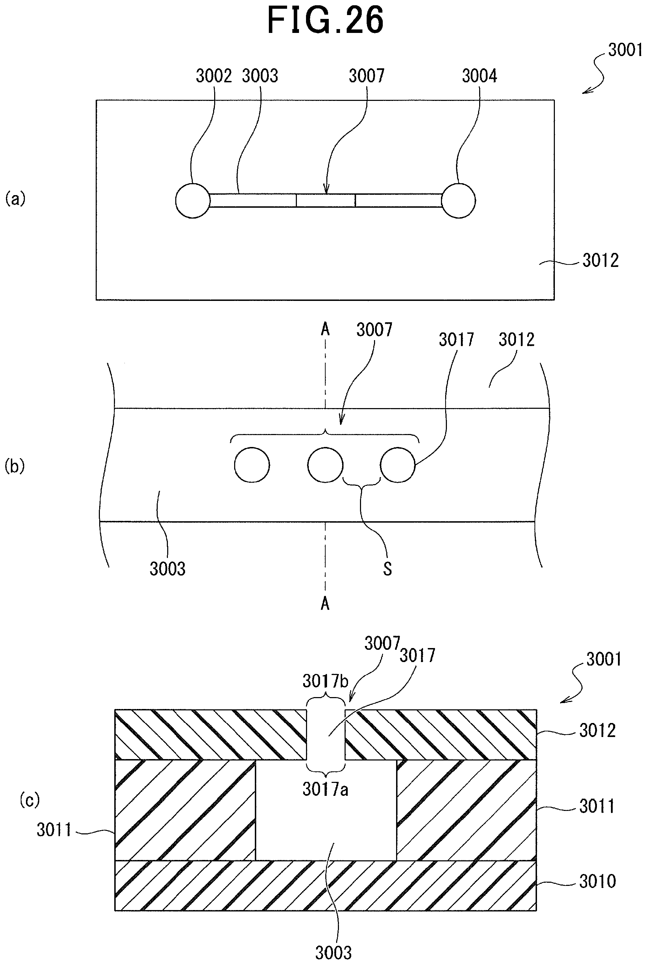

Fig. 26 is a set of schematic diagrams illustrating a configuration example of a microfluidic chip 3001 according to Embodiment 4-1 of the present disclosure (hereinafter referred to as the present embodiment). Specifically, Fig. 26(a) is a schematic plan view illustrating the microfluidic chip 3001 according to the present embodiment. Fig. 26(b) is a schematic plan view illustrating an enlarged bubble removal section 3007 provided to an upper lid layer 3012 of the microfluidic chip 3001 shown in Fig. 26(a). Fig. 26(c) is a schematic cross-sectional view illustrating a cross section of the microfluidic chip 3001 including the bubble removal section 3007, taken along the line A-A of Fig. 26(b).

As shown in Fig. 26(a), the microfluidic chip 3001 includes an inlet 3002 for introducing a fluid (e.g., liquid), a channel section 3003 through which the fluid introduced from the inlet 3002 flows, and an outlet 3004 for discharging the fluid from the channel section 3003. In the microfluidic chip 3001, the upper part of the channel section 3003 is covered with an upper lid layer 3012, and the inlet 3002, the outlet 3004, and a bubble removal section 3007 are provided as through-holes formed in the upper lid layer 3012. Details of the upper lid layer 3012 and the bubble removal section 3007 will be described later.

Fig. 26(a) shows the channel section 3003 that can be seen through a transparent upper lid layer 3012.

The microfluidic chip 3001 may be provided with at least one or more inlets 3002 and outlets 3004, or may be provided with multiple of each. The microfluidic chip 3001 may be provided with multiple channel sections 3003, or may be designed such that the fluid introduced from the inlet 3002 can be merged or separated. The bubble removal section 3007 may be provided at any position as long as it is in a region of the upper lid layer 3012 facing the channel section 3003. The bubble removal section 3007 is adequately arranged at a portion where a highest effect can be exerted in the configuration and usage of the microfluidic chip 3001.

Details of the structural components of the channel section 3003 of the microfluidic chip 3001 will be described later. As shown in Figs. 26(a) to 26(c), the microfluidic chip 3001 includes a substrate (example of the base section) 3010, a partition layer (example of the partition section) 3011 forming a channel on the substrate 3010, and an upper lid layer (example of the upper lid section) 3012 formed on the surface of the partition layer 3011 facing away from the substrate 3010 to serve as a lid for the channel section 3003. In the present embodiment, the upper lid layer 3012 is provided with the bubble removal section 3007 for removing bubbles generated in the channel section 3003. The bubble removal section 3007 of the present embodiment includes through-holes 3017 passing through the upper lid section in the thickness direction, details of which will be described later.

In the microfluidic chip 3001, the channel section 3003 through which the fluid introduced from the inlet 3002 flows is a region surrounded by the substrate 3010, the partition layer 3011, and the upper lid layer 3012. The channel section 3003 is defined within the partition layer 3011 provided on the substrate 3010, and one side of the channel section 3003 facing away from the substrate 3010 is covered with the upper lid layer 3012, as a lid component. In other words, the channel section 3003 is a space configured by the substrate 3010, partition layer 3011, and upper lid layer 3012. As mentioned above, a fluid is introduced into the channel section 3003 from the inlet 3002 (see Fig. 26(a)) provided to the upper lid layer 3012, and the fluid that has passed through the channel section 3003 is discharged from the outlet 3004.

The microfluidic chip 3001 of the present embodiment includes no adhesive layer between the partition layer 3011 and the upper lid layer 3012, details of which will be described later. In the present embodiment, the partition layer 3011 and the upper lid layer 3012 are joined together by welding. Herein, the adhesive layer refers to a layer containing an adhesive and used for bonding multiple structural components together. In the microfluidic chip 3001, the partition layer 3011 and the upper lid layer 3012 are joined together by welding (bonded together) without providing an adhesive layer therebetween, so that adhesive components are prevented from eluting into the channel and inhibiting reactions of the solution in the channel.

As shown in Fig. 26(c), the partition layer 3011 and the upper lid layer 3012 are separate bodies. Herein, the separate bodies refers to the partition layer 3011 and the upper lid layer 3012, for example, being formed of different resin materials. In this case, as a resin material for forming the upper lid layer 3012, a material having favorable characteristics for forming the upper lid and a through-hole as the bubble removal section, such as having glass transition temperature or exposure sensitivity different from those of the partition layer 3011, can be adequately selected. The present disclosure is not limited to this, but the partition layer 3011 and the upper lid layer 3012 may be defined to be separate bodies based on these layers forming a laminated structure with an interface formed therebetween. In this case, the partition layer 3011 and the upper lid layer 3012 may be formed using the same resin material.

### (4.1.1.1) Substrate

The substrate 3010 serves as a base of the microfluidic chip 3001, and the channel section 3003 is configured by the partition layer 3011 provided on the substrate 3010. In other words, the substrate 3010 and the partition layer 3011 configure a body of the microfluidic chip 3001.

The substrate 3010 can be formed using either a translucent material or a non-translucent material. For example, if the state inside the channel section 3003 (state of the fluid) is detected and observed using light, a material having good transparency to light can be used. As the translucent material, resins, glass, etc. can be used. Examples of the resin used as a translucent material for forming the substrate 3010 include acrylic resins, methacrylic resins, polypropylenes, polycarbonate resins, cycloolefin resins, polystyrene resins, polyester resins, urethane resins, silicone resins, and fluororesins, which are preferred from the perspective of suitability for forming the body of the microfluidic chip 3001.

For example, if the state inside the channel section 3003 (state of the fluid) is not required to be detected and observed using light, a non-translucent material may be used. Examples of non-translucent materials include silicon wafers and copper plates. The thickness of the substrate 3010 is not particularly limited, but is preferred to be in the range of 10 µm (0.01 mm) or more and 10 mm or less because some degree of rigidity is required in the channel formation process.

### (4.1.1.2) Partition layer

The partition layer 3011 is provided on the substrate to configure the channel section 3003. The partition layer 3011 can be formed using a resin material. For example, a photosensitive resin can be used as the resin material of the partition layer 3011.

The photosensitive resin forming the partition layer 3011 is preferred to have photosensitivity to light having a wavelength of 190 nm or more and 400 nm or less in the UV light region. As the photosensitive resin, photoresists such as liquid resists and dry film resists can be used. These photosensitive resins may be of positive type with which the photosensitive area becomes soluble, or may be of negative type with which the photosensitive area becomes insoluble. Examples of the photosensitive resin composition suitable for forming the partition layer 3011 in the microfluidic chip 3001 include negative radical type photosensitive resins containing alkalisoluble polymers, addition polymerizable monomers, and photopolymerization initiators. Examples of the photosensitive resin materials include acrylic resins, epoxy resins, polyamide resins, polyimide resins, polyurethane resins, polyester resins, polyether resins, polyolefin resins, polycarbonate resins, polystyrene resins, norbornene resins, phenol novolak resins, and other photosensitive resins. These resins may be used singly, or may be used by mixing or copolymerizing a plurality of them.

In the present embodiment, the resin material used for the partition layer 3011 is not limited to photosensitive resins, but may be, for example, silicone rubber (PDMS: polydimethylsiloxane) or synthetic resins. Examples of the synthetic resins include polymethyl methacrylate resins (PMMA), polycarbonates (PC), polystyrene resins (PS), polypropylenes (PP), cycloolefin polymers (COP), and cycloolefin copolymers (COC). The resin material for the partition layer 3011 is preferred to be appropriately selected according to usage.

The thickness of the partition layer 3011, i.e., the height of the channel section 3003, on the substrate 3010 is not particularly limited, but is required to be larger than the substances (e.g., drugs, bacteria, cells, red blood cells, white blood cells, etc.) to be analyzed and tested which are contained in the fluid introduced into the channel section 3003. Therefore, the thickness of the partition layer 3011, i.e., the height (depth) of the channel section 3003, is preferred to be in the range of 1 µm or more and 500 µm or less, more preferred to be in the range of 10 µm or more and 100 µm, and even more preferred to be in the range of 40 µm or more and 60 µm or less.

Similarly, since the width of the channel section 3003 is required to be larger than the substances to be analyzed and tested, the width of the channel section 3003 defined within the partition layer 3011 is preferred to be in the range of 1 µm or more and 500 µm or less, more preferred to be in the range of 10 µm or more and 100 µm, and even more preferred to be in the range of 10 µm or more and 30 µm or less.

The channel length determined by the partition layer 3011 is required to ensure sufficient reaction time for the reaction solution, and therefore, is preferred to be in the range of 10 mm or more and 100 mm or less, more preferred to be in the range of 30 mm or more and 70 mm or less, and even more preferred to be in the range of 40 mm or more and 60 mm or less.

### (4.1.1.3) Upper lid layer

As shown in Figs. 26(a) to 26(c), the upper lid layer 3012 in the microfluidic chip 3001 of the present embodiment is a lid component covering the channel section 3003. As mentioned above, the upper lid layer 3012 is provided to the surface of the partition layer 3011 facing away from the substrate 3010, and faces the substrate 3010 via the partition layer 3011. More specifically, as shown in Fig. 26(c), the side end portions of the upper lid layer 3012, as viewed in cross section, are supported by the partition layer 3011, the center region thereof faces the substrate 3010, and the center region defines the upper part of the channel section 3003.

The upper lid layer 3012 can be formed using either a translucent material or a non-translucent material. For example, if the state inside the channel is detected and observed using light, a material having good transparency to light can be used. A resin can be used as the translucent material. The resin used for forming the upper lid layer 3012 may be a photosensitive resin similarly to the partition layer 3011.

The photosensitive resin used for the upper lid layer 3012 is a resin having thermal fluidity. If a photosensitive resin having thermal fluidity is used as the material of the upper lid layer 3012, the upper lid layer 3012 can be formed in the microfluidic chip 3001 without using an adhesive. Thus, adhesive components are prevented from eluting into the channel and inhibiting reactions of the solution in the channel.

Conventionally, bonding of a partition layer to a lid component using an adhesive has required advanced equipment and technique, and this made the production method complicated. In the microfluidic chip 3001 according to the present embodiment, the upper lid layer 3012 can be formed on the partition layer 3011 more easily than in the conventional art, using a resin (photosensitive resin in the present example) having thermal fluidity as a material for the upper lid layer 3012. This can eliminate complexity of the production method.

The photosensitive resin having thermal fluidity is preferred to have a melt flow rate (MFR) in the range of 1 g/10 min or more and 100 g/10 min (230°C) or less. This can facilitate formation of the upper lid layer 3012 in the process of production described later.

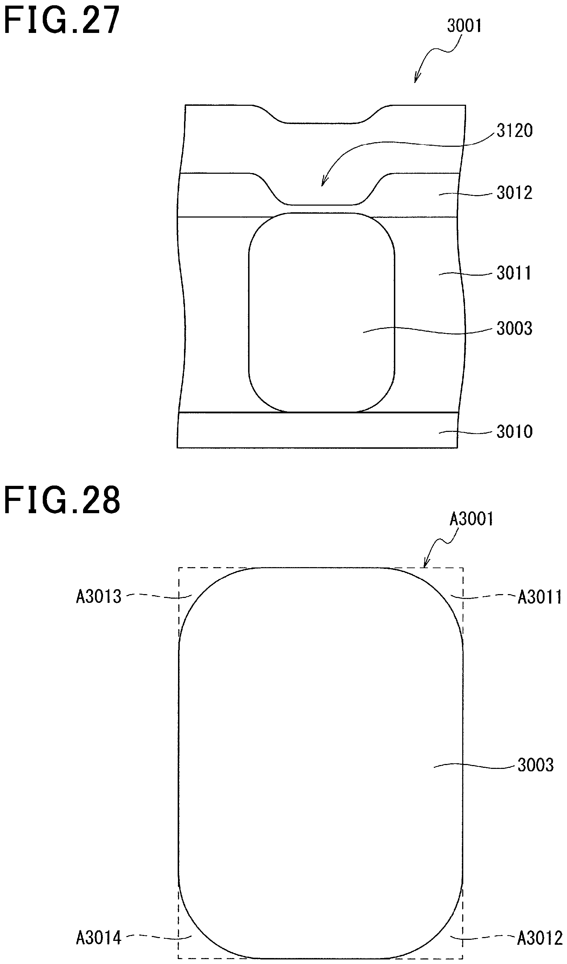

As will be described in detail later, the upper lid layer 3012 is formed by heat-treating and fluidizing (reflowing) a photosensitive resin having thermal fluidity formed on the partition layer 3011, and allowing it to flow onto the channel section 3003. Therefore, as shown in Fig. 27, the upper lid layer 3012 has a recess 3120. In other words, the upper lid layer 3012 has a recessed region. Specifically, the upper lid layer 3012 has a thickness which is reduced from the partition layer 3011 side toward the center of the channel section 3003, forming the recess 3120 as viewed in cross section.

The upper lid layer 3012 may be formed using a resin material with a glass transition temperature (Tg) lower than that of the partition layer 3011. For example, the glass transition temperature of the upper lid layer 3012 may be lower than that of the partition layer 3011 by 30°C to 50°C. In this case, the photosensitive resin forming the partition layer 3011 is hardly fluidized because the glass transition temperature (Tg) thereof is higher than that of the photosensitive resin forming the upper lid layer 3012. Accordingly, when forming the upper lid layer 3012, the partition layer 3011 is suppressed from being fluidized and changing the channel pattern.

The glass transition temperature (Tg) of the upper lid layer 3012 is preferred to be in the range of 100°C or more and 300°C or less.

The upper lid layer 3012 is preferred to have an exposure sensitivity in the range of 5 µC/cm² or more and 50 µC/cm² or less.

The upper lid layer 3012 may have an exposure sensitivity different from that of the partition layer 3011. For example, the exposure sensitivity of the upper lid layer 3012 may be higher than that of the partition layer 3011 by the range of 5 µC/cm² or more and 20 µC/cm² or less.

For example, the exposure sensitivity of the upper lid layer 3012 may be lower than that of the partition layer 3011 by 5 µC/cm² or more and 20 µC/cm² or less.

Thus, the upper lid layer 3012 may be configured to have an exposure sensitivity higher or lower than that of the partition layer 3011.

If the exposure sensitivity is different between the upper lid layer 3012 and the partition layer 3011, the exposure dose may be set to a value matching the photosensitive resin forming the partition layer 3011, so that the opening width of the photosensitive resin forming the partition layer 3011, i.e., the width of the channel pattern, can be made sufficiently large. Thus, when fluidizing the resin material of the upper lid layer during formation of the upper lid layer 3012, if fluidization has also occurred in the partition layer resin, the opening width of the photosensitive resin forming the partition layer 3011 can be made sufficiently large and a sufficient space can be retained as the channel section 3003.

### (4.1.1.3.1) Bubble removal section

Referring back to Fig. 26, as shown in Figs. 26(a) to 26(c), the upper lid layer 3012 is provided with the bubble removal section 3007 for removing bubbles generated in the channel section 3003. Specifically, the bubble removal section 3007 is formed as part of the upper lid layer 3012.

Thus, the microfluidic chip 3001 can remove bubbles generated in the channel section 3003, and can prevent adhesive components from eluting into the channel and inhibiting reactions of the solution in the channel.

The bubbles generated in the channel section 3003 include, for example, bubbles generated due to entrapment of air when injecting (introducing) a fluid such as a reaction solution into the channel section 3003 from the inlet 3002, bubbles due to boiling occurring due to heating of the introduced reaction solution, bubbles generated due to uneven flow of the reaction solution in the channel section 3003, bubbles generated from the reaction solution, or bubbles generated due to other causes.

As shown in Fig. 26(a), the bubble removal section 3007 is provided to a region in the upper lid layer 3012 facing the channel section 3003, so as to be located along the fluid transfer direction. For ease of understanding, in Fig. 26(a), the region, in which the bubble removal section 3007 is provided in the upper lid layer 3012, is illustrated with a rectangular border; however, the rectangular border is not an actually provided structural component. The upper lid layer 3012 may be provided with one bobble removal section 3007 or may be provided with two or more (multiple) bubble removal sections 3007 as necessary.

As shown in Figs. 26(b) and 26(c), the bubble removal section 3007 includes through-holes 3017 passing through the upper lid layer 3012 in the thickness direction. As shown in Fig. 26(b), the through-holes 3017 are in communication with the outside of (the environment external to) the channel section 3003. Thus, bubbles can be reliably removed from the channel section 3003. Specifically, when the fluid, such as a reaction solution, passes through the region in the channel section 3003 where the through-holes 3017 are provided, gases forming bubbles are discharged outside the channel section 3003, i.e., discharged into the external environment (atmosphere), via the through-holes 3017. Thus, bubbles can be efficiently removed from the channel section 3003.

The bubble removal section 3007 is formed of a resin, i.e., photosensitive resin having thermal fluidity, which is the same as the material of the upper lid layer 3012. Although details will be described later, due to use of the photosensitive resin having thermal fluidity as a material of the upper lid layer 3012, the bubble removal section 3007 having the through-holes 3017, i.e., micropores, can be formed simultaneously with the upper lid layer 3012 as a part thereof, without the need of bonding thereto a separate component using an adhesive or the like. As mentioned above, in the microfluidic chip 3001, the upper lid layer 3012 having the bubble removal section 3007 is joined to the partition layer 3011 by welding, without using an adhesive. Thus, in the microfluidic chip 3001, bubbles generated in the channel section 3003 can be removed, and adhesive components can be prevented from eluting into the channel and inhibiting reactions of the solution in the channel.

In the present example, the bubble removal section 3007 is configured by multiple through-holes 3017. The multiple through-holes 3017 are formed in the channel section 3003 along the liquid transfer direction. In the microfluidic chip 3001 of the present embodiment, the bubble removal section 3007 may have one through-hole 3017, or may have multiple through-holes 3017.

If multiple through-holes 3017 are provided to the bubble removal section 3007, the distance from one through-hole 3017 to the subsequent through-hole 3017, i.e., an arrangement interval S between the multiple through-holes 3017 shown in Fig. 26(b), is preferred to be in the range of 5 µm or more and 1,000 µm or less. With the arrangement interval S in the above range, bubbles generated in the channel section 3003 can be more efficiently removed.

As shown in Fig. 26(c), each through-hole 3017 provided to the bubble removal section 3007 in the present embodiment has an opening end 3017a on the channel section 3003 side with a diameter equal to that of an opening end 3017b on the side opposite from the channel section 3003, and the width of the through-hole 3017 passing through the upper lid layer 3012 is constant in the thickness direction. The present disclosure is not limited to this. For example, the diameter of the opening end 3017a of each through-hole 3017 on the channel section 3003 side may be different from the diameter of the opening end 3017b on the side opposite from the channel section 3003, and the width may vary accordingly in the thickness direction. For example, each through-hole 3017 may have a shape in which the opening end 3017a is larger in diameter than the opening end 3017b, with the width reduced accordingly from the opening end 3017a toward the opening end 3017b (e.g., trapezoidal shape in the cross section shown in Fig. 26(c)). Alternatively, each through-hole 3017 may have a shape in which the opening end 3017a is smaller in diameter than the opening end 3017b, with the width increased accordingly from the opening end 3017a toward the opening end 3017b (e.g., inverted trapezoidal shape in the cross section shown in Fig. 26(c)).

Alternatively, in each through-hole 3017, the opening end 3017a may be equal in diameter with the opening end 3017b, and the width may vary in the middle portion (portion between the opening ends 3017a and 3017b) in the thickness direction. In this case, for example, each through-hole 3017 may have a shape in which the width of the middle portion is decreased (constricted) or increased in the cross section shown in Fig. 26(c).

As shown in Fig. 26(b), the opening ends 3017a and 3017b of each through-hole 3017 configuring the bubble removal section 3007 may have a circular shape in plan view. The circular shape may include a perfect circle, ellipse, oval, and oblong circle. If the opening ends 3017a and 3017b of each through-hole 3017 have a circular shape, the diameter (maximum diameter) thereof is preferred to be in the range of 1 µm or more and 100 µm or less. Thus, bubbles can be removed from the channel section 3003 without causing leakage.

In the present disclosure, the shape of the opening ends 3017a and 3017b of each through-hole 3017 in plan view is not limited to circular. The shape of the opening ends 3017a and 3017b of each through-hole 3017 may be polygonal in plan view. In this case, the polygonal shape may have rounded corners, with the sides connected by arcs. If the opening ends 3017a and 3017b of each through-hole 3017 have a polygonal shape, the longitudinal width thereof is preferred to be in the range of 1 µm or more and 100 µm or less. Thus, bubbles can be removed from the channel section 3003 without causing liquid leakage.

The shape and the diameter of the opening ends 3017a and 3017b of each through-hole 3017 may be adequately designed within the above range according to the width or length of the channel section 3003.

The opening ends 3017a and 3017b do not have to have the same shape. For example, one of the opening ends 3017a and 3017b may be circular and the other may be polygonal.

Thus, in the microfluidic chip 3001 of the present embodiment, the upper lid layer 3012 has a function as a lid component and a function as a bubble removal section.

### (4.1.1.4) Channel configuration

Fig. 28 is a diagram illustrating an example of a cross section of the channel section 3003 of the microfluidic chip 3001 according to the present embodiment. In the present embodiment, the cross section of the channel section 3003 is preferred to have rounded corners (e.g., the sides of the channel section 3003 in a cross section are connected by arcs). Thus, the transfer speed or flow rate of the fluid (e.g., reaction solution) in the channel section 3003 can be stabilized, and the substances to be tested can be suppressed from being retained at the corners.

Referring to Fig. 28, a cross section of the channel section 3003 in the present embodiment will be described. Fig. 28 is a schematic cross-sectional view illustrating an enlarged cross section of the channel section 3003 having rounded corners. As shown in Fig. 28, there is a difference in area between a virtual cross section A3001 when the cross-sectional shape of the channel section 3003 is rectangular and the cross section of the channel section 3003 having rounded corners. Specifically, as shown in Fig. 28, the four corners of the cross section of the channel section 3003 each have an arc shape. Therefore, the area of the cross section of the channel section 3003 is smaller than that of the virtual cross section A3001 by the sum of the areas of the virtual corner portions A3011, A3012, A3013 and A3014.

In the present embodiment, the area of the cross section of the channel section 3003 having rounded corners may be in the range of 95% or more and 98% or less of the surface area of the virtual cross section A3001. Fig. 28 shows the channel section 3003 having a rectangular cross section with rounded corners; however, without being limited to this, the cross section may have shapes other than a rectangular shape (polygonal shape with rounded corners). In this case also, the area of the cross section of the channel section 3003 may be in the range of 95% or more and 98% or less of the area of the virtual cross section not having rounded corners.

The channel section 3003 is preferred to have a 10-point surface roughness Rz in the range of 0.001 µm or more and 0.03 µm or less. The 10-point surface roughness Rz of the channel section 3003 refers to the roughness of the side surfaces of parts of the partition layer 3011 on the channel section 3003 side, and the surface of the substrate 3010 on the channel section 3003 side.

If the 10-point surface roughness Rz is adequately designed within the above range, testing can be suitably performed according to the reaction solutions or the substances to be tested, and according to the desired testing conditions. For example, the surface roughness Rz of the channel section 3003 being in the range of 0.001 µm or more and 0.01 µm or less can reduce contact of the fluid (e.g., reaction solution) or substance to be tested introduced into the channel section 3003, with the inner surfaces of the channel section 3003 and can improve liquid transferability (e.g., liquid transfer speed or flow rate).

The surface roughness Rz of the channel section 3003 being greater than 0.02 µm and less than or equal to 0.03 µm can reduce the liquid transfer speed and can ensure time of stay in the channel section 3003, i.e., reaction time, of the fluids or substances to be tested introduced into the channel section 3003.

The surface roughness Rz of the channel section 3003 being greater than 0.01 µm and less than or equal to 0.02 µm can ensure both of an appropriate liquid transfer speed and reaction time of the fluids or substances to be tested in the channel section 3003.

The surface roughness of the channel section 3003 can be adequately controlled using a known etching method or the like during production of the microfluidic chip 3001.

### (4.1.2) Method of producing microfluidic chip

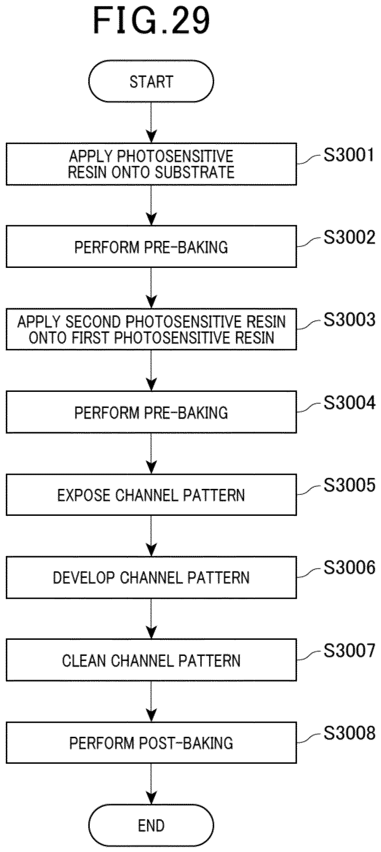

Next, a method of producing the microfluidic chip 3001 of the present embodiment will be described. Fig. 29 is a flowchart illustrating an example of a method of producing the microfluidic chip 3001 according to the present embodiment.

Description herein will be provided taking an example in which the partition layer 3011 is formed of a photosensitive resin.

### (Step S3001)

In the method of producing the microfluidic chip 3001 of the present embodiment, a resin for forming a partition layer 3011 (one example of the first photosensitive resin) is applied first onto a substrate 3010. Thus, a resin layer for forming a partition layer 3011 is provided on the substrate 3010. In the method of producing the microfluidic chip 3001 of the present embodiment, for example, a resin layer (first photosensitive resin layer) made of a photosensitive resin is formed on the substrate 3010.

The photosensitive resin layer is formed on the substrate 3010, for example, by applying a photosensitive resin onto the substrate 3010. Examples of the coating method include spin coating, spray coating, and bar coating, among which, spin coating is preferred from the perspective of film thickness controllability. The photosensitive resin that can be applied to the substrate 3010 can be in various forms such as liquid, solid, gel-like, and film-like forms. Of these forms, liquid resists are preferred to be used for forming the photosensitive resin layer. Such a liquid resist can be positive or negative type, which may be adequately determined according to the characteristics of the channel pattern.

The resin (e.g., photosensitive resin) may be applied to the substrate 3010 such that the thickness of the resin layer (e.g., photosensitive resin layer), i.e., the thickness of the partition layer 3011, will be in the range of 1 µm or more and 500 µm or less.

### (Step S3002)

Following formation of the first photosensitive resin layer of the partition resin on the substrate 3010, heat treatment (pre-baking treatment) is performed for the purpose of removing solvent contained in the resin (e.g., photosensitive resin) applied to the substrate 3010. In the method of producing the microfluidic chip 3001 of the present embodiment, pre-baking treatment is not essential but may be adequately performed under optimal conditions (temperature and duration) according to the characteristics of the resin used. For example, if the resin layer on the substrate 3010 is formed of a photosensitive resin, pre-baking may be performed under optimal temperature and duration adequately determined according the characteristics of the photosensitive resin. For the purpose of improving adhesion between the substrate and the photosensitive resin, the substrate may undergo an HMDS treatment or may be provided with a thin resin coating as necessary.

### (Step S3003)

Next, a resin for forming an upper lid layer 3012 (one example of the second photosensitive resin) is applied onto the first photosensitive resin layer. Thus, a resin layer for forming an upper lid layer 3012 is provided on the pre-baked partition resin. In the method of producing the microfluidic chip 3001 of the present embodiment, for example, a resin layer (second photosensitive resin layer) made of a photosensitive resin having thermal fluidity is formed on the substrate 3010. The second photosensitive resin layer can be formed on the substrate 3010 similarly to the first photosensitive resin layer formed at step S3001.

### (Step S3004)

Following formation of the second photosensitive resin layer of the upper lid resin on the first photosensitive resin layer of the partition resin, heat treatment (pre-baking treatment) is performed for the purpose of removing solvent contained in the upper lid resin applied to the first photosensitive resin layer. The pre-baking treatment of the present step is not essential but, similarly to the pre-baking treatment at step S3002, may be adequately performed under optimal conditions (temperature and duration) according to the characteristics of the resin used. For the purpose of improving adhesion between the first photosensitive resin layer and the second photosensitive resin, the substrate may undergo an HMDS treatment or may be provided with a thin resin coating as necessary. Thus, two photosensitive resin layers are formed on the substrate 3010.

As shown in steps S3001 to S3004, the two photosensitive resin layers (first and second photosensitive resin layers) for forming a partition layer 3011 and an upper lid layer 3012 are joined together by welding without using an adhesive. In other words, in the microfluidic chip 3001 of the present embodiment, the partition layer 3011 and the upper lid layer 3012 are joined together by welding. More specifically, in the microfluidic chip 3001 of the present embodiment, the partition layer 3011 and the upper lid layer 3012 can be joined together without using an intermediate component (e.g., adhesive). Thus, adhesive components can be prevented from eluting into the channel section 3003, and poor bonding can be avoided, which would have been induced by uneven adhesive thickness.

The number of the photosensitive resin layers is not limited to two as in the present embodiment, but may be three or more.

### (Step S3005)

Next, the resins (e.g., first and second photosensitive resins) applied to the substrate 3010 are exposed. Specifically, a channel pattern is drawn by exposure on the photosensitive resins applied to the substrate 3010. Exposure may be performed, for example, using an exposure device that uses UV light as a light source, or using a laser drawing device. Among them, proximity exposure using UV light as a light source or exposure using a contact exposure device is preferred. If a proximity exposure device is used, exposure is performed via a photomask with a channel pattern array for the microfluidic chip 3001. The photomask may be a photomask with a light-shielding film that has a double-layer structure of chromium and chromium oxide.

If the photosensitive resins (first and second photosensitive resin layers) applied to the substrate 3010 are positive resists, the exposed regions are dissolved, forming the channel section 3003, and the photosensitive resins in the unexposed regions remain as parts of the partition layer 3011 and the upper lid layer 3012. If the photosensitive resins applied to the substrate 3010 are negative resists, the photosensitive resins in the exposed regions remain as parts of the partition layer 3011 and the upper lid layer 3012, and the unexposed regions are dissolved, forming the channel section 3003. In this way, in the method of producing the microfluidic chip 3001 of the present embodiment, the partition layer 3011 configuring the channel section 3003 can be formed on the substrate 3010 using photolithography.

If a chemically amplified resist or the like is used for forming a resin layer on the substrate 3010, heat treatment (post-exposure baking: PEB) may be further performed after exposure to promote catalytic reaction of the acid generated due to exposure.

### (Step S3006)

Next, the exposed photosensitive resins are developed to form a channel pattern.

The development is performed, for example, using a spray-, dip- or paddle-type or other types of developing device, by reaction of the photosensitive resin with a developer. Examples of the developer include sodium carbonate aqueous solution, tetramethylammonium hydroxide, potassium hydroxide, and organic solvents. The developer is not limited to these, but an optimal developer may be adequately used according to the characteristics of the photosensitive resins. The concentration or the time of development can be adequately adjusted to optimal conditions according to the characteristics of the photosensitive resins.

### (Step S3007)

Next, the resin layers (photosensitive resin layers) on the substrate 3010 are cleaned to completely remove the developer used for development. Cleaning may be performed, for example, using a spray-, shower-, immersion-type or other types of cleaning device. An optimal cleaning solution may be adequately used from among, for example, pure water, isopropyl alcohol, and the like to remove the developer used for development. Cleaning is followed by drying using a spin dryer, IPA vapor dryer, natural drying, etc.

At this stage also, the second photosensitive resin layer of the upper lid resin remains on the partition layer 3011.

### (Step S3008)

Next, the channel pattern, i.e., the partition layer 3011 forming the channel section 3003 and the second photosensitive resin layer, undergoes heat treatment (post-baking). With this post-baking treatment, residual moisture generated during development or cleaning is removed. Also, with this post-baking treatment, an upper lid layer 3012 serving as a lid for the channel section 3003 and defining the upper part of the channel section 3003, and a bubble removal section 3007 for removing bubbles generated in the channel section 3003, are formed. Specifically, this post-baking treatment can promote fluidization (reflow) of the upper lid resin, i.e., the photosensitive resin having fluidity, and form an upper lid layer 3012 for the channel section 3003 and through-holes 3017 configuring the bubble removal section 3007. The post-baking treatment is performed with optimal temperature and duration determined according the characteristics of the upper lid resin. As shown in Fig. 26, the upper lid layer 3012 is a lid component covering the channel 3003 and thus is formed over the channel section 3003. The inlet 3002 and the outlet 3004 are open without being covered with the upper lid layer 3012.

For example, the post-baking treatment is performed using a hot plate, oven, etc. If the drying performed at the cleaning step S3007 is insufficient, the developer or moisture generated during cleaning may remain in the partition layer 3011. Solvent that has not been removed in the pre-baking treatment may also remain in the partition layer 3011. These materials can be removed by performing post-baking treatment.

As described above, the method of producing the microfluidic chip 3001 according to the present embodiment includes steps of applying a partition resin onto a substrate 3010 (step S3001), applying an upper lid resin onto the applied partition resin (step S3003), exposing the partition resin and the upper lid resin (step S3005), developing and cleaning the exposed partition resin and upper lid resin to form a partition layer 3011 defining a channel section 3003 on the substrate 3010 (steps S3006 and S3007), and heat-treating and fluidizing the upper lid resin on the partition layer 3011 to form an upper lid layer 3012 for the channel section 3003 and to provide a through-hole 3017 as a bubble removal section 3007 in the upper lid layer 3012 to remove bubbles generated in the channel section 3003 (step S3008).

Thus, the partition layer 3011 and the upper lid layer 3012 can be joined together by welding without using an adhesive layer, so that adhesive components are prevented from eluting into the channel section 3003 and inhibiting reactions of the solution in the channel.

### (4.1.3) Details of steps of producing partition layer and upper lid layer

In the method of producing the microfluidic chip 3001 of the present embodiment, production steps are adjusted according to the physical properties of the partition resin for forming the partition layer 3011 and the upper lid resin for forming the upper lid layer 3012. A specific example will be described below.

### (4.1.3.1) Formation of partition layer and upper lid layer using resins having different glass transition temperatures

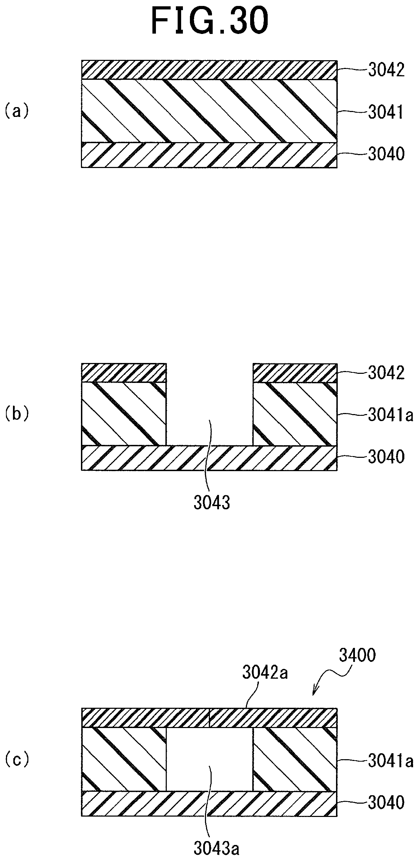

Referring to Fig. 30, a description will be given of forming the partition layer 3011 and the upper lid layer 3012 in the case where the partition resin and the upper lid resin have different glass transition temperatures. Fig. 30(a) is a schematic cross-sectional view illustrating a first photosensitive resin layer 3041 and a second photosensitive resin layer 3042 formed on a substrate 3040, Fig. 30(b) is a schematic cross-sectional view illustrating a channel pattern on the substrate 3040, and Fig. 30(c) is a schematic cross-sectional view illustrating a configuration of a microfluidic chip 3400 according to the present example.

In the present example, a description will be given of a method of producing a microfluidic chip under conditions in which the glass transition temperature (Tg) of the upper lid resin is lower than that of the partition resin [Glass transition temperature (Tg) of upper lid resin < Glass transition temperature (Tg) of partition resin].

As shown in Fig.30(a), in the present example, a first photosensitive resin layer 3041 is formed by applying a partition photosensitive resin onto the substrate 3040 at the coating step S3001. The partition photosensitive resin is applied to the substrate 3040 with a desired thickness using spin coating, for example. In the present example, a second photosensitive resin layer 3042 is formed by applying an upper lid photosensitive resin onto the first photosensitive resin layer 3041 at step S3003. In the present example, the upper lid photosensitive resin forming the second photosensitive resin layer 3042 has a glass transition temperature (Tg) lower than that of the partition photosensitive resin forming the first photosensitive resin layer 3041. The second photosensitive resin layer 3042 is applied with a desired thickness onto the first photosensitive resin layer 3041 using spin coating, similarly to the first photosensitive resin layer 3041.

In the present example, at the exposure step S3005, a channel pattern is drawn, via a photomask, on the first and second photosensitive resin layers 3041 and 3042 applied to the substrate 3040. For example, the present example uses a proximity exposure device using light with a wavelength of 350 nm or more and 400 nm or less in the UV light region as a light source. Next, at the development step S3006, a channel pattern 3043 is formed by developing the exposed first and second photosensitive resin layers 3041 and 3042. For example, a sodium carbonate solution may be sprayed as a developer. Next, at step S3007, the developed first and second photosensitive resin layers 3041 and 3042 are cleaned to completely remove the developer. For example, ultra-pure water may be sprayed for cleaning. Thus, as shown in Fig. 30(b), a partition layer 3041a is formed defining the channel pattern 3043.

Next, the microfluidic chip, in which the channel pattern 3043 has been formed, undergoes heat treatment (post-baking) at step S3008. In the present example, heat treatment is performed using a hot plate at a temperature near the glass transition temperatures (Tg) of the second photosensitive resin layer 3042. In the present example, heat treatment promotes fluidization (reflow) of the second photosensitive resin layer 3042, i.e., the upper lid resin, and portions of the upper lid resin on opposing right and left parts of the partition layer 3011 flow toward the center of the channel pattern 3043. The portions of the upper lid resin that have flowed from parts of the partition layer 3041a are joined together on the side opposite from the substrate 3040, i.e., on the upper side of the channel pattern 3043, to form an upper lid layer 3042a. Thus, as shown in Fig. 30(c), the upper part of the channel pattern 3043 is covered with the upper lid layer 3042a to form a channel section 3043a, thereby producing the microfluidic chip 3400.

As in the present example, if the glass transition temperature (Tg) of the first photosensitive layer 3041, i.e., the partition resin, forming the partition layer 3041a is higher than that of the second photosensitive resin layer 3042, i.e., the upper lid resin, the partition resin is not fluidized substantially even when the upper lid resin is fluidized (reflows) due to heat treatment. Accordingly, by satisfying the condition of [Glass transition temperature (Tg) of upper lid resin < Glass transition temperature (Tg) of partition resin], the upper lid layer 3042a can be easily formed by causing the upper lid resin to fluidize, without deforming the channel pattern due to fluidization of the upper lid resin.

### (4.1.3.2) Formation of partition layer and upper lid layer using resins with different exposure sensitivities (1)

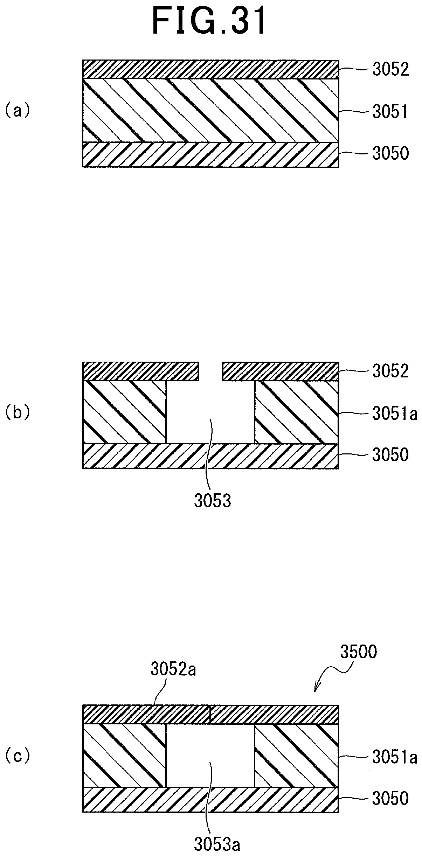

Referring to Fig. 31, a description will be given of forming the partition layer 3011 and the upper lid layer 3012 in the case where the partition resin and the upper lid resin have different exposure sensitivities. Fig. 31(a) is a schematic cross-sectional view illustrating a first photosensitive resin layer 3051 and a second photosensitive resin layer 3052 formed on a substrate 3050, Fig. 31(b) is a schematic cross-sectional view illustrating a channel pattern on the substrate 3050, and Fig. 31(c) is a schematic cross-sectional view illustrating a configuration of a microfluidic chip 3500 according to the present example.

In the present example, a description will be given of a method of producing a microfluidic chip under conditions in which the exposure sensitivity (C/cm²) of the upper lid resin is lower than that of the partition resin [Exposure sensitivity (C/cm²) of upper lid resin < Exposure sensitivity (C/cm²) of partition resin].

As shown in Fig.31(a), in the present example, a first photosensitive resin layer 3051 is formed by applying a partition photosensitive resin onto the substrate 3050 at the coating step S3001. The partition photosensitive resin is applied to the substrate 3050 with a desired thickness using spin coating, for example. The first photosensitive resin layer 3051, i.e., the partition resin, is a positive resist. In the present example, a second photosensitive resin layer 3052 is formed by applying an upper lid photosensitive resin onto the first photosensitive resin layer 3051 at step S3003. The second photosensitive resin layer 3052, i.e., the upper lid resin, is a positive resist. In other words, in the present example, positive resists are used as the partition photosensitive resin and the upper lid photosensitive resin.

In the present example, the upper lid photosensitive resin forming the second photosensitive resin layer 3052 has an exposure sensitivity (C/cm²) lower than that of the partition photosensitive resin forming the first photosensitive resin layer 3051. The second photosensitive resin layer 3052 is applied with a desired thickness onto the first photosensitive resin layer 3051 using spin coating, similarly to the first photosensitive resin layer 3051. For example, a chemically amplified resist may be used as the partition resin for forming the first photosensitive resin layer 3051, and a non-chemically amplified resist may be used as the upper lid resin for forming the second photosensitive resin layer 3052.

In the present example, at the exposure step S3005, a channel pattern is drawn, via a photomask, on the first and second photosensitive resin layers 3051 and 3052 applied to the substrate 3050. For example, the present example uses a proximity exposure device using light with a wavelength of 350 nm or more and 400 nm or less in the UV light region as a light source. An optimal exposure dose is determined herein for the first photosensitive resin layer 3051. As mentioned above, in the present example, the exposure sensitivity of the upper lid resin forming the second photosensitive resin layer 3052 is lower than that of the partition resin forming the first photosensitive resin layer 3051. Therefore, although reaction is insufficient in the second photosensitive resin layer 3052 with the exposure dose matching the first photosensitive resin layer 3051, there is no problem in pattern resolution.

Next, at the development step S3006, a channel pattern 3053 is formed by developing the exposed first and second photosensitive resin layers 3051 and 3052. For example, a sodium carbonate solution may be sprayed as a developer. Next, at step S3007, the developed first and second photosensitive resin layers 3051 and 3052 are cleaned to completely remove the developer. For example, ultra-pure water may be sprayed for cleaning. Thus, as shown in Fig. 31(b), a partition layer 3051a is formed defining the channel pattern 3053. In the present example, as mentioned above, the second photosensitive resin layer 3052 is made of a resin having an exposure sensitivity lower than that of the first photosensitive resin layer 3051. Therefore, as shown in Fig. 31(b), the opening width in the exposed region in the second photosensitive resin layer 3052 is smaller than the opening width of the partition layer 3051a formed of the first photosensitive resin layer 3051.

Next, the microfluidic chip, in which the channel pattern 3053 has been formed, undergoes heat treatment (post-baking) at step S3008. In the present example, heat treatment is performed using a hot plate at a temperature near the glass transition temperatures (Tg) of the first and second photosensitive resin layers 3051 and 3052. In the present example, heat treatment promotes fluidization (reflow) of the second photosensitive resin layer 3052, i.e., the upper lid resin, and portions of the upper lid resin on right and left parts of the partition layer 3051a flow toward the center of the channel pattern 3053. The portions of the upper lid resin that have flowed from parts of the partition layer 3051a are joined together on the side opposite from the substrate 3050, i.e., on the upper side of the channel pattern 3053, to form an upper lid layer 3052a. Thus, as shown in Fig. 31(c), the upper part of the channel pattern 3053 is covered with the upper lid layer 3052a to form a channel section 3053a, thereby producing the microfluidic chip 3500.

In the present example, the glass transition temperature (Tg) of the first photosensitive resin layer 3051, i.e., the partition resin, forming the partition layer 3051a is equal to that of the second photosensitive resin layer 3052, i.e., the upper lid resin, forming the upper lid layer 3052a. Therefore, when the upper lid resin is fluidized (reflows), the partition resin is also fluidized. However, in the present example, the first and second photosensitive resin layers 3051 and 3052 are each formed of a positive resist, and the exposure sensitivity of the second photosensitive resin layer 3052 is lower than that of the first photosensitive resin layer 3051. Therefore, as shown in Fig. 31(b), the opening width of the second photosensitive resin layer 3052 is formed to be smaller than the opening width of the partition layer 3051a formed of the first photosensitive resin layer 3051, and the opening width of the partition layer 3051a formed of the first photosensitive resin layer 3051 is formed to be sufficiently larger than the opening width of the second photosensitive resin layer 3052.

Accordingly, the width of the channel section 3053a is reduced by more than the opening width of the channel pattern 3053 is, due to occurrence of fluidization in the first photosensitive resin layer 3051 with a degree that is approximately the same as in the second photosensitive resin layer 3052; however, as shown in Fig. 31(c), the width of the channel section 3053a is sufficiently ensured.

Accordingly, if the first and second photosensitive resin layers 3051 and 3052 are each formed of a positive resist and if the condition of [Exposure sensitivity of upper lid resin < Exposure sensitivity of partition resin] is satisfied, the upper lid resin and the partition resin can be fluidized to easily form the upper lid layer 3052a, while sufficiently retaining the width of the channel section.

### (4.1.3.3) Formation of partition layer and upper lid layer using resins with different exposure sensitivities (2)

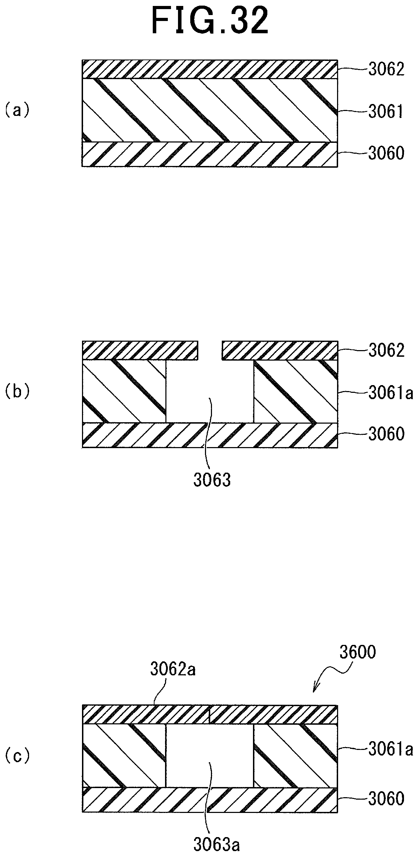

Referring to Fig. 32, a description will be given of forming the partition layer 3011 and the upper lid layer 3012 in the case where the partition resin and the upper lid resin have different exposure sensitivities. Fig. 32(a) is a schematic cross-sectional view illustrating a first photosensitive resin layer 3061 and a second photosensitive resin layer 3062 formed on a substrate 3060, Fig. 32(b) is a schematic cross-sectional view illustrating a channel pattern on the substrate 3060, and Fig. 32(c) is a schematic cross-sectional view illustrating a configuration of a microfluidic chip 3600 according to the present example.

In the present example, a description will be given of a method of producing a microfluidic chip under conditions in which the exposure sensitivity (C/cm²) of the upper lid resin is higher than that of the partition resin [Exposure sensitivity (C/cm²) of upper lid resin > Exposure sensitivity (C/cm²) of partition resin].

As shown in Fig.32(a), in the present example, a first photosensitive resin layer 3061 is formed by applying a partition photosensitive resin onto the substrate 3060 at the coating step S3001. The partition photosensitive resin is applied to the substrate 3060 with a desired thickness using spin coating, for example. The first photosensitive resin layer 3061, i.e., the partition resin, is a negative resist. In the present example, a second photosensitive resin layer 3062 is formed by applying an upper lid photosensitive resin onto the first photosensitive resin layer 3061 at step S3003. The second photosensitive resin layer 3062, i.e., the upper lid resin, is a negative resist. In other words, in the present example, negative resists are used as the partition photosensitive resin and the upper lid photosensitive resin.

In the present example, the upper lid photosensitive resin forming the second photosensitive resin layer 3052 has an exposure sensitivity (C/cm²) lower than that of the partition photosensitive resin forming the first photosensitive resin layer 3051. The second photosensitive resin layer 3052 is applied with a desired thickness onto the first photosensitive resin layer 3051 using spin coating, similarly to the first photosensitive resin layer 3051. For example, a chemically non-amplified resist may be used as the partition resin for forming the first photosensitive resin layer 3061, and a chemically amplified resist may be used as the upper lid resin for forming the second photosensitive resin layer 3062.

In the present example, at the exposure step S3005, a channel pattern is drawn, via a photomask, on the first and second photosensitive resin layers 3061 and 3062 applied to the substrate 3060. For example, the present example uses a proximity exposure device using light with a wavelength of 350 nm or more and 400 nm or less in the UV light region as a light source. An optimal exposure dose is determined herein for the first photosensitive resin layer 3061. As mentioned above, in the present example, the exposure sensitivity of the upper lid resin forming the second photosensitive resin layer 3062 is higher than that of the partition resin forming the first photosensitive resin layer 3061. Therefore, in the second photosensitive resin layer 3062, the exposure dose matching the first photosensitive resin layer 3061 is necessary and sufficient.

Next, at the development step S3006, a channel pattern 3063 is formed by developing the exposed first and second photosensitive resin layers 3061 and 3062. For example, a sodium carbonate solution may be sprayed as a developer. Next, at step S3007, the developed first and second photosensitive resin layers 3061 and 3062 are cleaned to completely remove the developer. For example, ultra-pure water may be sprayed for cleaning. Thus, as shown in Fig. 32(b), a partition layer 3061a is formed defining the channel pattern 3063. In the present example, the exposure sensitivity of the second photosensitive resin layer 3062 is higher than that of the first photosensitive resin layer 3061. Therefore, as shown in Fig. 32(b), after exposure, the region in the second photosensitive resin layer 3062 remaining as a pattern in the exposed portion becomes larger than the first photosensitive resin layer 3061. Consequently, as shown in Fig. 32(b), the opening width in the exposed region in the second photosensitive resin layer 3062 becomes smaller than the opening width of the partition layer 3061a formed of the first photosensitive resin layer 3061.

Next, the microfluidic chip, in which the channel pattern 3063 has been formed, undergoes heat treatment (post-baking) at step S3008. In the present example, heat treatment is performed using a hot plate at a temperature near the glass transition temperatures (Tg) of the first and second photosensitive resin layers 3061 and 3062. In the present example, heat treatment promotes fluidization (reflow) of the second photosensitive resin layer 3062, i.e., the upper lid resin, and portions of the upper lid resin on right and left parts of the partition layer 3061a flow toward the center of the channel pattern 3063. The portions of the upper lid resin that have flowed from parts of the partition layer 3061a are joined together on the side opposite from the substrate 3060, i.e., on the upper side of the channel pattern 3063, to form an upper lid layer 3062a. Thus, as shown in Fig. 32(c), the upper part of the channel pattern 3063 is covered with the upper lid layer 3062a to form a channel section 3063a, thereby producing the microfluidic chip 3600.

Similarly to the microfluidic chip 3500, in the microfluidic chip 3600 of the present example, the glass transition temperature (Tg) of the first photosensitive resin layer 3061, i.e., the partition resin, forming the partition layer 3061a is equal to that of the second photosensitive resin layer 3062, i.e., the upper lid resin, forming the upper lid layer 3062a. Therefore, when the upper lid resin is fluidized (reflows), the partition resin is also fluidized. In the present example, the first and second photosensitive resin layers 3061 and 3062 are each formed of a negative resist, and the exposure sensitivity of the second photosensitive resin layer 3062 is higher than that of the first photosensitive resin layer 3061. Therefore, as shown in Fig. 32(b), the opening width of the second photosensitive resin layer 3062 is formed to be smaller than the opening width of the partition layer 3061a formed of the first photosensitive resin layer 3061, and the opening width of the partition layer 3061a formed of the first photosensitive resin layer 3061 is formed to be sufficiently larger than the opening width of the second photosensitive resin layer 3062.

Accordingly, the width of the channel section 3063a is reduced by more than the opening width of the channel pattern 3063 is, due to occurrence of fluidization in the first photosensitive resin layer 3061 with a degree that is approximately the same as in the second photosensitive resin layer 3062; however, as shown in Fig. 32(c), the width of the channel section 3063a is sufficiently ensured.

Accordingly, if the first and second photosensitive resin layers 3061 and 3062 are each formed of a negative resist and if the condition of [Exposure sensitivity of upper lid resin > Exposure sensitivity of partition resin] is satisfied, the upper lid resin and the partition resin can be fluidized to easily form the upper lid layer 3062a, while sufficiently retaining the width of the channel section.

The description so far has been given of an example in which the partition layer and the upper lid layer are formed as separate bodies (multilayer configuration) in the microfluidic chip of the present embodiment. As described above, according to the production method of the present embodiment, the lid component (upper lid layer) covering the channel section of the microfluidic chip can be formed using existing photolithography techniques without using an intermediate layer such as an adhesive. Thus, adhesive components are prevented from eluting into the channel and inhibiting reactions of the solution in the channel.

Also, omission of an adhesive can avoid quality deterioration due to poor bonding which would have been caused by uneven film thickness of the adhesive. Furthermore, the production method can be simplified compared to the case where a partition layer and an upper lid layer are bonded together using an intermediate component such as an adhesive.

The present disclosure is not limited to this, but the partition layer and the upper lid layer may be configured using three or more layers. In this case also, the second photosensitive resin layer forming the upper lid layer may have a multilayer configuration. Photosensitive resins having thermal fluidity have characteristics such that
the higher the layer, the greater the fluidity of the resin upon heat treatment. Therefore, in the case of forming an upper lid layer using a second photosensitive layer with a multilayer configuration, parts of upper layers are joined together faster than the lower layers. Accordingly, in the upper lid layer formed of a second photosensitive resin layer with a multilayer configuration, the opening width becomes narrower the higher the layer, and parts of the uppermost layer or parts of the layers close to the uppermost layer are joined together.

### (4.1.3.4) Formation of bubble removal section in upper lid layer

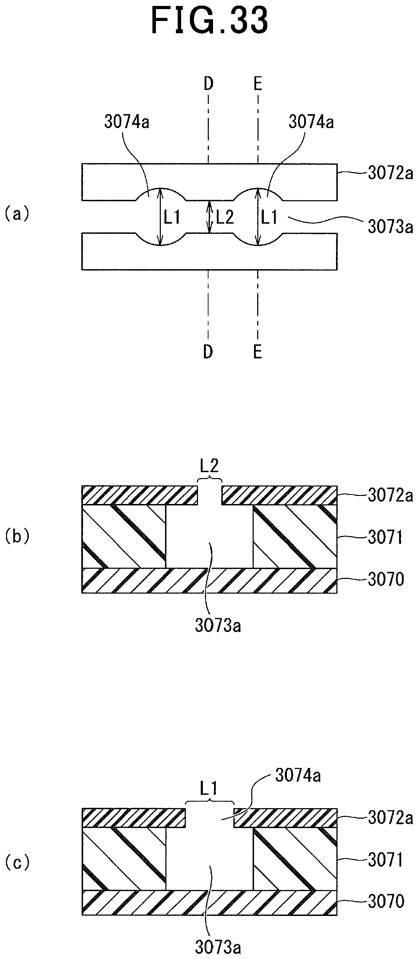

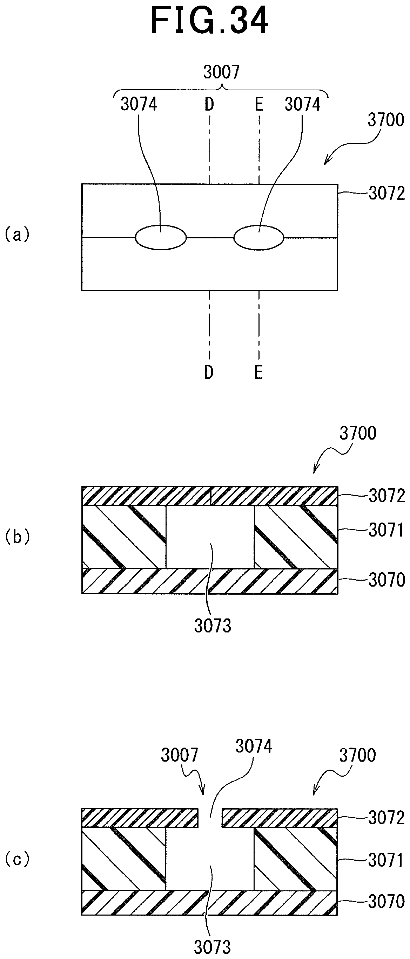

In the method of producing the microfluidic chip 3001 of the present embodiment, the bubble removal section 3007 is formed together with forming the upper lid layer 3012. Referring to Figs. 33 and 34, formation of the bubble removal section 3007 will be described.

Fig. 33(a) is a schematic plan view illustrating a channel pattern 3073a before undergoing heat treatment (post-baking) at step S3008, i.e., after washing away the developer at step S3007. Fig. 33(b) is a schematic cross-sectional view illustrating a cross section of a substrate 3070, a partition layer 3071, and a second photosensitive resin layer 3072a on the partition layer 3071, defining a channel pattern 3073a, taken along the line D-D of Fig. 33(a). Fig. 33(c) is a schematic cross-sectional view illustrating a cross section of the substrate 3070, the partition layer 3071, and the second photosensitive resin layer 3072a on the partition layer 3071, defining the channel pattern 3073a, taken along the line E-E of Fig. 33(a). As shown in Figs. 33(b) and 33(c), the channel pattern 3073a defined by the substrate 3070 is a space serving as a channel section through which a fluid flows in the microfluidic chip.

As shown in Figs. 33(a) to 33(c), the second photosensitive resin layer 3072a for forming an upper lid layer 3072 described later is arranged at the upper part of the channel pattern 3073a before post-baking. More specifically, the partition layer 3071 is formed on the substrate 3070, and the second photosensitive resin layer 3072a is formed on the partition layer 3071.

As shown in Figs. 33(a) to 33(c), the second photosensitive resin layer 3072a at the upper part of the channel pattern 3073a is provided with regions 3074a for forming through-holes that configure a bubble removal section. The regions 3074a are provided by, for example, controlling the exposure pattern when drawing the channel pattern. The regions 3074a each have an opening width L1 which is larger than an opening width L2 of the region other than the regions 3074a. The opening widths L1 and L2 can be adequately adjusted according to the difference in characteristics (difference in glass transition temperature or exposure sensitivity) between the partition layer resin for forming the partition layer 3071 and the upper lid resin for forming the second photosensitive resin layer 3072a, or using the photomask (exposure pattern) for drawing the channel pattern.

Fig. 34(a) is a schematic plan view illustrating a microfluidic chip 3700 prepared by performing heat treatment (post-baking) at step S3008 on the channel pattern 3073a shown in Figs. 33(a) to 33(c). Fig. 34(b) is a schematic cross-sectional view illustrating a cross section of the microfluidic chip 3700 taken along the line D-D of Fig. 34(a), and Fig. 34(c) is a schematic cross-sectional view illustrating the microfluidic chip 3700 taken along the line E-E of Fig. 34(a).

In the microfluidic chip 3700, a bubble removal section 3007 is formed by post-baking the portions corresponding to the regions 3074a of Fig. 33(a). More specifically, as shown in Fig. 34(b), fluidization of the second photosensitive resin layer 3072a is promoted by post-baking on opposing right and left parts of the partition layer 3071, and portions of the photosensitive resin are joined together on the upper part of the channel section 3073 near the center thereof. Thus, an upper lid layer 3072 is formed.

However, in the regions 3074a provided with the larger opening width L1 in advance (see Fig. 33(a)), parts of the second photosensitive resin layer 3072a are not completely joined together and retain gaps. Thus, as shown in Fig. 34(c), through-holes 3074 are formed. Thus, the bubble removal section 3007 having two through-holes 3074 has been formed in the upper lid layer 3072. In the present example, each through-hole 3074 of the bubble removal section 3007 in the upper lid layer 3072 has an opening end having a circular shape (specifically, elliptical shape) in plan view. The shape of the opening end can be adequately controlled by the shape of the exposure pattern (photomask).

Thus, in the present embodiment, through-holes (through-holes 3074 in the present example) configuring the bubble removal section 3007 can be formed by adjusting the opening widths in advance in the resin layer (second photosensitive resin layer 3072a) for the upper lid layer provided at the upper part of the channel pattern 3073a. Specifically, the bubble removal section 3007 can be easily formed simultaneously with the upper lid layer in the heat treatment step S3008 for forming an upper lid layer (the upper lid layer 3072 in the present example). In other words, the upper lid layer and multiple bubble-removal through-holes of the microfluidic chip 3700 can be formed using existing photolithography techniques.

Therefore, the production method can be simplified compared to the case where, for example, a bubble removal structure formed separately from the upper lid layer is bonded to the upper lid layer using an adhesive. Furthermore, no adhesive is used for forming the bubble removal section 3007. Therefore, adhesive components are prevented from eluting into the channel and inhibiting reactions of the solution in the channel.

In the present embodiment, the microfluidic chips 3400, 3500, 3600 and 3700 have configurations similar to the configuration of the microfluidic chip 3001. Also, the substrates 3040, 3050, 3060 and 3070 of these microfluidic chips are similar to the substrate 3010, the partition layers 3041a, 3051a, 3061a and 3071 are similar to the partition layer 3011, the upper lid layers 3042a, 3052a, 3062a and 3072 are similar to the upper lid layer 3012, and the channel sections 3043a, 3053a, 3063a and 3073 are similar to the channel section 3003.

### (4.1.4) Effects of Embodiment 4-1

The microfluidic chip 3001 described above has the following effects.
(1) The microfluidic chip 3001 according to the present embodiment includes a substrate 3010, a partition layer 3011 forming a channel section 3003 on the substrate 3010, and an upper lid layer 3012 formed on the surface of the partition layer 3011 facing away from the substrate 3010 to serve as a lid for the channel section 3003, wherein the upper lid layer 3012 is provided with a bubble removal section 3007 that is a part of the upper lid layer 3012 to remove bubbles generated in the channel section 3003, and no adhesive layer is provided between the partition layer 3011 and the upper lid layer 3012.

Thus, bubbles generated in the channel section can be removed, and adhesive components can be prevented from eluting into the channel and inhibiting reactions of the solution in the channel. Also, omission of an adhesive can avoid poor bonding which would have been caused by uneven film thickness of the adhesive.

(2) The microfluidic chip 3001 according to the present embodiment includes a substrate 3010, a partition layer 3011 forming a channel section 3003 on the substrate 3010, and an upper lid layer 3012 formed on the surface of the partition layer 3011 facing away from the substrate 3010 to serve as a lid for the channel section 3003, wherein the upper lid layer 3012 is provided with a bubble removal section 3007 that is a part of the upper lid layer 3012 to remove bubbles generated in the channel section 3003, and the upper lid layer 3012 and the partition layer 3011 are joined together by welding.

Thus, bubbles generated in the channel section can be removed, and adhesive components can be prevented from eluting into the channel and inhibiting reactions of the solution in the channel. Also, omission of an adhesive can avoid poor bonding which would have been caused by uneven film thickness of the adhesive.

(3) The microfluidic chip 3001 according to the present embodiment includes a channel section 3003 and an upper lid layer 3012 serving as a lid for the channel section 3003, wherein the material of the upper lid layer 3012 is a resin having thermal fluidity, and the upper lid layer 3012 is provided with a bubble removal section 3007 that is a part of the upper lid layer 3012 to remove bubbles generated in the channel section 3003.

Thus, bubbles generated in the channel section can be removed, and adhesive components can be prevented from eluting into the channel and inhibiting reactions of the solution in the channel. Also, the upper lid layer 3012 and the partition layer 3011 can be joined together using existing photolithography techniques, and thus, poor bonding which would have been caused by uneven film thickness of an adhesive can be avoided.

(4) In the microfluidic chip 3001 according to the present embodiment, the bubble removal section 3007 may be provided to a region in the upper lid layer 3012 facing the channel section 3003, so as to be located along the fluid transfer direction.

Thus, bubbles generated in the microchannel can be efficiently removed.

(5) In the microfluidic chip 3001 according to the present embodiment, the bubble removal section 3007 may have a through-hole 3017 passing through the upper lid layer 3012 in the thickness direction.

Thus, bubbles generated in the microchannel can be more reliably removed.

(6) In the microfluidic chip 3001 according to the present embodiment, the through-hole 3017 may have an opening end with a circular shape in plan view, and the opening end of the through-hole 3017 may have a diameter in the range of 1 µm or more and 100 µm or less.

Thus, bubbles can be removed from the channel section 3003 without causing liquid leakage.

(7) In the microfluidic chip 3001 according to the present embodiment, the through-hole 3017 may have an opening end with a polygonal shape in plan view, and the opening end of the through-hole 3017 may have a longitudinal width in the range of 1 µm or more and 100 µm or less.

Thus, bubbles can be removed from the channel section 3003 without causing liquid leakage.

(8) In the microfluidic chip 3001 according to the present embodiment, the bubble removal section 3007 may have multiple through-holes 3017, and the multiple through-holes 3017 may have an arrangement interval S therebetween in the range of 5 µm or more and 1,000 µm or less.

Thus, bubbles generated in the channel section 3003 can be more efficiently removed.

(9) In the microfluidic chip 3001 according to the present embodiment, the channel section 3003 may have a cross section with rounded corners.

Thus, the transfer speed or flow rate of the fluid (e.g., reaction solution) in the channel section 3003 can be stabilized, and the substances to be tested can be suppressed from being retained at the corners.

(10) In the microfluidic chip 3001 according to the present embodiment, the upper lid section may be a separate body from the partition section defining the channel.

Thus, a resin having favorable characteristics as an upper lid can be adequately selected.

(11) In the microfluidic chip 3001 according to the present embodiment, the partition layer 3011 forming the channel section 3003 and the upper lid layer 3012 may each be formed of a resin material, and the resin material may be a photosensitive resin having photosensitivity to light with a wavelength of 190 nm or more and 400 nm or less in the UV light region.

Thus, the lid component (upper lid layer) of the microfluidic chip can be formed using reflow of the photosensitive resins without using an adhesive, so that adhesive components are prevented from eluting into the channel and inhibiting reactions of the solution in the channel.

(12) In the microfluidic chip 3001 according to the present embodiment, the upper lid layer 3012 may be a separate body from the partition layer 3011 and may have a glass transition temperature lower than that of the partition layer 3011.

Thus, when forming the upper lid layer 3012, the partition layer 3011 can be suppressed from being fluidized and changing the channel pattern.

(13) In the microfluidic chip 3001 according to the present embodiment, the upper lid layer 3012 may be a separate body from the partition layer 3011 and may be configured to have an exposure sensitivity higher or lower than that of the partition layer 3011.

Thus, when fluidizing the resin material of the upper lid layer during formation of the upper lid layer 3012, if fluidization has also occurred in the partition layer resin, the opening width of the photosensitive resin forming the partition layer 3011 can be made sufficiently large and a sufficient space can be retained as the channel section 3003.

(14) The method of producing the microfluidic chip 3001 according to the present embodiment includes steps of applying a photosensitive resin for a partition layer 3011 onto a substrate 3010, applying a photosensitive resin for an upper lid layer 3012 onto the applied photosensitive resin for a partition layer 3011, exposing the photosensitive resin for a partition layer 3011 and the photosensitive resin for an upper lid layer 3012, developing and cleaning the exposed photosensitive resin for a partition layer 3011 and the exposed photosensitive resin for an upper lid layer 3012 to form a partition layer 3011 defining a channel section 3003 on the substrate 3010, and heat-treating and fluidizing the photosensitive resin for an upper lid layer 3012 on the partition layer 3011 to form an upper lid layer 3012 for the channel section 3003 and to provide a through-hole 3017 as a bubble removal section 3007 in the upper lid layer 3012 to remove bubbles generated in the channel section 3003.

Thus, there can be provided a microfluidic chip that can remove bubbles generated in the microchannel and can prevent adhesive components from eluting into the channel and inhibiting reactions of the solution in the channel.

### (4.2) Embodiment 4-2

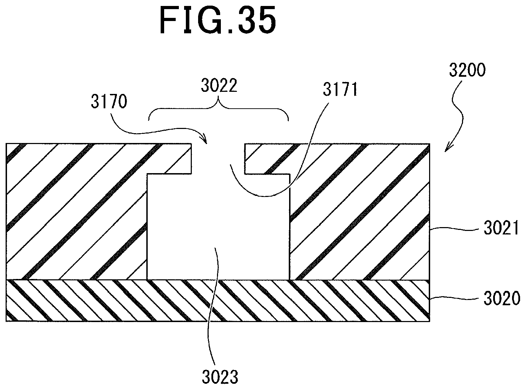

Referring to Fig. 35, a microfluidic chip according to Embodiment 4-2 of the present disclosure will be described. Fig. 35 is a cross-sectional view illustrating a configuration example of a microfluidic chip 3200 according to Embodiment 4-2 of the present disclosure.

The microfluidic chip 3200 includes a substrate 3020, a partition layer 3021 forming a channel section 3023 on the substrate 3020, and an upper lid layer 3022 formed of a part of the partition layer 3021. In other words, in the microfluidic chip 3200, the partition layer 3021 is formed integrally with the upper lid layer 3022. This is a point different from the microfluidic chips 3001, 3400, 3500, 3600 and 3700 of Embodiment 4-1.

### (4.2.1) Configuration of microfluidic chip 3200

The partition layer 3021 and the upper lid layer 3022 of the microfluidic chip 3200 will be described focusing on differences from the partition layer 3011 and the upper lid layer 3012 of Embodiment 4-1. Since the configurations of the components (substrate 3020, channel section 3023, bubble removal section 3170, and through-hole 3171) other than the upper lid layer 3022 are similar to those of the substrate 3010, channel section 3003, bubble removal section 3007, and through-hole 3017 of the microfluidic chip 3001, explanation will be omitted.

The materials of the partition layer 3021 and the upper lid layer 3022 of the microfluidic chip 3200 may be similar to that of the partition layer 3011 of the microfluidic chip 3001.

Fig. 35 shows a configuration example of the microfluidic chip 3200 in which the partition layer 3021 is formed integrally with the upper lid layer 3022. In the microfluidic chip 3200, since the partition layer 3021 serves as the upper lid layer 3022, the production processes can be simplified.

### (4.2.2) Method of producing microfluidic chip 3200

An example of a method of producing the microfluidic chip 3200 will be described.

The method of producing the microfluidic chip 3200 can omit step S3003 (step of applying the second photosensitive resin) from the method of producing the microfluidic chip 3001 shown in Fig. 29. Thus, the production processes can be simplified.

Referring to Fig. 36, the method of producing the microfluidic chip 3200 will be described in more detail.

Fig. 36 is a set of diagrams illustrating processes in the method of producing the microfluidic chip 3200 according to the present embodiment. In the method of producing the microfluidic chip 3200, a channel pattern is formed as in the steps S3001, S3002 and S3004 to S3007 of the method of producing the microfluidic chip 3001 shown in Fig. 29, except that only one layer of a photosensitive resin is formed on the substrate 3020. Therefore, explanation for the steps S3001, S3002 and S3004 to S3007 will be omitted.

Fig. 36(a) is a schematic plan view illustrating a channel pattern 3220 when producing the microfluidic chip 3200 according to the present embodiment. The partition layer 3021 is provided with an inlet 3032 for introducing a liquid, a channel section 3023 through which the liquid flows, and an outlet 3034 for discharging the liquid. Explanation for the inlet 3032 and the outlet 3034 will be omitted because they have configurations similar to those of the inlet 3002 and the outlet 3004 of the microfluidic chip 3001 according to Embodiment 4-1 described above.

Fig. 36(b) is a cross-sectional view taken along the line B-B of Fig. 36(a). The partition layer 3021 is formed on the substrate 3020 as a base. The region surrounded by the substrate 3020 and the partition layer 3021 is a region 3032a in the channel pattern 3220 serving as the inlet 3032 for introducing a fluid (e.g., reaction solution). In order to introduce a fluid, no upper lid layer 3022 is formed on the upper part of the inlet 3032.

Fig. 36(c) is a cross-sectional view taken along the line C-C of Fig. 36(a). The partition layer 3021 is formed on the substrate 3020 as a base component, and the region surrounded by the substrate 3020 and the partition layer 3021 is a region 3023a in the channel pattern 3220 defining the channel section 3023 through which a fluid flows. The region 3023a has an opening width which is formed to be smaller than that of the region 3032a serving as the inlet 3032.

The channel pattern 3220 formed is subjected to heat treatment (post-baking). Heat treatment is performed, for example, using a hot plate, oven, etc. Post-baking is performed for the purpose of fluidizing (reflowing) the partition resin (partition layer 3021) by heating the resin up to the glass transition temperature (Tg) thereof. This is a point different from the heat treatment in the method of producing the microfluidic chip 3001 according to Embodiment 4-1. Fluidization (reflow) of a photosensitive resin is characterized by the tendency that the photosensitive resin is fluidized more on the side opposite from the substrate 3020, i.e., on the upper part of the channel pattern 3220, depending on the characteristics of the resin. Due to fluidization of the upper part of the channel pattern 3220, parts of the partition layer 3021 are joined together to function as an upper lid component of the channel pattern 3220, i.e., as the upper lid layer 3022 covering the region 3023a of the channel pattern 3220. Thus, an upper lid layer 3022 integrated with the partition layer 3021 is formed, and thus a microfluidic chip 3200 of the present embodiment is prepared.

Fig. 36(d) is a schematic plan view illustrating the microfluidic chip 3200 after heat treatment (post-baking). Due to heat treatment, the partition layer 3021 is fluidized to form an upper lid layer 3022, as a result of which a channel section 3023 is defined as shown in Fig. 36(f) described later. Although not shown in Fig. 36(d), the channel section 3023 can be seen through a transparent upper lid layer 3022. The inlet 3032 and the outlet 3034 are reduced in size due to fluidization of the partition layer 3021; however, since they are designed in advance to have a larger size to compensate for size reduction, they are not closed by the upper lid layer 3022 and remain as through-holes. This is similar to the production of the microfluidic chip 3001 according to Embodiment 4-1.

Fig. 36(e) is a schematic cross-sectional view illustrating the microfluidic chip 3200 taken along the line B-B of Fig. 36(d). Due to post-baking on a hot plate 3025, the resin on the upper part of the partition layer 3021 flows toward the center of the inlet 3032 in the width direction; however, the inlet 3032 is not closed and the opening end remains in communication with the outside. In other words, the function of the inlet 3032 for introducing a fluid is maintained.

Fig. 36(f) is a schematic cross-sectional view illustrating the microfluidic chip 3200 taken along the line C-C of Fig. 36(d). Due to post-baking on the hot plate 3025, portions of the resin on one side of the partition layer 3021 facing away from the substrate 3020 (on the upper side of the partition layer 3021) flow from right and left and are joined together near the center of the channel section 3023 in the width direction to form the upper lid layer 3022. Thus, according to the present embodiment, the lid component (upper lid layer) covering the channel section of the microfluidic chip can be formed using existing photolithography techniques without using an adhesive. Thus, adhesive components are prevented from eluting into the channel and inhibiting reactions of the solution in the channel.

Also, omission of an adhesive can avoid quality deterioration due to poor bonding which would have been caused by uneven film thickness of the adhesive. Furthermore, the production method can be simplified compared to the case where a partition layer and an upper lid layer are bonded together using an intermediate component such as an adhesive. In addition, poor bonding can be more reliably suppressed by the integration of the partition layer 3021 with the upper lid layer 3022.

### (4.2.2.1) Formation of bubble removal section in upper lid layer

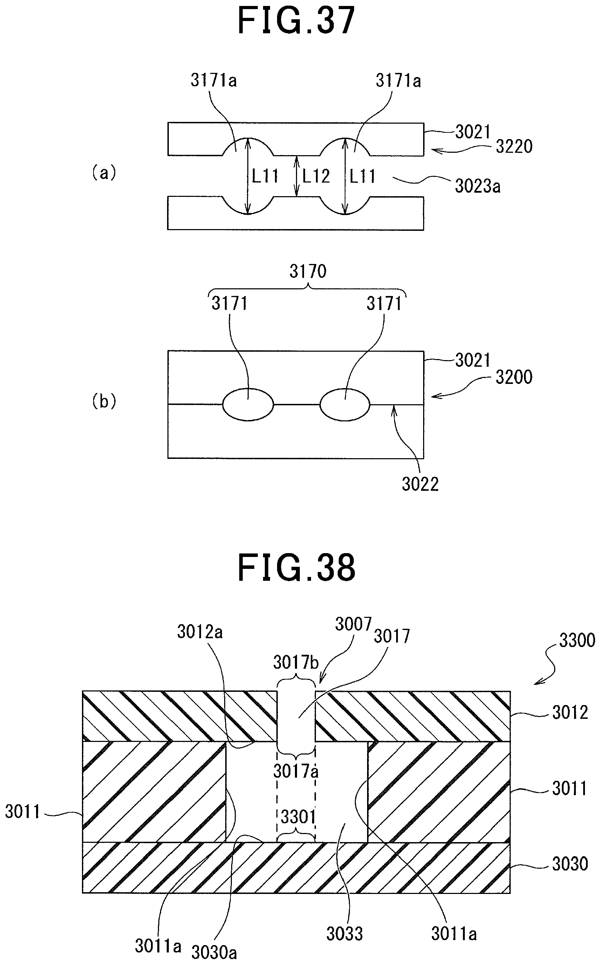

In the microfluidic chip 3200 of the present embodiment also, the bubble removal section is formed together with forming the upper lid layer as in Embodiment 4-1 described above. Referring to Fig. 37, formation of the bubble removal section 3170 in the upper lid layer 3022 will be described.

Fig. 37(a) is a schematic plan view illustrating a channel pattern 3220 before performing heat treatment (post-baking) at step S3008, i.e., after washing away the developer at step S3007. As shown in Fig. 37(a), in the channel pattern 3220, the upper part of the partition layer 3021 is provided with regions 3171a for forming through-holes configuring a bubble removal section. The regions 3171a are provided by, for example, controlling the exposure pattern when drawing the channel pattern. In the partition layer 3021, the regions 3171a for forming through-holes configuring a bubble removal section each have an opening width L11 which is made larger in advance than an opening width L12 of the region other than the regions 3171a.

Fig. 37(b) is a schematic plan view illustrating a microfluidic chip 3200 prepared by performing heat treatment (post-baking) at step S3008 on the channel pattern 3220. As shown in Fig. 37(b), as a result of performing post-baking, an upper lid layer 3022 and a bubble removal section 3170 have been formed in the microfluidic chip 3200.

Specifically, in the microfluidic chip 3200, portions of the resin on opposing right and left parts of the partition layer 3021 are fluidized by post-baking and flow toward the center of the region 3023a serving as the channel section 3023 in the width direction, and are joined together on the upper part of the channel section 3073 in the region 3023a near the center thereof. Thus, an upper lid layer 3022 is formed. In this case, in the regions 3171a provided with the larger opening width L11 in advance (see Fig. 37(a)), portions of the resin on parts of the partition layer 3021 are not completely joined together and retain gaps. Thus, as shown in Fig. 37(b), two through-holes 3171 are formed. Thus, the bubble removal section 3170 having the through-holes 3171 has been formed in the upper lid layer 3022.

Thus, in the present embodiment, through-holes (through-holes 3171 in the present example) configuring the bubble removal section 3170 can be formed by adjusting the opening widths in advance at the upper part of the partition layer 3021 of the channel pattern 3220. Specifically, the bubble removal section 3170 can be easily formed simultaneously with the upper lid layer in the heat treatment step S3008 for forming an upper lid layer (the upper lid layer 3022 in the present example) by fluidizing the upper part of the partition layer 3021. In other words, the upper lid layer and multiple bubble-removal through-holes of the microfluidic chip 3200 can be formed using existing photolithography techniques.

Therefore, the production method can be simplified compared to the case where, for example, a bubble removal structure formed separately from the partition layer 3021 forming the upper lid layer 3022 is bonded to the upper lid layer using an adhesive. Furthermore, no adhesive is used for forming the bubble removal section 3170. Therefore, adhesive components are prevented from eluting into the channel and inhibiting reactions of the solution in the channel.

As described above, the method of producing the microfluidic chip 3200 according to the present embodiment includes steps of applying a partition resin onto a substrate 3010 (step S3001), exposing the partition resin (step S3005), developing and cleaning the exposed partition resin to form a partition layer 3021 defining a channel section 3023 on the substrate 3020 (steps S3006 and S3007), and heat-treating the partition layer 3021 and fluidizing the partition resin to form an upper lid layer 3022 and to provide a through-hole 3171 as a bubble removal section 3170 in the upper lid layer 3022 to remove bubbles generated in the channel section 3023.

Thus, the upper lid layer 3022 can be joined to and integrated with the partition layer 3021 as a part thereof by welding, without using an adhesive, and therefore, adhesive components are prevented from eluting into the channel section 3023 and inhibiting reactions of the solution in the channel.

### (4.3) Embodiment 4-3

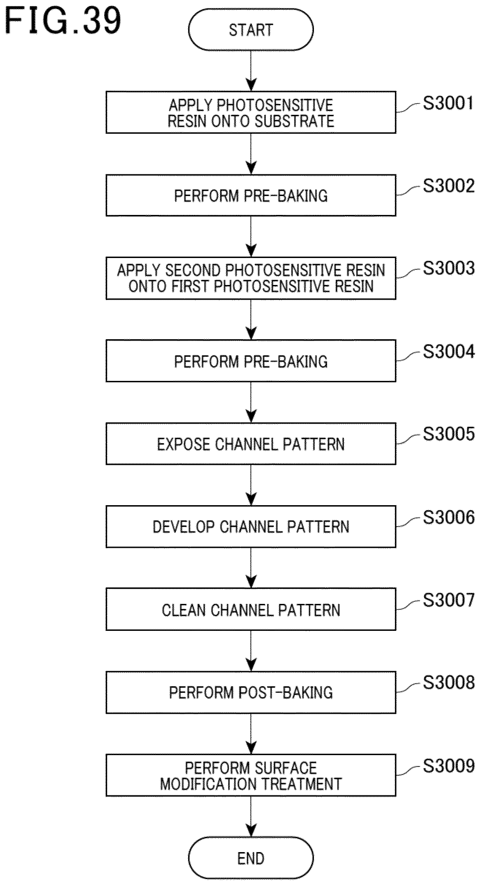

Referring to Figs. 38 and 39, a microfluidic chip according to Embodiment 4-3 of the present disclosure will be described. Fig. 38 is a cross-sectional view illustrating a configuration example of a microfluidic chip 3300 according to Embodiment 4-3 of the present disclosure.

The microfluidic chip 3300 includes a substrate 3030, a partition layer 3011 forming a channel section 3033 on the substrate 3030, and an upper lid layer 3012. Specifically, the microfluidic chip 3300 is different from the microfluidic chips 3001, 3400, 3500, 3600 and 3700 of Embodiment 4-1 and the microfluidic chip 3200 of Embodiment 4-2 in that it is provided with the substrate 3030 and the channel section 3033 formed on the substrate 3030.

### (4.3.1) Configuration of microfluidic chip 3300

The substrate 3030 and the channel section 3033 of the microfluidic chip 3300 will be described focusing on differences from the substrate 3010 and the channel section 3003 of Embodiment 4-1. Since the configurations of the components (partition layer 3011, upper lid layer 3012, bubble removal section 3007, and through-hole 3017) other than the substrate 3030 and the channel section 3033 of the microfluidic chip 3300 are similar to those of the partition layer 3011, upper lid layer 3012, bubble removal section 3007, and through-hole 3017 of the microfluidic chip 3001, explanation will be omitted.

The material of the substrate 3030 of the microfluidic chip 3300 may be similar to that of the substrate 3010 of the microfluidic chip 3001.

Fig. 38 is a schematic cross-sectional view illustrating a configuration example of the microfluidic chip 3300. As shown in Fig. 38, in the microfluidic chip 3300 of the present embodiment, a region in the substrate (one example of the base section) 3030 facing the through-hole 3017 of the bubble removal section 3007 is surface-modified. Specifically, a surface 3030a of the substrate 3030 surrounded by parts of the partition layer 3011 includes a region facing the through-hole 3017 (region right beneath the through-hole 3017) which is provided with a surface-modified section 3301. The surface-modified section 3301 may be referred to as a surface modification region. Due to having the surface-modified section 3301, the microfluidic chip 3300 of the present embodiment can prevent adhesive components from eluting into the channel and inhibiting reactions of the solution in the channel, and can improve bubble removal efficiency.

The surface-modified section 3301 is improved in wettability and imparted with hydrophilicity due to the surface modification treatment. In other words, the substrate (one example of the base section) 3030 has hydrophilicity in the region facing the through-hole 3017 of the bubble removal section 3007. Specifically, the surface-modified section 3301 has higher wettability than in other regions in the substrate 3030 configuring the channel section 3033, or channel-side surfaces 3011a of parts of the partition layer 3011, and channel-side surface 3012a of the upper-lid layer 3012. Thus, the microfluidic chip 3300 can more reliably improve bubble removal efficiency.

The surface modification treatment refers to a special treatment applied to a material surface to change the composition or structure of the material so that new functions can be imparted to the properties of the material. As an example of the surface modification treatment (hydrophilic treatment) for imparting hydrophilicity to (hydrophilizing) the surface-modified section 3301, an ultraviolet (UV) treatment of applying UV light can be mentioned. The ultraviolet (UV) treatment, many devices of which are comparatively inexpensive, can achieve hydrophilization of the surface targeted for surface modification treatment, by removing (cleaning) the organic film on the material surface (the surface 3030a in the present example) or forming silanol groups (Si-OH groups) on the material surface, using the energy of ultraviolet light emitted from the low-pressure mercury lamp as a light source and the power of ozone generated by the energy.

In the present embodiment, the surface modification treatment for forming the surface-modified section 3301 is not limited to the UV treatment but may, for example, be a plasma treatment of applying plasma (atmospheric pressure, vacuum, oxygen). The plasma treatment can also impart hydrophilicity to (hydrophilize) the surface targeted for surface modification treatment.

In Fig. 38, for ease of understanding, the widths of opening ends 3017a and 3017b of the through-hole 3017 match the width of the surface-modified section 3301; however, the maximum width of the surface-modified section 3301 does not necessarily match the widths of the opening ends 3017a and 3017b, depending on the conditions or environment of the UV treatment or plasma treatment. For example, the maximum width of the surface-modified section 3301 may become slightly larger than the widths of the opening ends 3017a and 3017b.

Bubbles contained in the fluid (e.g., reaction solution) passing through the microchannel may inhibit reactions of the solution or destabilize liquid transfer due to retention, etc. of the reaction solution in the microchannel. Therefore, as mentioned above, removal of bubbles in the fluids of the microchannels is an important issue for microfluidic devices.

As a method of efficiently removing bubbles in the fluids of microchannels, it is effective to impart hydrophobicity (water repellency) to the channel-side surfaces of the structural components (e.g., the lid component of the microchannel) having bubble removal sections. Such a method that can be considered may be to change the surfaces of the upper-side structural components of the microchannels to materials having hydrophobicity. However, if the materials of the structural components configuring the microchannels are changed to materials having hydrophobicity, adverse effects such as poor fluid flow may occur in large areas of the microchannels. Accordingly, it is preferred to impart the effect of improving bubble removal efficiency only to the regions around the bubble removal sections.

In this regard, the present inventors have noted that, if hydrophobicity is imparted to the region in the bottom surface of a microchannel (the channel-side surface of the structural component of the channel bottom) facing the bubble removal section, the region around the bubble removal section in the upper surface of the microchannel (the channel-side surface of the structural component including the bubble removal section) becomes relatively hydrophobic, and this can improve bubble removal efficiency.

If the bottom surface of the microchannel has hydrophilicity (high wettability), the fluid (e.g., liquid containing water as a main component, such as a reaction solution and culture fluid) passing through the microchannel will have high affinity for the bottom surface having hydrophilicity and will have low affinity for the upper surface which has become relatively hydrophobic.

On the other hand, bubbles contained in the fluid will have low affinity for the bottom surface region in the microchannel facing the bubble removal section, but will have high affinity for the region in the upper surface of the microchannel around the bubble removal section. Therefore, the bubbles contained in the fluid are efficiently separated from the fluid passing through the microchannel in the region in the microchannel near the bubble removal section, and thus bubbles can be more efficiently removed.

Specifically, in the microfluidic chip 3300 of the present embodiment, hydrophilicity is imparted to the region facing the through hoe 3017 of the bubble removal section 3007 provided to the upper lid layer 3012, and therefore, the region around the through-hole 3017 on the channel-side surface 3012a of the upper lid layer 3012 becomes relatively hydrophobic. In other words, in the channel section 3033, a high wettability region (the surface-modified section 3301) and a low wettability region (the surface 3012a in the region around the through-hole 3017) are provided at opposing positions. Accordingly, in the channel section 3033, there is a difference in wettability in the region between the surface-modified section 3301 and the through-hole 3017. Thus, the microfluidic chip 3300 can further improve bubble removal efficiency.

More specifically, as mentioned above, the fluid passing through the channel section 3033 will have high affinity for the surface-modified section 3301 of the substrate 3030 having hydrophilicity and will have low affinity for the surface 3012a in the region around the through-hole 3017 that has become relatively hydrophobic. Therefore, in the channel section 3033, the fluid is pushed down and moves closer to the surface-modified section 3301 having hydrophilicity (high wettability).

Contrarily, bubbles contained in the fluid will have low affinity for the surface-modified section 3301 having hydrophilicity and will have high affinity for the surface 3012a in the region around the through-hole 3017. Therefore, contrary to the fluid that has been pushed down and moved closer to the surface-modified section 3301, bubbles in the fluid move toward the surface 3012a facing the surface-modified section 3301. Thus, bubbles are efficiently separated from the fluid in the region between the surface-modified section 3301 of the channel section 3033 and the through-hole 3017, as a result of which, bubbles are more efficiently removed via the through-hole 3017.

Since the surface 3012a around the through-hole 3017 facing the surface-modified section 3301 of the substrate 3030 has become relatively hydrophobic, bubbles that have moved from the fluid to the region around the through-hole 3017 are easily popped. Therefore, bubble removal efficiency is further improved.

### (Water contact angle)

An example of an index for evaluating hydrophilicity of the surface-modified section 3301 of the substrate 3030 is a water contact angle. In the microfluidic chip 3300 of the present embodiment, the water contact angle at the surface-modified section 3301 on the surface 3030a of the substrate 3030 is preferred to be 90 degrees or less, and more preferred to be 30 degrees or less. If the water contact angle is 90 degrees or less, the surface-modified section 3301 can be said to have sufficient hydrophilicity for further improving the bubble removal effect.

The water contact angle can be controlled by adjusting the UV irradiation energy (unit: mJ) or adjusting UV irradiation time. Higher UV irradiation energy can make the water contact angle smaller, i.e., can improve hydrophilicity, at the surface targeted for irradiation (surface-modified section 3301 in the present example). Similarly, longer UV irradiation can make the water contact angle smaller, i.e., can improve hydrophilicity, at the surface targeted for irradiation (surface-modified section 3301).

The water contact angle can be measured, for example, using a contact angle meter PCA-1 manufactured by Kyowa Interface Science Co., Ltd. In the measurement using the contact angle meter, a syringe filled with water is loaded on the contact angle meter, and parameters such as waiting time [msec] before contact angle measurement, measurement time interval [msec], and number of consecutive measurements [times] are set. Next, water is dropped from the syringe onto the surface of a sample having the same configuration as that of the substrate 3030 and the contact angle (static contact angle) of the droplet is measured, while capturing images thereof. The static contact angle is measured using a drop method. In the drop method, a contact angle θ is calculated from a radius r and a height h of the droplet after landing, assuming that the droplet is part of a sphere.

### (4.3.2) Method of producing microfluidic chip 3300

Referring to Fig. 39, an example of a method of producing the microfluidic chip 3300 will be described. Fig. 39 is a flowchart illustrating an example of a method of producing the microfluidic chip 3300 according to the present embodiment. As shown in Fig. 39, in addition to the steps S3001 to S3008 of the method of producing the microfluidic chip 3001 shown in Fig. 29, the method of producing the microfluidic chip 3300 further includes a step of performing a surface modification treatment on the region in the partition layer 3011-side surface 3030a of the substrate 3030 facing the through-hole 3017 (step S3009). Thus, a surface-modified section 3301 can be provided to the substrate 3030 of the microfluidic chip 3300.

Specifically, at step S3009 following the heat treatment (post-baking treatment) of step S3008 in the method of producing the microfluidic chip 3300 of the present embodiment, surface modification treatment is performed on the surface 3030a of the substrate 3030 via the through-hole 3017 of the upper lid layer 3012 formed during the post-baking treatment. Thus, there can be obtained a microfluidic chip in which adhesive components can be prevented from eluting into the channel and inhibiting reactions of the solution in the channel, and bubble removal efficiency can be improved.

In the method of producing the microfluidic chip 3300 of the present embodiment, for example, UV treatment is performed, at step S3009, as surface modification treatment. More specifically, surface modification treatment (hydrophilization treatment) is performed by applying UV light to the region in the substrate 3030 facing the through-hole 3017 of the upper lid layer 3012 to impart hydrophilicity to the region. In this case, UV light is applied through the through-hole 3017. Thus, a surface-modified section 3301 can be provided at a position facing the through-hole 3017. Accordingly, bubble removal efficiency in the microfluidic chip can be more reliably improved.

The surface modification treatment (hydrophilization treatment) at step S3009 is not limited to UV treatment, but plasma treatment may be performed.

### (4.3.3) Modifications

The microfluidic chip 3300 according to the present embodiment has so far been described; however, mode of the microfluidic chip including the surface-modified section 3301 is not limited to this.

For example, the height of the channel section 3033 does not have to be constant but may vary. For example, the height of the partition layer 3011 may vary, or the surface 3030a of the substrate 3030 may be provided with slopes or asperities.

Usually, increase in height (depth) of the microchannels could reduce bubble removal efficiency; however, providing the surface-modified section 3301 having hydrophilicity at a position facing the through-hole 3017 of the bubble removal section 3007, bubble removal efficiency can be improved in the region where the height (depth) of the channel section 3033 is relatively large.

Also, for example, the channel section 3033 may have curves (rounded corners) in plan view. Usually, if microchannels have curved portions (corners), liquid transfer speed may be reduced, which leads to retention of the fluid and accordingly reduction in bubble removal efficiency; however, providing the surface-modified section 3301 having hydrophilicity at a position facing the through-hole 3017, bubble removal efficiency can be improved irrespective of the channel section 3033 having curved portions.

The microfluidic chip 3300 according to the present embodiment has a configuration of the microfluidic chip 3001 of Embodiment 4-1 with the surface-modified section 3301 added; however, the present disclosure is not limited to this. For example, the surface-modified section 3301 may be provided to the microfluidic chip 3200 of Embodiment 4-2 described above. Thus, bubble removal efficiency can also be improved in the microfluidic chip with a configuration in which the partition layer 3021 is integrated with the upper lid layer 3022.

### <Fourth Example>

The following description relates to specific examples of the microfluidic chip and the method of producing the same, according to the fourth embodiment of the present disclosure. The fourth embodiment of the present disclosure should not be limited to the following examples.

### (Example 4-1)

A microfluidic chip and a method of producing the same according to Embodiment 4-1 will be described.

First, a partition layer photosensitive resin was applied onto a glass substrate to form a first photosensitive resin layer. The partition layer photosensitive resin used herein was a negative transparent liquid resin (negative liquid resist) containing an epoxy resin. The glass transition temperature (Tg) of the photosensitive resin (negative liquid resist) was 160°C. The negative liquid resist was applied onto the glass substrate using a spin coater set to 1,100 rpm and 30 sec. The rotation speed and time of the spin coater were controlled so that the thickness of the first photosensitive resin layer would be 50 µm.

Next, the partition layer photosensitive resin (negative liquid resist) underwent heat treatment (pre-baking) on a hot plate, for the purpose of removing residual solvent contained therein. Pre-baking was performed at 90°C for 20 minutes.

Next, an upper lid photosensitive resin was applied onto the first photosensitive resin layer to form a second photosensitive resin layer. The upper lid photosensitive resin was similar to the negative liquid resist mentioned above, except for having a glass transition temperature (Tg) (110°C) which was lower than that of the partition layer photosensitive resin by 50°C. Pre-baking was performed under conditions similar to the partition layer photosensitive resin to remove residual solvent contained in the upper lid photosensitive resin.

Next, the photosensitive resin layers (first and second photosensitive resin layers) on the glass substrate were exposed to draw a channel pattern. Specifically, the photosensitive resin was subjected to pattern exposure via a photomask having a microchannel pattern array. The photomask was formed of a light-shielding film with a double-layer structure of chromium and chromium oxide. A proximity exposure device was used for the exposure. The exposure device had a high-pressure mercury lamp as a light source, with a cut filter that was an i-line filter inserted for exposure. The exposure dose was 170 mJ/cm². In this case, the microchannel pattern array of the photomask also included the pattern of the region for forming a through-hole of the bubble removal section.

Next, the exposed photosensitive resin layers were developed to form a channel pattern. Specifically, the photosensitive resin layers were developed for 60 seconds using an alkaline developer (TMAH 2.38%) to dissolve the unexposed portions and to pattern a channel structure.

Subsequently, shower cleaning was performed using ultra-pure water to remove the developer from the photosensitive resin layers on the substrate, followed by drying using a spin dryer. At this stage, the upper side of the channel section of the channel pattern opposite from the glass substrate was open. Also, the second photosensitive resin layer remained on the partition layer.

Next, the channel pattern was subjected to heat treatment (post-baking) in an oven at 160°C for 30 minutes. In this case, due to reflow of the photosensitive resin, the upper parts of the channel pattern, i.e., parts of the second photosensitive resin layer on opposing parts of the partition layer, flowed toward the center of the channel section to form an upper lid layer and a through-hole configuring the bubble removal section. Thus, a microfluidic chip of the present example was obtained. At both ends of the channel section, an inlet and an outlet, as through-holes, were formed without being closed by the upper lid layer.

In the microfluidic chip of the present example prepared as described above, the partition layer and the upper lid layer are joined together by welding without using an adhesive, and the upper lid layer is formed by fluidizing the upper lid layer resin (second photosensitive resin layer) during post-baking. Thus, adhesive components are prevented from eluting into the channel and inhibiting reactions of the solution in the channel.

10 µL of a colored reaction solution was collected with a pipette and introduced into the microfluidic chip of the present example from the inlet of the channel, and the state of liquid transfer was microscopically observed. The introduced reaction solution smoothly passed through the channel without leakage, and liquid transfer was good. In this way, the fluid passed smoothly through the microfluidic chip of the present example without leakage, and thus it was confirmed that the microfluidic chip was imparted with basic characteristics of a microfluidic chip.

The reaction solution passing through the channel contained bubbles generated when the solution was injected therein; however it was confirmed that these bubbles were efficiently externally discharged from the through-hole of the bubble removal section. Therefore, the solution passed through the bubble removal section contained no bubbles that would interrupt observation. Accordingly, it was confirmed that the microfluidic chip of the present example could allow a fluid to smoothly pass therethrough and was imparted with a function of efficiently removing bubbles contained in the fluid of the channel.

### (Example 4-2)

A microfluidic chip and a method of producing the same according to Embodiment 4-2 will be described.

A microfluidic chip according to Example 4-2 was prepared as in Example 4-1, except that the partition layer was formed integrally with the upper lid layer.

Specifically, first, as in Example 4-1, a partition layer photosensitive resin was applied onto a glass substrate to form a first photosensitive resin layer, followed by pre-baking.

Next, the first photosensitive resin layer on the glass substrate was exposed under conditions similar to Example 4-1 to draw a channel pattern.

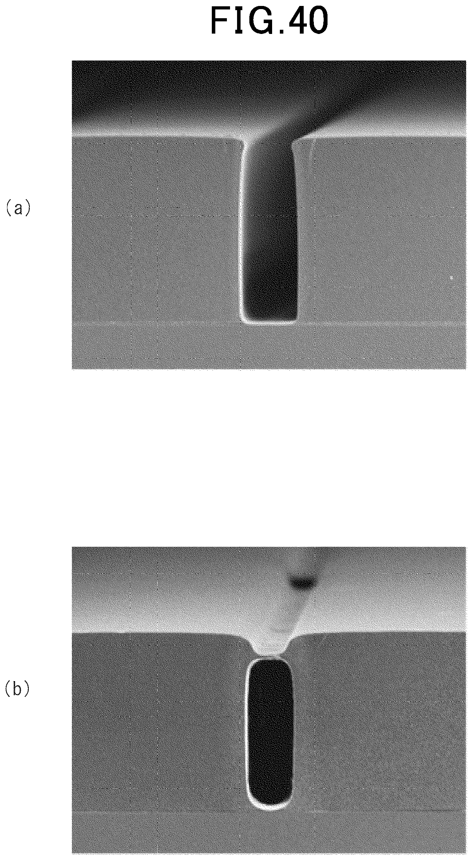

Next, the exposed first photosensitive resin layer was developed under conditions similar to Example 4-1, followed by removing the developer and drying using a spin dryer, thereby forming a channel pattern. Fig. 40(a) shows an example of a cross-sectional SEM image of the channel pattern at this point.

In Fig. 40(a), the recess sandwiched between opposing parts of the partition layer is the channel section of the channel pattern. As shown in Fig. 40(a), in the channel section of the channel pattern after removal of the developer and drying, the upper side thereof opposite from the glass substrate is open. The opening on the upper side of the channel section is similar in Example 4-1.

Next, the channel pattern underwent post-baking under conditions as in Example 4-1 (baked in an oven at 160°C for 30minutes). In this case, due to reflow of the photosensitive resin, the upper parts of the channel pattern, i.e., portions of the photosensitive resin (negative liquid resist) on opposing parts of the partition layer, flowed toward the center of the channel section to form an upper lid layer and a through-hole configuring the bubble removal section. Thus, a microfluidic chip of the present example was obtained.

Fig. 40(b) shows an example of a cross-sectional SEM image of the microfluidic chip after post-baking. As shown in Fig. 40(b), it was confirmed that the upper parts of the channel pattern after post-baking, i.e., portions of the photosensitive resin on parts of the partition layer, were fluidized and joined together near the center of the channel section to form an upper lid layer.

As shown in Fig. 40(b), it was confirmed that the upper lid layer formed due to fluidization of the partition layer had a reduced thickness toward the center of the channel section to form a recess. As shown in Fig. 40(b), it was confirmed that the cross section of the channel section had rounded corners. Furthermore, as shown at the upper center of Fig. 40(b), it was confirmed that a through-hole configuring the bubble removal section was locally formed in the upper lid layer in the depth direction. These points are similar to Example 4-1 in which the partition layer and the upper lid layer are separate bodies.

At both ends of the channel section, an inlet and an outlet, as through-holes, were formed without being closed by the upper lid layer.

In the microfluidic chip of the present example prepared as described above, an upper lid layer is formed due to the upper parts of the partition layer being fluidized by post-baking, and there is no adhesive used between the partition layer and the upper lid layer. Thus, adhesive components are prevented from eluting into the channel and inhibiting reactions of the solution in the channel.

Liquid transfer testing was performed for the microfluidic chip of the present example as in Example 4-1. The reaction solution introduced in the testing smoothly passed through the channel without leakage, and liquid transfer was good. In this way, the fluid passed smoothly through the microfluidic chip of the present example without leakage, and thus it was confirmed that the microfluidic chip was imparted with basic characteristics of a microfluidic chip.

The reaction solution passing through the channel contained bubbles generated when the solution was injected therein; however it was confirmed that these bubbles were efficiently externally discharged from the through-hole of the bubble removal section. Therefore, the solution passed through the bubble removal section contained no bubbles that would interrupt observation. Accordingly, it was confirmed that the microfluidic chip of the present example could allow a fluid to smoothly pass therethrough and was imparted with a function of efficiently removing bubbles contained in the fluid of the channel.

### (Example 4-3)

A microfluidic chip and a method of producing the same according to Embodiment 4-3 will be described.

A microfluidic chip of Example 4-3 was prepared as in Example 4-1 except that a surface modification treatment was performed on the microfluidic chip after post-baking.

Specifically, ultraviolet (UV) treatment was performed for 5 minutes using a tabletop ozone cleaning device (OC1801C10X) so that the accumulated amount of ultraviolet (UV) light would be 10,000 mJ. Thus, a surface-modified section was formed in the region in the substrate surface facing the through-hole. In this case, UV light was applied to the substrate surface through the through-hole of the upper lid layer. The UV treatment can remove (clean) the organic film on the irradiated surface (substrate surface) or form silanol groups (Si-OH groups) on the material surface, and can hydrophilize the region in the substrate surface facing the through-hole of the upper lid layer.

In the microfluidic chip of the present example, the UV treatment via the through-hole can locally hydrophilize the surface below (e.g., the region immediately below) the through-hole of the bubble removal section. Thus, bubbles contained in the fluid are efficiently separated from the fluid passing through the microchannel, and can be efficiently removed.

In the microfluidic chip of the present example prepared as described above, the partition layer and the upper lid layer are joined together by welding without using an adhesive, and the upper lid layer is formed by fluidizing the upper lid layer resin (second photosensitive resin layer) during post-baking. Thus, adhesive components are prevented from eluting into the channel and inhibiting reactions of the solution in the channel.

Liquid transfer testing was performed for the microfluidic chip of the present example as in Example 4-1.

The reaction solution introduced during testing smoothly passed through the channel without leakage, and liquid transfer was good. In this way, the fluid passed smoothly through the microfluidic chip of the present example without leakage, and thus it was confirmed that the microfluidic chip was imparted with basic characteristics of a microfluidic chip.

The reaction solution passing through the channel contained bubbles generated when the solution was injected therein; however it was visually confirmed that these bubbles were efficiently discharged outside from the through-hole of the bubble removal section. Therefore, the solution passed through the bubble removal section contained no bubbles that would interrupt observation.

Accordingly, it was confirmed that the microfluidic chip of the present example could allow a fluid to smoothly pass therethrough and was imparted with a function of efficiently removing bubbles contained in the fluid of the channel.

After the UV treatment for the microfluidic chip of the present example, the water contact angle at the surface-modified section on the substrate surface was measured. As mentioned above, in the measurement of the water contact angle, a static contact angle was measured with a drop method using a contact angle meter PCA-1 manufactured by Kyowa Interface Science Co., Ltd. In the present example, the water contact angle at the surface-modified section was 10 degrees. Accordingly, it was found that the microfluidic chip of the present example had hydrophilicity that was sufficient for improving bubble removal efficiency.

### (Example 4-4)

A microfluidic chip and a method of producing the same according to a modification of Embodiment 4-3 will be described.

A microfluidic chip of Example 4-4 was prepared by integrating a partition layer with an upper lid layer as in Example 4-2 except that a surface modification treatment was performed on the microfluidic chip after post-baking.

Specifically, ultraviolet (UV) treatment as a surface modification treatment was performed on a post-baked microfluidic chip for 5 minutes using a tabletop ozone cleaning device (OC1801C10X) so that the accumulated amount of ultraviolet (UV) light would be 10,000 mJ. Thus, a surface-modified section was formed in the region in the substrate surface facing the through-hole of the upper lid layer. In this case, as in Example 4-3, UV light was applied to the substrate surface through the through-hole of the upper lid layer.

Liquid transfer testing was performed for the microfluidic chip of the present example as in Example 4-1.

The reaction solution introduced during testing smoothly passed through the channel without leakage, and liquid transfer was good. In this way, the fluid passed smoothly through the microfluidic chip of the present example without leakage, and thus it was confirmed that the microfluidic chip was imparted with basic characteristics of a microfluidic chip.

The reaction solution passing through the channel contained bubbles generated when the solution was injected therein; however it was visually confirmed that these bubbles were efficiently discharged outside from the through-hole of the bubble removal section. Therefore, the solution passed through the bubble removal section contained no bubbles that would interrupt observation.

Accordingly, it was confirmed that the microfluidic chip of the present example could allow a fluid to smoothly pass therethrough and was imparted with a function of efficiently removing bubbles contained in the fluid of the channel.

After the UV treatment for the microfluidic chip of the present example, the water contact angle at the surface-modified section on the substrate surface was measured. As mentioned above, in the measurement of the water contact angle, a static contact angle was measured with a drop method using a contact angle meter PCA-1 manufactured by Kyowa Interface Science Co., Ltd. In the present example, the water contact angle at the surface-modified section was 10 degrees. Accordingly, it was found that the microfluidic chip of the present example had hydrophilicity that was sufficient for improving bubble removal efficiency.

For example, the fourth embodiment can have the following configurations.
(1) A microfluidic chip, including
   a base section;
   a partition section forming a channel on the base section; and
   an upper lid section formed on the surface of the partition section facing away from the base section to serve as a lid for the channel, wherein
   the upper lid section is provided with a bubble removal section that is a part of the upper lid section and removes bubbles generated in the channel; and
   no adhesive layer is provided between the partition section and the upper lid section.
(2) A microfluidic chip, including
   a base section;
   a partition section forming a channel on the base section; and
   an upper lid section formed on the surface of the partition section facing away from the base section to serve as a lid for the channel, wherein
   the upper lid section is provided with a bubble removal section that is a part of the upper lid section and removes bubbles generated in the channel; and
   the upper lid section and the partition section are joined together by welding.
(3) A microfluidic chip, including
   a channel; and
   an upper lid section that serves as a lid for the channel, wherein
   the material of the upper lid section is a resin having thermal fluidity; and
   the upper lid section is provided with a bubble removal section that is a part of the upper lid section and removes bubbles generated in the channel.
(4) The microfluidic chip according to (3), wherein
   the bubble removal section is provided in a region in the upper lid section facing the channel, so as to be located along a fluid transfer direction.
(5) The microfluidic chip according to any one of (1) to (4), wherein
   the bubble removal section includes a through-hole passing through the upper lid section in the thickness direction.
(6) The microfluidic chip according to (5), wherein
   the opening ends of the through-hole have a circular shape in plan view.
(7) The microfluidic chip according to (6), wherein
   the opening ends of the through-hole have a diameter in the range of 1 µm or more and 100 µm or less.
(8) The microfluidic chip according to (5), wherein
   the opening ends of the through-hole have a polygonal shape in plan view.
(9) The microfluidic chip according to (8), wherein
   the opening ends of the through-hole have a longitudinal width in the range of 1 µm or more and 100 µm or less.
(10) The microfluidic chip according to any one of (5) to (9), wherein
   the bubble removal section includes multiple through-holes; and
   the arrangement interval between the multiple through-holes is in the range of 5 µm or more and 1,000 µm or less.
(11) The microfluidic chip according to any one of (5) to (10), wherein
   the base section configuring the bottom surface of the channel has a surface-modified region facing the through-hole.
(12) The microfluidic chip according to any one of (5) to (11), wherein
   the base section configuring the bottom surface of the channel has a hydrophilic region facing the through-hole.
(13) The microfluidic chip according to any one of (1) to (12), wherein
   the channel has a cross section with rounded corners.
(14) The microfluidic chip according to any one of (1) to (13), wherein
   the upper lid section and the partition section forming the channel are separate from or integrated with each other.
(15) The microfluidic chip according to any one of (1) to (14), wherein
   the upper lid section includes a recess.
(16) The microfluidic chip according to any one of (1) to (15), wherein
   the partition section and the upper lid section forming the channel are each formed of a resin material; and
   the resin material is a photosensitive resin having photosensitivity to light with a wavelength of 190 nm or more and 400 nm or less in the UV light region.
(17) The microfluidic chip according to any one of (1) to (16), wherein
   the upper lid section is a separate body from the partition section forming the channel and has a glass transition temperature lower than that of the partition section.
(18) The microfluidic chip according to any one of (1) to (17), wherein
   the upper lid section is a separate body from the partition section forming the channel and has an exposure sensitivity higher or lower than that of the partition section.
(19) A method of producing a microfluidic chip including steps of
   applying a photosensitive resin onto a base section;
   exposing the applied photosensitive resin;
   developing and cleaning the exposed photosensitive resin to form a partition section defining a channel on the base section; and
   heat-treating the partition section and fluidizing the photosensitive resin to form an upper lid section for the channel and to provide a through-hole as a bubble removal section in the upper lid section to remove bubbles generated in the channel.
(20) A method of producing a microfluidic chip, including steps of
   applying a first photosensitive resin onto a base section;
   applying a second photosensitive resin onto the applied first photosensitive resin;
   exposing the first photosensitive resin and the second photosensitive resin;
   developing and cleaning the exposed first photosensitive resin and the exposed second photosensitive resin to form a partition section defining a channel on the base section; and
   heat-treating and fluidizing the second photosensitive resin on the partition section to form an upper lid section for the channel and to provide a through-hole as a bubble removal section in the upper lid section to remove bubbles generated in the channel.
(21) The method of producing a microfluidic chip according to (19) or (20), further including a step of
   performing a surface modification treatment on a region in the base section facing the through-hole.
(22) The method of producing a microfluidic chip according to (21), wherein
   ultraviolet light is applied to the region in the base section through the through-hole to perform the surface modification treatment and impart hydrophilicity to the region in the base section.

Several embodiments of the present disclosure have so far been described.

The microfluidic chip and the method of producing a microfluidic chip according to the first to fourth embodiments of the present disclosure should not be limited to the modes of the above embodiments (first to fourth embodiments) and First to Fourth Examples, but can be modified in various ways within the range not impairing the features of the invention.

The configurations of the first to fourth embodiments may be adequately combined with each other.

### [Industrial Applicability]

The first to fourth embodiments of the present disclosure can be favorably used as a high-quality microfluidic chip having no bonding failure or channel deformation, with the partition layer controlled in elastic modulus and Vickers hardness, and can be favorably used as a method of producing the microfluidic chip, in the fields of microfluidic chips aiming research, diagnosis, testing, analysis, culture, etc.

### [Reference Signs List]

1, 102, 103, 1001, 1200, 1400, 1500, 1600, 2001, 2200, 2300, 2310, 2400, 2500, 2600, 3001, 3200, 3400, 3500, 3600, 3700 Microfluidic chip
2, 1002, 1032, 2002, 2032, 3002, 3032 Inlet
3, 23, 33, 1003, 1023, 1043a, 1053a, 1063a, 2003, 2023, 2043a, 2053a, 2063a, 3003, 3023, 3043a, 3053a, 3063a, 3073 Channel section
4, 1004, 1034, 2004, 2034, 3004, 3034 Outlet
10, 20, 30, 1010, 1020, 1040, 1050, 1060, 2010, 2020, 2040, 2050, 2060, 3010, 3020,
3040, 3050, 3060, 3070 Substrate
11, 21, 31, 1011, 1021, 1041a, 1051a, 1061a, 2011, 2021, 2041a, 2051a, 2061a, 3011, 3021, 3041a, 3051a, 3061a, 3071 Partition layer
12, 22, 32 Cover layer
24, 34 Adhesive layer
1012, 1022, 1042a, 1052a, 1062a, 3012, 3022, 3042a, 3052a, 3062a, 3072 Upper lid layer
1025, 2025, 3025 Hot plate
1120,2120 Recess
2012, 2022, 2042a, 2052a, 2062a Intermediate layer
2013, 2130 Adhesive layer
2014, 2024 Cover layer
2033, 3017, 3074, 3171 Through-hole
2220 Channel pattern
2122a Gap
3007, 3170 Bubble removal section

## Claims

1. A microfluidic chip comprising
a base section;
a partition section that is configured by a resin material and provided on the base section to form a channel; and
a cover section that is provided on a surface of the partition section facing away from the base section, wherein
the partition section has an elastic modulus in a range of 1 MPa or more and 10 GPa or less.

2. The microfluidic chip according to claim 1, wherein
the partition section has a Vickers hardness of 2 HV or more.

3. The microfluidic chip according to claim 1 or 2, wherein
the cover section has an elastic modulus higher than that of the partition section.

4. The microfluidic chip according to claim 1 or 2, wherein
the cover section has an elastic modulus lower than that of the partition section.

5. The microfluidic chip according to any one of claims 1 to 4, wherein
the resin material forming the partition section is a photosensitive resin having photosensitivity to light with a wavelength of 190 nm or more and 400 nm or less in the UV light region.

6. A microfluidic chip comprising
a base section;
a partition section that forms a channel on the base section; and
an upper lid section that is formed on a surface of the partition section facing away from the base section to serve as a lid for the channel, wherein
the upper lid section and the partition section are joined together by welding.

7. The microfluidic chip according to claim 6, wherein
the upper lid section is separate from or integrated with the partition section forming the channel.

8. The microfluidic chip according to claim 6 or 7, wherein
the upper lid section includes a recess.

9. The microfluidic chip according to any one of claims 6 to 8, wherein
the upper lid section is a separate body from the partition section forming the channel and has a glass transition temperature lower than that of the partition section.

10. The microfluidic chip according to any one of claims 6 to 9, wherein
the upper lid section is provided with a bubble removal section that is a part of the upper lid section and removes bubbles generated in the channel.

11. The microfluidic chip according to claim 10, wherein
the bubble removal section includes a through-hole passing through the upper lid section in a thickness direction.

12. The microfluidic chip according to claim 11, wherein
the base section configuring a bottom surface of the channel has a region facing the through-hole, the region being surface-modified.

13. The microfluidic chip according to claim 11 or 12, wherein
the base section configuring the bottom surface of the channel has a region facing the through-hole, the region having hydrophilicity.

14. A microfluidic chip comprising
a base section;
a partition section that forms a channel on the base section;
a cover section that is located on one side of the partition section facing away from the base section to serve as a lid for the channel; and
an intermediate component that is located between the cover section and the channel.

15. The microfluidic chip according to claim 14, wherein
the intermediate component is part of the partition section; and
the cover section is bonded to the partition section via the intermediate component.

16. The microfluidic chip according to claim 14 or 15, wherein
the intermediate component and the partition section are joined together by welding.

17. A method of producing a microfluidic chip, comprising steps of
applying a resin onto a base section;
exposing the applied resin;
developing and cleaning the exposed resin to form a partition section that defines a channel on the base section;
post-baking the partition section; and
bonding a cover section to a surface of the partition section facing away from the base section, wherein
the partition section has an elastic modulus in a range of 1 MPa or more and 10 GPa or less.

18. A method of producing a microfluidic chip, comprising steps of
applying a first photosensitive resin onto a base section;
applying a second photosensitive resin onto the applied first photosensitive resin;
exposing the first photosensitive resin and the second photosensitive resin;
developing and cleaning the exposed first photosensitive resin and the exposed second photosensitive resin to form a partition section that defines a channel on the base section; and
heat-treating and fluidizing the second photosensitive resin on the partition section to form an upper lid section for the channel.
